# EUROPEAN PATENT APPLICATION

(11) **EP 4 368 631 A1**
(43) Date of publication of application: **15.05.2024**
(21) Application number: 22837695.0
(22) Date of filing: 06.07.2022
(51) Int. Cl.: C07K 14/00, A61K 9/08, A61K 38/10, A61K 38/16, A61P 31/14, C07K 7/08, C12N 7/06

(54) **PEPTIDE HAVING ANTI-VIRAL ACTIVITY, ANTI-VIRAL AGENT COMPRISING SAID PEPTIDE, AND METHOD FOR PRODUCING SAID ANTI-VIRAL AGENT**

(30) Priority: 07.07.2021 JP 2021113006; 07.09.2021 JP 2021145370; 28.06.2022 JP 2022103949
(71) Applicant: National University Corporation Tokyo Medical and Dental University, Tokyo 113-8510 (JP); Cespia Inc., Chiyoda-ku, Tokyo 100-0004 (JP)
(72) Inventor: FUJIYOSHI, Yoshinori, Tokyo 113-8510 (JP); NAKAMURA, Shun, Tokyo 113-8510 (JP); NOMURA, Risa, Tokyo 113-8510 (JP); TANIMURA, Yukihiro, Tokyo 113-8510 (JP); KAMEGAWA, Akiko, Tokyo 113-8510 (JP); NUMOTO, Nobutaka, Tokyo 113-8510 (JP)
(74) Representative: Mewburn Ellis LLP
(86) International application number: PCT/JP2022/026804
(87) International publication number: WO 2023/282281

(57) **Abstract**

Provided is a peptide capable of binding to a receptor binding domain (RBD) in SARS-CoV-2, chemically synthesized easily, having extremely high solubility to ultra-pure water or a buffer solution and a high possibility of being put to practical use as a therapeutic drug for COVID-19.

## Description

### Technical Field

The present invention relates to a peptide interacting with a receptor binding domain (RBD) in severe acute respiratory syndrome coronavirus 2 (SARS-CoV-2), an anti-viral agent containing the peptide, and a method for producing the anti-viral agent.

### Background Art

The global pandemic of Coronavirus Disease 2019 (COVID-19) caused by severe acute respiratory syndrome coronavirus 2 (SARS-CoV-2) has not been predicted to end, despite of taking worldwide public health measures. The clinical symptom caused by SARS-CoV-2 extends from asymptomatic infection to severe pneumonia, multiple organ failure and death.

Currently, mRNA vaccines and the like are supplied by a plurality of pharmaceutical companies for preventing and treating SARS-CoV-2 infection and diseases caused by the infection. However, multiple types of variant strains have emerged including a SARS-CoV-2 variant, which is a mutant of SARS-CoV-2 (wild-type SARS-CoV-2) found in Wuhan of China, so-called UK variant, South African variant and Brazilian variant, and make it difficult to end COVID-19. The wild-type SARS-CoV-2 herein refers to SARS-CoV-2 having a receptor binding domain (RBD) constituted of the amino acid sequence in which the position 339 is a glycine residue, the position 371 is a serine residue, the position 373 is a serine residue, the position 375 is a serine residue, the position 376 is a threonine residue, the position 405 is an aspartic acid residue, the position 408 is an arginine residue, the position 417 is a lysin residue, the position 440 is an asparagine residue, the position 446 is a glycine residue, the position 452 is a leucine residue, the position 477 is a serine residue, the position 478 is a threonine residue, the position 484 is a glutamic acid residue, the position 493 is a glutamine residue, the position 496 is a glycine residue, the position 498 is a glutamine residue, the position 501 is an asparagine residue, and the position 505 is a tyrosine residue. In contrast, a SARS-CoV-2 variant refers to SARS-CoV-2 having a receptor binding domain (RBD) constituted of the amino acid sequence having at least one mutation selected from the group consisting of a mutation into an aspartic acid residue at position 339, a mutation into a leucine residue or a phenylalanine residue at position 371, a mutation into a proline residue at position 373, a mutation into a phenylalanine residue at position 375, a mutation into an alanine residue at position 376, a mutation into an asparagine residue at position 405, a mutation into a serine residue at position 408, a mutation into an asparagine residue or threonine residue at position 417, a mutation into a lysine residue at position 440, a mutation into a serine residue at position 446, a mutation into an arginine residue at position 452, a mutation into an asparagine residue at position 477, a mutation into a lysine residue at position 478, a mutation into a lysine residue, a glutamine residue or an alanine residue at position 484, a mutation into an arginine residue at position 493, a mutation into a serine residue at position 496, a mutation into an arginine residue at position 498, a mutation into a tyrosine residue at position 501, and a mutation into a histidine residue at position 505.

To describe more specifically, examples of SARS-CoV-2 variants include: SARS-CoV-2 (PANGO lineage: B.1.1.7, sometimes referred to commonly as SARS-CoV-2 UK variant) having a receptor binding domain (RBD) constituted of the amino acid sequence having a mutation into a tyrosine residue at position 501 and wild-type amino acid residues at positions 339, 371, 373, 375 376, 405, 408, 417, 440, 446, 452, 477, 478, 484, 493, 496, 498 and 505; SARS-CoV-2 (PANGO lineage: B.1.351, sometimes referred to commonly as SARS-CoV-2 South African variant) having a receptor binding domain (RBD) constituted of the amino acid sequence having a mutation into an asparagine residue at position 417, a mutation into a lysine residue at position 484, and a mutation into a tyrosine residue at position 501, and wild-type amino acid residues at positions 339, 371, 373, 375, 376, 405, 408, 440, 446, 452, 477, 478, 493, 496, 498 and 505; SARS-CoV-2 (PANGO lineage: P.1, sometimes referred to commonly as SARS-CoV-2 Brazilian variant) having a receptor binding domain (RBD) constituted of the amino acid sequence having a mutation into a threonine residue at position 417, a mutation into a lysine residue at position 484, and a mutation into a tyrosine residue at position 501, and wild-type amino acid residues at positions 339, 371, 373, 375, 376, 405, 408, 440, 446, 452, 477, 478, 493, 496, 498 and 505; SARS-CoV-2 (PANGO lineage: B.1.617.2, sometimes referred to commonly as SARS-CoV-2 Indian variant (δ type)) having a receptor binding domain (RBD) constituted of the amino acid sequence having a mutation into an arginine residue at position 452 and a mutation into a lysine residue at position 478, and wild-type amino acid residues at positions 339, 371, 373, 375, 376, 405, 408, 417, 440, 446, 477, 484, 493, 496, 498, 501 and 505; SARS-CoV-2 (PANGO lineage: B.1.617.1, sometimes referred to commonly as SARS-CoV-2 Indian variant (κ type)) having a receptor binding domain (RBD) constituted of the amino acid sequence having a mutation into an arginine residue at position 452 and a mutation into a glutamine residue at position 484, and wild-type amino acid residues at positions 339, 371, 373, 375, 376, 405, 408, 417, 440, 446, 477, 478, 493, 496, 498, 501 and 505; SARS-CoV-2 (PANGO lineage: B.1.1.529/BA.1, sometimes referred to commonly as a SARS-CoV-2 Omicron variant (sublineage BA.1)) having a receptor binding domain (RBD) constituted of the amino acid sequence having a mutation into an aspartic acid residue at position 339, a mutation into a leucine residue at position 371, a mutation into a proline residue at position 373, a mutation into a phenylalanine residue at position 375, a mutation into an asparagine residue at position 417, a mutation into a lysine residue at position 440, a mutation into a serine residue at position 446, a mutation into an asparagine residue at position 477, a mutation into a lysine residue at position 478, a mutation into an alanine residue at position 484, a mutation into an arginine residue at position 493, a mutation into a serine residue at position 496, a mutation into an arginine residue at position 498, a mutation into a tyrosine residue at position 501, and a mutation into a histidine residue at position 505, and wild-type amino acid residues at positions 376, 405, 408 and 452; and SARS-CoV-2 (PANGO lineage: B.1.1.529/BA.2, sometimes referred to commonly as a SARS-CoV-2 Omicron variant (sublineage BA.2)) having a receptor binding domain (RBD) constituted of the amino acid sequence having a mutation into an aspartic acid residue at position 339, a mutation into a phenylalanine residue at position 371, a mutation into a proline residue at position 373, a mutation into a phenylalanine residue at position 375, a mutation into an alanine residue at position 376, a mutation into an asparagine residue at position 405, a mutation into a serine residue at position 408, a mutation into an asparagine residue at position 417, a mutation into a lysine residue at position 440, a mutation into an asparagine residue at position 477, a mutation into a lysine residue at position 478, a mutation into an alanine residue at position 484, a mutation into an arginine residue at position 493, a mutation into an arginine residue at position 498, a mutation into a tyrosine residue at position 501 and a mutation into a histidine residue at position 505, and wild-type amino acid residues at positions 446, 452 and 496.

Non Patent Literature 1 discloses a mini-protein constituted of 56 amino acid residues or 64 amino acid residues and exhibiting high affinity to the receptor binding domain (RBD) in wild-type SARS-CoV-2, and that particularly a specific protein (referred to as LCB1) consisting of 56 amino acid residues has the highest affinity to the receptor binding domain (RBD) in wild-type SARS-CoV-2. In the mini-protein called LCB1, three helixes interact to form a predetermined three-dimensional structure, which is capable of binding to the receptor binding domain (RBD) in wild-type SARS-CoV-2.

### Citation List

### Non Patent Literature

Non Patent Literature 1: L Cao et al., Science 370, 426-431 (2020)

### Summary of Invention

### Technical Problem

As disclosed in Non Patent Literature 1, as a drug discovery strategy, it has been expected to develop a therapeutic drug for COVID-19 using a protein (peptide) interacting with the receptor binding domain (RBD) in SARS-CoV-2 as a target. However, the protein disclosed in Non Patent Literature 1 (more specifically, e.g., a protein defined as LCB1 therein) has a sequence that is too long to be chemically synthesized at low cost by current technology. Because of this, the possibility of realizing a commercially available therapeutic drug using the protein disclosed in Non Patent Literature 1 (more specifically, e.g., a protein defined as LCB1 therein) as a main component is extremely low. In addition, it was found, by the studies conducted by the present inventors, that a protein LCB 1 most strongly binding to the receptor binding domain (RBD) in wild-type SARS-CoV-2, of the proteins disclosed in Non Patent Literature 1, cannot maintain the state of binding to the receptor binding domain (RBD) of a SARS-CoV-2 variant such as the SARS-CoV-2 UK variant (B.1.1.7).

Then, an object of the present invention is to provide a peptide capable of strongly binding to the receptor binding domain (RBD) of wild-type SARS-CoV-2, or a SARS-CoV-2 variant such as the SARS-CoV-2 UK variant (B.1.1.7), chemically synthesized easily, having extremely high solubility to ultra-pure water or a buffer solution, and a high possibility of being put to practical use as a therapeutic drug for COVID-19; at the same time, provide a pharmaceutical composition containing the peptide and a method for producing the peptide.

### Solution to Problem

The present inventors conducted intensive studies with a view to attaining the above objects. As a result, they succeeded in developing a group of peptides having two helix structures, high binding activity to the receptor binding domain (RBD) in wild-type SARS-CoV-2 and the receptor binding domain (RBD) in a SARS-CoV-2 variant such as the SARS-CoV-2 UK variant (B.1.1.7), maintaining a stable three-dimensional structure, chemically synthesized easily, and having extremely high solubility to ultra-pure water or a buffer solution. Based on the success, they arrived at the present invention. The present invention includes the followings.

[1] A peptide comprising a first region comprising a first helix and a second region comprising a second helix in the direction from an N terminal to a C terminal, wherein a sequence of a site in the first region that binds to the receptor binding domain (RBD) in SARS-CoV-2 comprises the following amino acid sequence (a), (b), (c) or (d):
   (a) an amino acid sequence of SEQ ID NO: 1,
   (b) an amino acid sequence of SEQ ID NO: 61,
   (c) an amino acid sequence of SEQ ID NO: 59, or
   (d) an amino acid sequence having a sequence identity of 80% or more with the amino acid sequence of SEQ ID NO: 1, 61 or 59;

   a sequence of a site in the second region that binds to the receptor binding domain (RBD) in SARS-CoV-2 comprises the following amino acid sequence (e), (f) or (g):
      (e) an amino acid sequence of SEQ ID NO: 2,
      (f) an amino acid sequence of SEQ ID NO: 57, or
      (g) an amino acid sequence having a sequence identity of 80% or more with the amino acid sequence of SEQ ID NO: 2 or 57; and
   a bond is formed between an amino acid residue within 5 residues of the N terminal in the sequence of the site in the first region that binds to the receptor binding domain (RBD) in SARS-CoV-2 and an amino acid residue within 5 residues from C terminal in the sequence of the site in the second region that binds to the receptor binding domain (RBD) in SARS-CoV-2, whereby the peptide binds to the receptor binding domain (RBD) in SARS-CoV-2.
[2] The peptide according to [1], comprising the first region comprising the first helix and the second region comprising the second helix in the direction from the N terminal to the C terminal, wherein the sequence of the site in the first region that binds to the receptor binding domain (RBD) in SARS-CoV-2 comprises the following amino acid sequence (a) or (d1):
   (a) the amino acid sequence of SEQ ID NO: 1, or
      (d1) an amino acid sequence having a sequence identity of 80% or more with the amino acid sequence of SEQ ID NO: 1;
      the sequence of the site in the second region that binds to the receptor binding domain (RBD) in SARS-CoV-2 comprises the following amino acid sequence (e) or (g1):
         (e) the amino acid sequence of SEQ ID NO: 2, or
         (g1) an amino acid sequence having a sequence identity of 80% or more with the amino acid sequence of SEQ ID NO: 2; and
      a bond is formed between an amino acid residue within 5 residues from N terminal in the sequence of the site in the first region that binds to the receptor binding domain (RBD) in SARS-CoV-2, and an amino acid residue within 5 residues from C terminal in the sequence of the site in the second region that binds to the receptor binding domain (RBD) in SARS-CoV-2, whereby the peptide binds to the receptor binding domain (RBD) in SARS-CoV-2.
[3] The peptide according to [1], comprising the first region comprising the first helix and the second region comprising the second helix in the direction from the N terminal to the C terminal, wherein the sequence of the site in the first region that binds to the receptor binding domain (RBD) in SARS-CoV-2 comprises the following amino acid sequence (b) or (d2):
   (b) the amino acid sequence of SEQ ID NO: 61, or
   (d2) an amino acid sequence having a sequence identity of 80% or more with the amino acid sequence of SEQ ID NO: 61;
   the sequence of the site in the second region that binds to the receptor binding domain (RBD) in SARS-CoV-2 comprises the following amino acid sequence (f) or (g2):
      (f) the amino acid sequence of SEQ ID NO: 57, or
      (g2) an amino acid sequence having a sequence identity of 80% or more with the amino acid sequence of SEQ ID NO: 57; and
      a bond is formed between an amino acid residue within 5 residues from N terminal in the sequence of the site in the first region that binds to the receptor binding domain (RBD) in SARS-CoV-2 and an amino acid residue within 5 residues from C terminal in the sequence of the site in the second region that binds to the receptor binding domain (RBD) in SARS-CoV-2, whereby the peptide binds to the receptor binding domain (RBD) in SARS-CoV-2.
[4] The peptide according to [1], comprising the first region comprising the first helix and the second region comprising the second helix in the direction from the N terminal to the C terminal, wherein the sequence of the site in the first region that binds to the receptor binding domain (RBD) in SARS-CoV-2 comprises the following amino acid sequence (c) or (db3):
   (c) the amino acid sequence of SEQ ID NO: 59, or
      (d3) an amino acid sequence having a sequence identity of 80% or more with the amino acid sequence of SEQ ID NO: 59;
      the sequence of the site in the second region that binds to the receptor binding domain (RBD) in SARS-CoV-2 comprises the following amino acid sequence (f) or (g2):
         (f) the amino acid sequence of SEQ ID NO: 57, or
         (g2) an amino acid sequence having a sequence identity of 80% or more with the amino acid sequence of SEQ ID NO: 57; and
      a bond is formed between an amino acid residue within 5 residues from N terminal in the sequence of the site in the first region that binds to the receptor binding domain (RBD) in SARS-CoV-2 and an amino acid residue within 5 residues from C terminal in the sequence of the site in the second region that binds to the receptor binding domain (RBD) in SARS-CoV-2, whereby the peptide binds to the receptor binding domain (RBD) in SARS-CoV-2.
[5] The peptide according to any one of [1] to [4], wherein the sequence of the site in the first region that binds to the receptor binding domain (RBD) in SARS-CoV-2 comprises the following amino acid sequence (h), (i), (j), (k) or (l):
   (h) an amino acid sequence of SEQ ID NO: 56,
   (i) an amino acid sequence of SEQ ID NO: 59,
   (j) an amino acid sequence of SEQ ID NO: 61,
   (k) an amino acid sequence of SEQ ID NO: 63, or
   (l) an amino acid sequence of SEQ ID NO: 65; and
   the sequence of the site in the second region that binds to the receptor binding domain (RBD) in SARS-CoV-2 comprises the amino acid sequence of SEQ ID NO: 57.
[6] The peptide according to [5], wherein the sequence of the site in the first region that binds to the receptor binding domain (RBD) in SARS-CoV-2 consists of the amino acid sequence of SEQ ID NO: 56.
[7] The peptide according to [5], wherein the sequence of the site in the first region that binds to the receptor binding domain (RBD) in SARS-CoV-2 consists of the amino acid sequence of SEQ ID NO: 59.
[8] The peptide according to [5], wherein the sequence of the site in the first region that binds to the receptor binding domain (RBD) in SARS-CoV-2 consists of the amino acid sequence of SEQ ID NO: 61.
[9] The peptide according to [5], wherein the sequence of the site in the first region that binds to the receptor binding domain (RBD) in SARS-CoV-2 consists of the amino acid sequence of SEQ ID NO: 63.
[10] The peptide according to [5], wherein the sequence of the site in the first region that binds to the receptor binding domain (RBD) in SARS-CoV-2 consists of the amino acid sequence of SEQ ID NO: 65.
[11] The peptide according to any one of [1] to [4], wherein, as the amino acid residue within 5 residues from N terminal and the amino acid residue within 5 residues from C terminal, a lysine residue and an aspartic acid residue, a lysine residue and a glutamic acid residue, an arginine residue and a glutamic acid residue, or an arginine residue and an aspartic acid residue are used in combination.
[12] The peptide according to [1] or [2], wherein the amino acid sequence (d) or (d1) is an amino acid sequence obtained by substituting at least one amino acid residue selected from the group consisting of the amino acid residue at position 1, the amino acid residue at position 8, the amino acid residue at position 11 and the amino acid residue at position 18 in the amino acid sequence of SEQ ID NO: 1, with another amino acid residue.
[13] The peptide according to [1] or [2], wherein the amino acid sequence (g) or (g1) is an amino acid sequence obtained by substituting at least one amino acid residue selected from the group consisting of the amino acid residue at position 2, the amino acid residue at position 14 and the amino acid residue at position 18 in the amino acid sequence of SEQ ID NO: 2, with another amino acid residue.
[14] The peptide according to [1] or [2], wherein the amino acid sequence (d) and (d1) are amino acid sequences obtained by substituting the arginine residue at position 15 and the glutamic acid residue at position 19 in the amino acid sequence of SEQ ID NO: 1 with a lysine residue and an aspartic acid residue, respectively.
[15] The peptide according to [1] or [2], wherein the amino acid sequence (g) or (g1) is an amino acid sequence obtained by substituting the glycine residue at position 2 in the amino acid sequence of SEQ ID NO: 2 with an alanine residue.
[16] The peptide according to [1] or [2], wherein the amino acid sequence (g) or (g1) is an amino acid sequence obtained by substituting the arginine residue at position 18 in the amino acid sequence of SEQ ID NO: 2 with a lysine residue.
[17] The peptide according to [1] or [2], wherein the amino acid sequence (d) or (d1) is an amino acid sequence in which the tyrosine residue at position 10 and the methionine acid residue at position 13 in the amino acid sequence of SEQ ID NO: 1 are further conserved.
[18] The peptide according to [1] or [2], wherein the amino acid sequence (g) or (g1) is an amino acid sequence in which the histidine residue at position 1, the serine residue, the aspartic acid residue at position 10 and the tyrosine residue at position 13 in the amino acid sequence of SEQ ID NO: 2 are further conserved.
[19] The peptide according to any one of [1] to [4], wherein the sequence of the site in the first region that binds to the receptor binding domain (RBD) in SARS-CoV-2 and/or the sequence of the site in the second region that binds to the receptor binding domain (RBD) in SARS-CoV-2 is constituted of 18 to 22 amino acid residues.
[20] The peptide according to any one of [1] to [4], consisting of the first region and the second region and having a full-length sequence of 39 amino acid residues.
[21] The peptide according to any one of [1] to [4], consisting of one amino acid sequence selected from the group consisting of SEQ ID NOs: 52 to 55, 58, 60, 62, 64 and 66.
[22] A peptide that binds to a receptor binding domain (RBD) in SARS-CoV-2, wherein the peptide has a first region and a second region in the direction from the N terminal to the C terminal, wherein
   the first region comprises an amino acid sequence selected from the following (h), (i), (j), (k) or (l):
      (h) an amino acid sequence of SEQ ID NO: 56,
      (i) an amino acid sequence of SEQ ID NO: 59,
      (j) an amino acid sequence of SEQ ID NO: 61,
      (k) an amino acid sequence of SEQ ID NO: 63, or
      (l) an amino acid sequence of SEQ ID NO: 65; and
   the second region comprises an amino acid sequence of SEQ ID NO: 57.
[23] The peptide according to [22], wherein the first region consists of the amino acid sequence of SEQ ID NO: 56.
[24] The peptide according to [22], wherein the first region consists of the amino acid sequence of SEQ ID NO: 59.
[25] The peptide according to [22], wherein the first region consists of the amino acid sequence of SEQ ID NO: 61.
[26] The peptide according to [22], wherein the first region consists of the amino acid sequence of SEQ ID NO: 63.
[27] The peptide according to [22], wherein the first region consists of the amino acid sequence of SEQ ID NO: 65.
[28] The peptide according to any one of [22] to [27], wherein the second region consists of the amino acid sequence of SEQ ID NO: 57.
[29] A peptide that binds a receptor binding domain (RBD) in SARS-CoV-2, the peptide consisting of an amino acid sequence of SEQ ID NO: 58.
[30] A peptide that binds a receptor binding domain (RBD) in SARS-CoV-2, the peptide consisting of an amino acid sequence of SEQ ID NO: 60.
[31] A peptide that binds a receptor binding domain (RBD) in SARS-CoV-2, the peptide consisting of an amino acid sequence of SEQ ID NO: 62.
[32] A peptide that binds a receptor binding domain (RBD) in SARS-CoV-2, the peptide consisting of an amino acid sequence of SEQ ID NO: 64.
[33] A peptide that binds a receptor binding domain (RBD) in SARS-CoV-2, the peptide consisting of an amino acid sequence of SEQ ID NO: 66.
[34] A pharmaceutical composition comprising the peptide according to any one of [1] to [33] as an active ingredient.
[35] The pharmaceutical composition according to [34], comprising at least one pharmaceutically acceptable carrier.
[36] The pharmaceutical composition according to [34] or [35] for treating COVID-19.
[37] The peptide according to any one of [1] to [33], for use in treatment of COVID-19.
[38] A method for treating COVID-19, comprising administering a therapeutically effective amount of the peptide according to any one of [1] to [33] to a patient in need thereof.
[39] Use of the peptide according to any one of [1] to [33], for producing a pharmaceutical composition for treating COVID-19.
[40] The pharmaceutical composition, peptide, treatment method or use according to any one of [36] to [39], wherein the COVID-19 is caused by infection with any one of wild-type SARS-CoV-2 having a receptor binding domain (RBD) in which an amino acid residue at position 339 is a glycine residue, an amino acid residue at position 371 is a serine residue, an amino acid residue at position 373 is a serine residue, an amino acid residue at position 375 is a serine residue, an amino acid residue at position 376 is a threonine residue, an amino acid residue at position 405 is an aspartic acid residue, an amino acid residue at position 408 is an arginine residue, an amino acid residue at position 417 is a lysine residue, an amino acid residue at position 440 is an asparagine residue, an amino acid residue at position 446 is a glycine residue, an amino acid residue at position 452 is a leucine residue, an amino acid residue at position 477 is a serine residue, an amino acid residue at position 478 is a threonine residue, an amino acid residue at position 484 is a glutamic acid residue, an amino acid residue at position 493 is a glutamine residue, an amino acid residue at position 496 is a glycine residue, an amino acid residue at position 498 is a glutamine residue, an amino acid residue at position 501 is an asparagine residue, and an amino acid residue at position 505 is a tyrosine residue; a first SARS-CoV-2 variant in which the amino acid residue at position 501 is a tyrosine residue; a second SARS-CoV-2 variant in which the amino acid residue at position 484 is a lysine residue; a third SARS-CoV-2 variant in which the amino acid residue at position 417 is an asparagine residue, the amino acid residue at position 484 is a lysine residue, and the amino acid residue at position 501 is a tyrosine residue; a fourth SARS-CoV-2 variant in which the amino acid residue at position 417 is a threonine residue, the amino acid residue at position 484 is a lysine residue, and the amino acid residue at position 501 is a tyrosine residue; a fifth SARS-CoV-2 variant in which the amino acid residue at position 452 is an arginine residue and the amino acid residue at position 478 is a lysine residue; a sixth SARS-CoV-2 variant in which the amino acid residue at position 452 is an arginine residue, and the amino acid residue at position 484 is a glutamine residue; a seventh SARS-CoV-2 variant in which the amino acid residue at position 339 is an aspartic acid residue, the amino acid residue at position 371 is a leucine residue, the amino acid residue at position 373 is a proline residue, the amino acid residue at position 375 is a phenylalanine residue, the amino acid residue at position 417 is an asparagine residue, the amino acid residue at position 440 is a lysine residue, the amino acid residue at position 446 is a serine residue, the amino acid residue at position 477 is an asparagine residue, the amino acid residue at position 478 is a lysine residue, the amino acid residue at position 484 is an alanine residue, the amino acid residue at position 493 is an arginine residue, the amino acid residue at position 496 is a serine residue, the amino acid residue at position 498 is an arginine residue, the amino acid residue at position 501 is a tyrosine residue, and the amino acid residue at position 505 is a histidine residue; and an eighth SARS-CoV-2 variant in which the amino acid residue at position 339 is an aspartic acid residue, the amino acid residue at position 371 is a phenylalanine residue, the amino acid residue at position 373 is a proline residue, the amino acid residue at position 375 is a phenylalanine residue, the amino acid residue at position 376 is an alanine residue, the amino acid residue at position 405 is an asparagine residue, the amino acid residue at position 408 is a serine residue, the amino acid residue at position 417 is an asparagine residue, the amino acid residue at position 440 is a lysine residue, the amino acid residue at position 477 is an asparagine residue, the amino acid residue at position 478 is a lysine residue, the amino acid residue at position 484 is an alanine residue, the amino acid residue at position 493 is an arginine residue, the amino acid residue at position 498 is an arginine residue, the amino acid residue at position 501 is a tyrosine residue, and the amino acid residue at position 505 is a histidine residue.
[41] The pharmaceutical composition for treating COVID-19 according to [36], wherein the composition is an aqueous solution having the peptide dissolved therein.

The present application claims the priority of JP Application Nos. 2021-113006, 2021-145370 and 2022-103949, the contents of which are incorporated herein.

### Advantageous Effects of Invention

The peptide according to the present invention is excellent in binding ability to a receptor binding domain (RBD) in wild-type SARS-CoV-2 or a SARS-CoV-2 variant, chemically synthesized easily, and has extremely high solubility to ultra-pure water or a buffer solution and a high possibility of being put to practical use as a therapeutic drug for COVID-19. Because of this, the peptide according to the present invention can prevent infection of a cell with wild-type SARS-CoV-2 and a SARS-CoV-2 variant, *in vivo.*

Furthermore, the therapeutic drug for COVID-19 according to the present invention contains a peptide excellent in binding ability to a receptor binding domain (RBD) of wild-type SARS-CoV-2 or a SARS-CoV-2 variant, chemically synthesized easily, and having extremely high solubility to ultra-pure water or a buffer solution as an active ingredient. Accordingly, the therapeutic drug for COVID-19 according to the present invention can prevent infection of a cell with wild-type SARS-CoV-2 and a SARS-CoV-2 variant *in vivo* and is expected as an effective therapeutic drug for COVID-19.

### Brief Description of Drawings

[Figure 1] Figure 1 is a characteristic chart showing CD spectra of Ca1, Cb1, Cb2, Cb3, Cb4, Cb5, Cb6, Cb7 and Ce1 produced in Examples.
[Figure 2] Figure 2 is a characteristic chart showing the measurement results of the interaction between Ce1 produced in an Example and the receptor binding domain (RBD) in wild-type SARS-CoV-2.
[Figure 3] Figure 3 is photographs showing the results of a plaque reduction assay using wild-type SARS-CoV-2 and carried out for Ce1 produced in an Example, and figures showing Ce1 concentration conditions.
[Figure 4] Figure 4 is a characteristic chart showing the relationship between the concentrations of Ce1 peptide shown in Figure 3 and the inhibition rate.
[Figure 5] Figure 5 is photographs showing the results of a plaque reduction assay using wild-type SARS-CoV-2 and carried out for LCB 1 peptide, and figures showing LCB1 concentration conditions.
[Figure 6] Figure 6 is photographs showing the results of a plaque reduction assay using wild-type SARS-CoV-2 and carried out for Ca1, and figures showing Ca1 concentration conditions.
[Figure 7] Figure 7 is a characteristic chart showing the relationship between the concentrations of LCB1 peptide shown in Figure 5 and the inhibition rate.
[Figure 8] Figure 8 is a characteristic chart showing the relationship between the concentrations of Ca1 peptide shown in Figure 6 and the inhibition rate.
[Figure 9] Figure 9 is a characteristic chart showing the measurement results of the interaction between Ce4 produced in an Example and the receptor binding domain (RBD) in wild-type SARS-CoV-2.
[Figure 10] Figure 10 is a characteristic chart showing the measurement results of the interaction between Ce4 produced in an Example and the receptor binding domain (RBD) in wild-type SARS-CoV-2 for 3 hours and 2 minutes from initiation of binding.
[Figure 11] Figure 11 is a characteristic chart showing the measurement results of the interaction between Ce9 produced in an Example and the receptor binding domain (RBD) in wild-type SARS-CoV-2.
[Figure 12] Figure 12 is a characteristic chart showing the measurement results of the interaction between Ce6 produced in an Example and the receptor binding domain (RBD) in wild-type SARS-CoV-2.
[Figure 13] Figure 13 is a characteristic chart showing the measurement results of the interaction between Ce5 produced in an Example and the receptor binding domain (RBD) in wild-type SARS-CoV-2.
[Figure 14] Figure 14 is a characteristic chart showing the measurement results of the interaction between Ce14 produced in an Example and the receptor binding domain (RBD) in wild-type SARS-CoV-2.
[Figure 15] Figure 15 is a characteristic chart showing the measurement results of the interaction between Ce15 produced in an Example and the receptor binding domain (RBD) in wild-type SARS-CoV-2.
[Figure 16] Figure 16 is a characteristic chart showing the measurement results of the interaction between Ce16 produced in an Example and the receptor binding domain (RBD) in wild-type SARS-CoV-2.
[Figure 17] Figure 17 is a characteristic chart showing the measurement results of the interaction between Ce41 produced in an Example and the receptor binding domain (RBD) in wild-type SARS-CoV-2.
[Figure 18] Figure 18 is a characteristic chart showing the measurement results of the interaction between Ce41 produced in an Example and the receptor binding domain (RBD) in the SARS-CoV-2 UK variant (B.1.1.7).
[Figure 19] Figure 19 is a characteristic chart showing the measurement results of the interaction between Ce41 produced in an Example and the receptor binding domain (RBD) in the SARS-CoV-2 Brazilian variant (P. 1).
[Figure 20] Figure 20 is a characteristic chart showing the measurement results of the interaction between Ce41 produced in an Example and the receptor binding domain (RBD) in the SARS-CoV-2 South African variant (B.1.351).
[Figure 21] Figure 21 is a characteristic chart showing the measurement results of the interaction between Ce41 produced in an Example and the receptor binding domain (RBD) in the SARS-CoV-2 Indian variant (δ type (B.1.617.2).
[Figure 22] Figure 22 is a characteristic chart showing the measurement results of the interaction between Ce41 produced in an Example and the receptor binding domain (RBD) in the SARS-CoV-2 Indian variant (κ type) (B.1.617.1).
[Figure 23] Figure 23 is photographs showing the results of a plaque reduction assay using the SARS-CoV-2 UK variant (B.1.1.7) and carried out for Ce41 produced in an Example, and figures showing Ce41 concentration conditions.
[Figure 24] Figure 24 is a characteristic chart showing the relationship between the concentrations of Ce41 peptide shown in Figure 23 and the inhibition rate.
[Figure 25] Figure 25 is a characteristic chart showing the measurement results of the interaction between Ce41 produced in an Example and the receptor binding domain (RBD) in the SARS-CoV-2 UK variant (B.1.1.7) for 3 hours and 2 minutes from initiation of binding.
[Figure 26] Figure 26 is a characteristic chart showing the measurement results of the interaction between Ce59 produced in an Example and the receptor binding domain (RBD) in wild-type SARS-CoV-2.
[Figure 27] Figure 27 is a characteristic chart showing the measurement results of the interaction between Ce59 produced in an Example and the receptor binding domain (RBD) in the SARS-CoV-2 UK variant (B.1.1.7).
[Figure 28] Figure 28 is a characteristic chart showing the measurement results of the interaction between Ce59 produced in an Example and the receptor binding domain (RBD) in the SARS-CoV-2 Brazilian variant (P.1).
[Figure 29] Figure 29 is a characteristic chart showing the measurement results of the interaction between Ce59 produced in an Example and the receptor binding domain (RBD) in the SARS-CoV-2 South African variant (B.1.351).
[Figure 30] Figure 30 is a characteristic chart showing the measurement results of the interaction between Ce59 produced in an Example and the receptor binding domain (RBD) in the SARS-CoV-2 Indian variant (δ type) (B.1.617.2).
[Figure 31] Figure 31 is a characteristic chart showing the measurement results of the interaction between Ce59 produced in an Example and the receptor binding domain (RBD) in the SARS-CoV-2 Indian variant (κ type)(B.1.617.1).
[Figure 32] Figure 32 is photographs showing the results of a plaque reduction assay using the SARS-CoV-2 Indian variant (δ type)(B.1.617.2) and carried out for Ce59 produced in an Example, and figures showing Ce59 concentration conditions.
[Figure 33] Figure 33 is a characteristic chart showing the relationship between the concentrations of Ce59 peptide shown in Figure 32 and the inhibition rate.
[Figure 34] Figure 34 is photographs showing the results of a plaque reduction assay using the SARS-CoV-2 UK variant (B.1.1.7) and carried out for Ce59 produced in an Example, and figures showing Ce59 concentration conditions.
[Figure 35] Figure 35 is a characteristic chart showing the relationship between the concentrations of Ce59 peptide shown in Figure 34 and the inhibition rate.
[Figure 36] Figure 36 is a characteristic chart showing the measurement results of the interaction between Ce59 produced in an Example and the receptor binding domain (RBD) in the SARS-CoV-2 UK variant (B.1.1.7) for 3 hours and 2 minutes from initiation of binding.
[Figure 37] Figure 37 is a characteristic chart showing the measurement results of the interaction between Ce113 produced in an Example and the receptor binding domain (RBD) in the SARS-CoV-2 UK variant (B.1.1.7).
[Figure 38] Figure 38 is a characteristic chart showing the measurement results of the interaction between Ce113 produced in an Example and the receptor binding domain (RBD) in the SARS-CoV-2 Indian variant (δ type) (B.1.617.1).
[Figure 39] Figure 39 is a characteristic chart showing the measurement results of the interaction between Ce113 produced in an Example and the receptor binding domain (RBD) in the SARS-CoV-2 Omicron (sublineage BA.1) variant (B.1.1.529BA.1).
[Figure 40] Figure 40 is a characteristic chart showing the measurement results of the interaction between Ce113 produced in an Example and the receptor binding domain (RBD) in the SARS-CoV-2 Omicron (sublineage BA.2) variant (B.1.1.529/BA.2).
[Figure 41] Figure 41 is a characteristic chart showing the measurement results of the interaction between Ce172 produced in an Example and the receptor binding domain (RBD) in the SARS-CoV-2 UK variant (B.1.1.7).
[Figure 42] Figure 42 is a characteristic chart showing the measurement results of the interaction between Ce172 produced in an Example and the receptor binding domain (RBD) in the SARS-CoV-2 Indian variant (δ type) (B.1.617.1).
[Figure 43] Figure 43 is a characteristic chart showing the measurement results of the interaction between Ce172 produced in an Example and the receptor binding domain (RBD) in the SARS-CoV-2 Omicron (sublineage BA.1) variant (B.1.1.529BA.1).
[Figure 44] Figure 44 is a characteristic chart showing the measurement results of the interaction between Ce172 produced in an Example and the receptor binding domain (RBD) in the SARS-CoV-2 Omicron (sublineage BA.2) variant (B.1.1.529/BA.2).
[Figure 45] Figure 45 is photographs showing the results of a plaque reduction assay using the SARS-CoV-2 UK variant (B.1.1.7) and carried out for Ce172 produced in an Example, and figures showing Ce172 concentration conditions.
[Figure 46] Figure 46 is a characteristic chart showing the relationship between the concentrations of Ce172 peptide shown in Figure 45 and the inhibition rate.
[Figure 47] Figure 47 is photographs showing the results of a plaque reduction assay using the SARS-CoV-2 Indian variant (δ type) (B.1.617.1) and carried out for Ce172 produced in an Example, and figures showing Ce172 concentration conditions.
[Figure 48] Figure 48 is a characteristic chart showing the relationship between the concentrations of Ce172 peptide shown in Figure 47 and the inhibition rate.
[Figure 49] Figure 49 is photographs showing the results of a plaque reduction assay using the SARS-CoV-2 Omicron (sublineage BA.1) variant (B.1.1.529BA.1) and carried out for Ce172 produced in an Example, and figures showing Ce172 concentration conditions.
[Figure 50] Figure 50 is a characteristic chart showing the relationship between the concentrations of Ce172 peptide shown in Figure 49 and the inhibition rate.
[Figure 51] Figure 51 is a characteristic chart showing the measurement results of the interaction between Ce173 produced in an Example and the receptor binding domain (RBD) in the SARS-CoV-2 UK variant (B.1.1.7).
[Figure 52] Figure 52 is a characteristic chart showing the measurement results of the interaction between Ce173 produced in an Example and the receptor binding domain (RBD) in the SARS-CoV-2 Indian variant (δ type) (B.1.617.1).
[Figure 53] Figure 53 is a characteristic chart showing the measurement results of the interaction between Ce173 produced in an Example and the receptor binding domain (RBD) in the SARS-CoV-2 Omicron (sublineage BA.1) variant (B.1.1.529BA.1).
[Figure 54] Figure 54 is a characteristic chart showing the measurement results of the interaction between Ce173 produced in an Example and the receptor binding domain (RBD) in the SARS-CoV-2 Omicron (sublineage BA.2) variant (B.1.1.529/BA.2).
[Figure 55] Figure 55 is photographs showing the results of a plaque reduction assay using the SARS-CoV-2 UK variant (B.1.1.7) and carried out for Ce173 produced in an Example, and shows the relationship between the Ce173 peptide concentration and the inhibition rate.
[Figure 56] Figure 56 is photographs showing the results of a plaque reduction assay using the SARS-CoV-2 Indian variant (δ type) (B.1.617.1) and carried out for Ce173 produced in an Example, and shows the relationship between the Ce173 peptide concentration and the inhibition rate.
[Figure 57] Figure 57 is photographs showing the results of a plaque reduction assay using the SARS-CoV-2 Omicron (sublineage BA.1) variant (B.1.1.529BA.1) and carried out for Ce173 produced in an Example, and shows the relationship between the Ce173 peptide concentration and the inhibition rate.
[Figure 58] Figure 58 is a characteristic chart showing the measurement results of the interaction between Ce174 produced in an Example and the receptor binding domain (RBD) in the SARS-CoV-2 UK variant (B.1.1.7).
[Figure 59] Figure 59 is a characteristic chart showing the measurement results of the interaction between Ce174 produced in an Example and the receptor binding domain (RBD) in the SARS-CoV-2 Indian variant (δ type) (B.1.617.1).
[Figure 60] Figure 60 is a characteristic chart showing the measurement results of the interaction between Ce174 produced in an Example and the receptor binding domain (RBD) in the SARS-CoV-2 Omicron (sublineage BA.1) variant (B.1.1.529BA.1).
[Figure 61] Figure 61 is a characteristic chart showing the measurement results of the interaction between Ce174 produced in an Example and the receptor binding domain (RBD) in the SARS-CoV-2 Omicron (sublineage BA.2) variant (B.1.1.529/BA.2).
[Figure 62] Figure 62 is photographs showing the results of a plaque reduction assay using the SARS-CoV-2 UK variant (B.1.1.7) and carried out for Ce174 produced in an Example, and shows the relationship between Ce174 peptide concentration and the inhibition rate.
[Figure 63] Figure 63 is photographs showing the results of a plaque reduction assay using the SARS-CoV-2 Indian variant (δ type) (B.1.617.1) and carried out for Ce174 produced in an Example, and shows the relationship between Ce174 peptide concentration and the inhibition rate.
[Figure 64] Figure 64 is photographs showing the results of a plaque reduction assay using the SARS-CoV-2 Omicron (sublineage BA.1) variant (B.1.1.529BA.1) and carried out for Ce174 produced in an Example, and shows the relationship between Ce174 peptide concentration and the inhibition rate.
[Figure 65] Figure 65 is a characteristic chart showing the measurement results of the interaction between Ce149 produced in an Example and the receptor binding domain (RBD) in the SARS-CoV-2 UK variant (B.1.1.7).
[Figure 66] Figure 66 is a characteristic chart showing the measurement results of the interaction between Ce149 produced in an Example and the receptor binding domain (RBD) in the SARS-CoV-2 Indian variant (δ type) (B.1.617.1).
[Figure 67] Figure 67 is a characteristic chart showing the measurement results of the interaction between Ce149 produced in an Example and the receptor binding domain (RBD) in the SARS-CoV-2 Omicron (sublineage BA.1) variant (B.1.1.529BA.1).
[Figure 68] Figure 68 is a characteristic chart showing the measurement results of the interaction between Ce149 produced in an Example and the receptor binding domain (RBD) in the SARS-CoV-2 Omicron (sublineage BA.2) variant (B.1.1.529/BA.2).
[Figure 69] Figure 69 is photographs showing the results of a plaque reduction assay using the SARS-CoV-2 UK variant (B.1.1.7) and carried out for Ce149 produced in an Example, and figures showing Ce149 concentration conditions.
[Figure 70] Figure 70 is a characteristic chart showing the relationship between the concentrations of Ce149 peptide shown in Figure 69 and the inhibition rate.
[Figure 71] Figure 71 is photographs showing the results of a plaque reduction assay using the SARS-CoV-2 Indian variant (δ type) (B.1.617.2) and carried out for Ce149 produced in an Example, and figures showing Ce149 concentration conditions.
[Figure 72] Figure 72 is a characteristic chart showing the relationship between the concentrations of Ce149 peptide shown in Figure 71 and the inhibition rate.
[Figure 73] Figure 73 is photographs showing the results of a plaque reduction assay using the SARS-CoV-2 Omicron (sublineage BA.1) variant (B.1.1.529BA.1) and carried out for Ce149 produced in an Example, and figures showing Ce149 concentration conditions.

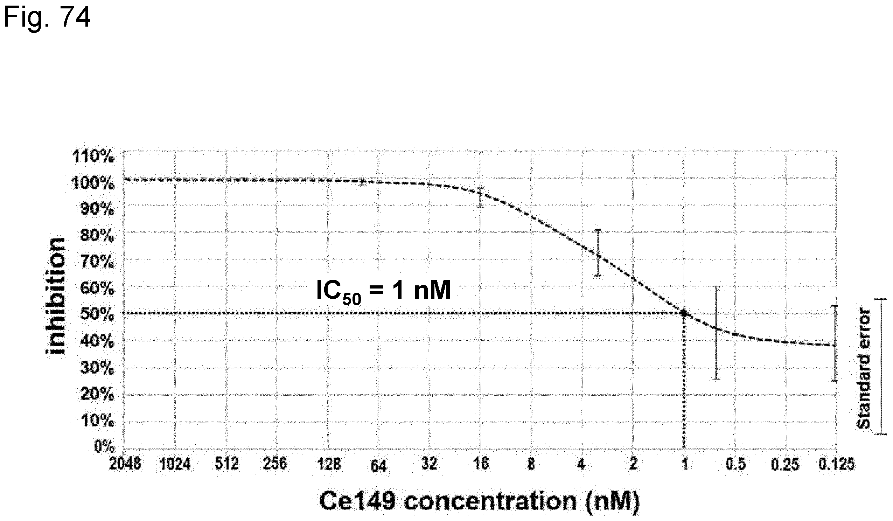

[Figure 74] Figure 74 is a characteristic chart showing the relationship between the concentrations of Ce149 peptide shown in Figure 73 and the inhibition rate.
[Figure 75] Figure 75 is a characteristic chart showing CD spectra of Ce4, Ce9, Ce41, Ce59, Ce113, Ce149, Ce172, Ce173 and Ce174 produced in Examples.
[Figure 76] Figure 76 is a characteristic chart showing the interaction between the second lysine residue in the first region and the 5 residues at the C terminal in the second region in the three-dimensional structure model of Ce41 produced in an Example.
[Figure 77] Figure 77 is a characteristic chart showing the measurement results of the interaction between Ce41 produced in an Example and the receptor binding domain (RBD) in the SARS-CoV-2 Omicron (sublineage BA.1) variant (B.1.1.529BA.1).
[Figure 78] Figure 78 is a characteristic chart showing the measurement results of the interaction between Ce59 produced in an Example and the receptor binding domain (RBD) in the SARS-CoV-2 Omicron (sublineage BA.1) variant (B.1.1.529/BA.1).

### Description of Embodiments

Now, the invention according to the disclosure will be more specifically described.

The peptide according to the disclosure can bind to a receptor binding domain (RBD) of wild-type SARS-CoV-2 or a SARS-CoV-2 variant. Owing to this, the peptide according to the disclosure can inhibit the binding of ACE2 (angiotensin-converting enzyme 2) expressed on a cell and the receptor binding domain (RBD) in SARS-CoV-2, thereby preventing infection of the cell with the SARS-CoV-2. As to wild-type SARS-CoV-2, the structure analysis results of a spike (S) protein present on a viral surface are disclosed in Wrapp, D. et al. Cryo-EM structure of the 2019-nCoV spike in the prefusion conformation, Science 367, 1260-1263 (2020). It is known that the S protein forms a trimer and has a basic amino acid sequence that is cleaved with a proteolytic enzyme of a host, furin. The S protein is cleaved with furin into S 1 subunit responsible for receptor binding ability and S2 subunit responsible for membrane fusion capability. The peptide according to the disclosure can bind to a receptor binding domain (RBD) present in S1 subunit. In the disclosure, the "receptor binding domain (RBD)" refers to a receptor binding domain (RBD) present in S 1 subunit of the spike protein.

The peptide according to the disclosure has two helix structures (for convenience's sake, the helix structure at the N terminal side is referred to as the first helix and the helix structure at the C terminal side as the second helix). The peptide according to the disclosure contains a region having the first helix at the N terminal side (for convenience's sake, referred to as the first region) and a region having the second helix at the C terminal side (for convenience's sake, referred to as the second region). A bond is formed between an amino acid residue within 5 residues from N terminal in the sequence of a site in the first region that binds to the receptor binding domain (RBD) in SARS-CoV-2 and an amino acid residue within 5 residues from C terminal in the sequence of a site in the second region that binds to the receptor binding domain (RBD) in SARS-CoV-2. As described above, one of the characteristics of the peptide according to the disclosure is that a desired three-dimensional structure can be maintained by the bond formed between an amino acid residue within 5 residues from N terminal contained in the first region and an amino acid residue within 5 residues from C terminal contained in the second region. In this disclosure, examples of the bond, which is formed between an amino acid residue within 5 residues from N terminal contained in the first region and an amino acid residue within 5 residues from C terminal contained in the second region, include a salt bridge and a hydrogen bond. The bond will be sometimes referred to specifically as a salt bridge or a hydrogen bond, conversely, a salt bridge or a hydrogen bond can be called as the bond.

Note that, the "amino acid residue within 5 residues from N terminal" means an amino acid residue positioned between the first and fifth amino acid residues counted from the N terminal and does not mean that the position of the amino acid residue involved in the formation of the above bond is limited to the N terminal. Similarly, the "amino acid residue within 5 residues from C terminal" means an amino acid residue positioned between the first and fifth amino acid residues counted from the C terminal and does not mean that the position of the amino acid residue involved in the formation of the above bond is limited to the C terminal.

One of the characteristics of the peptide according to the disclosure is that hydrophilic amino acid residues (more specifically, for example, the arginine residue at position 15 and the glutamic acid residue at position 19 in SEQ ID NO: 1) are introduced in the first helix to form a salt bridge, which realizes stabilization and hydrophilization of the helix structure to provide extremely high solubility to ultra-pure water or a buffer solution.

In the peptide according to the disclosure, the first region and second region have three-dimensional structures respectively mentioned above and have amino acid sequences that can bind to the receptor binding domain (RBD) in SARS-CoV-2, and thus, can be defined by the respective amino acid sequences. More specifically, the sequence of a site in the first region that binds to the receptor binding domain (RBD) in SARS-CoV-2 can be defined by the following amino acid sequence (a), (b), (c) or (d):
(a) the amino acid sequence of SEQ ID NO: 1,
(b) the amino acid sequence of SEQ ID NO: 61,
(c) the amino acid sequence of SEQ ID NO: 59,
(d) an amino acid sequence having a sequence identity of 80% or more with the amino acid sequence of SEQ ID NO: 1, 61 or 59.

The sequence of a site in the second region that binds to the receptor binding domain (RBD) in SARS-CoV-2 can be defined by the following amino acid sequence (e), (f) or (g):
(e) the amino acid sequence of SEQ ID NO: 2,
(f) the amino acid sequence of SEQ ID NO: 57,
(g) an amino acid sequence having a sequence identity of 80% or more with the amino acid sequence of SEQ ID NO: 2 or 57.

Furthermore, the sequence of a site in the first region that binds to the receptor binding domain (RBD) in SARS-CoV-2 can be defined by the following amino acid sequence (a) or (d1):
(a) the amino acid sequence of SEQ ID NO: 1,
(d1) an amino acid sequence having a sequence identity of 80% or more with the amino acid sequence of SEQ ID NO: 1.

The sequence of a site in the second region that binds to the receptor binding domain (RBD) in SARS-CoV-2 can be defined by the following amino acid sequence (e) or (g1):
(e) the amino acid sequence of SEQ ID NO: 2,
(g1) an amino acid sequence having a sequence identity of 80% or more with the amino acid sequence of SEQ ID NO: 2.

Furthermore, the sequence of a site in the first region that binds to the receptor binding domain (RBD) in SARS-CoV-2 can be defined by the following amino acid sequence (b) or (d2):
(b) the amino acid sequence of SEQ ID NO: 61,
(d2) an amino acid sequence having a sequence identity of 80% or more with the amino acid sequence of SEQ ID NO: 61.

The sequence of a site in the second region that binds to the receptor binding domain (RBD) in SARS-CoV-2 can be defined by the following amino acid sequence (f) or (g2):
(f) the amino acid sequence of SEQ ID NO: 57,
(g2) an amino acid sequence having a sequence identity of 80% or more with the amino acid sequence of SEQ ID NO: 57.

Furthermore, the sequence of a site in the first region that binds to the receptor binding domain (RBD) in SARS-CoV-2 can be defined by the following amino acid sequence (c) or (d3):
(c) the amino acid sequence of SEQ ID NO: 59,
(d3) an amino acid sequence having a sequence identity of 80% or more with the amino acid sequence of SEQ ID NO: 59.

The sequence of a site in the second region that binds to the receptor binding domain (RBD) in SARS-CoV-2 can be defined by the following amino acid sequence (f) or (g2):
(f) the amino acid sequence of SEQ ID NO: 57,
(g2) an amino acid sequence having a sequence identity of 80% or more with the amino acid sequence of SEQ ID NO: 57.

The amino acid sequence (a), (b) or (c) defining the first region has a lysine residue (the amino acid residue at position 2 from the N terminal in SEQ ID NO: 1, 59 or 61) and the amino acid sequence (e) or (f) defining the second region has an aspartic acid residue (the amino acid residue at position 1 from the C terminal in SEQ ID NO: 2 or 57). The peptides according to the disclosure, specified by SEQ ID NO: 1, 59 or 61 and SEQ ID NO: 2 or 57, can stabilize a desired three-dimensional structure by forming a salt bridge between the lysine residue and the aspartic acid residue.

However, the salt bridge to be formed between the first region and the second region is not limited to that formed of the above lysine residue and the above aspartic acid residue. For example, even if the amino acid residue at position 2 from the N terminal of SEQ ID NO: 1, 59 or 61 is an aspartic acid residue and the amino acid residue at position 1 from the C terminal of SEQ ID NO: 2 or 57 is a lysine residue, a salt bridge can be formed between the aspartic acid residue and the lysine residue to stabilize a desired three-dimensional structure.

The combination of amino acid residues forming a salt bridge is not limited to a combination of a lysine residue and an aspartic acid residue as mentioned above and, for example, a combination of a lysine residue and a glutamic acid residue, a combination of an arginine residue and a glutamic acid residue, and a combination of an arginine residue and an aspartic acid residue are acceptable. More specifically, as an example, even if an arginine residue is present at position 2 from the N terminal of SEQ ID NO: 1, 59 or 61 and a glutamic acid residue is present at position 1 form the C terminal of SEQ ID NO: 2 or 57, a salt bridge can be formed between the arginine residue and the glutamic acid residue to stabilize a desired three-dimensional structure. Note that, when a salt bridge is formed between an arginine residue and a glutamic acid residue, the glutamic acid residue may be present at position 2 from the N terminal of SEQ ID NO: 1, 59 or 61 and the arginine residue may be present at position 1 from the C terminal of SEQ ID NO: 2 or 57.

The amino acid sequence defined in the above (d), (d1), (d2) or (d3) is the amino acid sequence of a site binding to the receptor binding domain (RBD) in SARS-CoV-2 and may be an amino acid sequence having a sequence identity of 80% or more, 85% or more, 86% or more, 87% or more, 88% or more, 89% or more, 90% or more, 91% or more, 92% or more, 93% or more, 94% or more or 95% or more with the amino acid sequence of SEQ ID NO: 1, 59 or 61 as long as it conserves the amino acid residue involved in the formation of the above bond between the first region and the second region.

The amino acid sequence defined in the above (d), (d1), (d2) or (d3) is the amino acid sequence of a site binding to the receptor binding domain (RBD) in SARS-CoV-2 and may be an amino acid sequence having a substitution of 1 to 4 amino acid residues in the amino acid sequence of SEQ ID NO: 1, 59 or 61 as long as it conserves the amino acid residue involved in the formation of the above bond between the first region and the second region. Note that, an amino acid sequence having a substitution of one amino acid residue in the amino acid sequence of SEQ ID NO: 1, 59 or 61 has a sequence identity of 95% with the amino acid sequence of SEQ ID NO: 1, 59 or 61. An amino acid sequence having a substitution of 2, 3 and 4 amino acid residues in the amino acid sequence of SEQ ID NO: 1, 59 or 61 has a sequence identity of 90%, 85% and 80%, respectively, to the amino acid sequence of SEQ ID NO: 1, 59 or 61.

If 1 to 4 amino acid residues are substituted for the amino acid residues in the amino acid sequence of SEQ ID NO: 1, 59 or 61, the amino acid residue(s) to be substituted is not particularly limited but can be selected from the group consisting of the amino acid residue at position 1 (aspartic acid residue), the amino acid residue at position 8 (lysine residue), the amino acid residue at position 11 (glutamic acid residue) and the amino acid residue at position 18 (glutamic acid residue) in the amino acid sequence of SEQ ID NO: 1, 59 or 61. The amino acid residue at position 1 (aspartic acid residue), the amino acid residue at position 8 (lysine residue), the amino acid residue at position 11 (glutamic acid residue) and the amino acid residue at position 18 (glutamic acid residue) in the amino acid sequence of SEQ ID NO: 1, 59 or 61 contribute to stabilization of a helix structure but the interaction of these residues with the receptor binding domain (RBD) in SARS-CoV-2 is weak, and thus, these residues can be substituted with amino acid residues having analogous properties.

The aspartic acid residue at position 1 in SEQ ID NO: 1, 59 or 61 is not particularly limited but can be substituted with an asparagine residue or a glutamic acid residue. Aspartic acid, asparagine and the like have an analogous property (called as N-cap) of initiating the formation of a helix. Aspartic acid and glutamic acid both are acidic amino acids and have analogous properties. Thus, even if the aspartic acid residue at position 1 in SEQ ID NO: 1, 59 or 61 is substituted with an asparagine residue or a glutamic acid residue, the structure of a first helix and the interaction with the receptor binding domain (RBD) in SARS-CoV-2 are not significantly affected and the function of the peptide according to the disclosure can be maintained.

The lysine residue at position 8 in SEQ ID NO: 1, 59 or 61 is not particularly limited but can be substituted with an arginine residue or a histidine residue. Lysine, arginine, and histidine are basic amino acids and have analogous properties. Thus, even if the lysine residue at position 8 in SEQ ID NO: 1, 59 or 61 is substituted with an arginine residue or a histidine residue, the structure of a first helix and the interaction with the receptor binding domain (RBD) in SARS-CoV-2 are not significantly affected and the function of the peptide according to the disclosure can be maintained. In particular, lysine and arginine have a common property in that they have no aromatic ring. Thus, even if the lysine residue at position 8 is substituted with an arginine residue, the structure of a first helix and the interaction with the receptor binding domain (RBD) in SARS-CoV-2 are not significantly affected and the function of the peptide according to the disclosure can be highly maintained.

The glutamic acid residue at position 11 and the glutamic acid residue at position 18 in SEQ ID NO: 1, 59 or 61 are not particularly limited but can be substituted with an aspartic acid residue. The glutamic acid and aspartic acid are both acidic amino acids and having analogous properties. Thus, even if the glutamic acid residue at position 11 and/or the glutamic acid residue at position 18 in SEQ ID NO: 1, 59 or 61 are substituted with an aspartic acid residue, the structure of a first helix and the interaction with the receptor binding domain (RBD) in SARS-CoV-2 are not significantly affected and the function of the peptide according to the disclosure can be maintained.

The amino acid sequence specified in the above (g), (g1) or (g2) is an amino acid sequence of a site binding to the receptor binding domain (RBD) in SARS-CoV-2 and may be an amino acid sequence having a sequence identity of 80% or more, 85% or more, 86% or more, 87% or more, 88% or more, 89% or more, 90% or more, 91% or more, 92% or more, 93% or more, 94% or more or 95% or more with the amino acid sequence of SEQ ID NO: 2 or 57 as long as it conserves the amino acid residue involved in the formation of the above bond between the first region and the second region.

The amino acid sequence specified in the above (g), (g1) or (g2) is an amino acid sequence of a site binding to the receptor binding domain (RBD) in SARS-CoV-2 and may be an amino acid sequence having a substitution of 1 to 3 amino acid residues in the amino acid sequence of SEQ ID NO: 2 or 57 as long as it conserves the amino acid residue involved in the formation of the above bond between the first region and the second region. Note that, an amino acid sequence having a substitution of one amino acid residue in the amino acid sequence of SEQ ID NO: 2 or 57 has a sequence identity of 94.7% with the amino acid sequence of SEQ ID NO: 2 or 57. The amino acid sequences having a substitution of 2 and 3 amino acid residues in the amino acid sequence of SEQ ID NO: 2 or 57 have a sequence identity of 89.5% and 84.2%, respectively, with the amino acid sequence of SEQ ID NO: 2 or 57.

If the residues in the amino acid sequence of SEQ ID NO: 2 or 57 are substituted with 1 to 3 amino acid residues, the residue(s) to be substituted is not particularly limited but can be selected from the group consisting of the amino acid residue at position 2 (glycine residue), the amino acid residue at position 14 (glutamic acid residue) and the amino acid residue at position 18 (arginine residue).

The glycine residue at position 2 in SEQ ID NO: 2 or 57 can be substituted with an alanine residue. The glycine residue at position 2 in SEQ ID NO: 2 or 57 is the most suitable amino acid residue to all receptor binding domains (RBD) of SARS-CoV-2 in which the asparagine residue at position 501 in the amino acid sequence of the receptor binding domain in SARS-CoV-2 is changed by a tyrosine residue, but the substitution with an alanine residue makes the binding to the receptor binding domain (RBD) in wild-type SARS-CoV-2 further stronger.

In the peptide according to the disclosure, the amino acid residue at position 1 in SEQ ID NO: 2 or 57 is preferably a histidine residue. If the amino acid residue at position 1 in SEQ ID NO: 2 or 57 is a histidine residue, it interacts with the amino acid residue at position 19 in SEQ ID NO: 1, 59 or 61, for example, a glutamic acid residue, and can contribute to structure stabilization. The histidine residue further contributes to the stabilization of a helix bundle structure.

The glutamic acid residue at position 14 in SEQ ID NO: 2 or 57 can be substituted, but is not particularly limited with, an aspartic acid residue. The glutamic acid and aspartic acid both are acidic amino acids and have analogous properties. Thus, even if the glutamic acid residue at position 14 in SEQ ID NO: 2 or 57 is substituted with an aspartic acid residue, the structure of a second helix and interaction with the receptor binding domain (RBD) in SARS-CoV-2 are not significantly affected and the function of the peptide according to the disclosure can be maintained. In particular, lysine and arginine have a common property in that they have no aromatic ring. Thus, even if the arginine residue at position 18 is substituted with a lysine residue, the structure of a second helix and the interaction with the receptor binding domain (RBD) in SARS-CoV-2 are not significantly affected and the function of the peptide according to the disclosure can be highly maintained.

Further, the arginine residue at position 18 in SEQ ID NO: 2 or 57 can be substituted, but not particularly limited with, a lysine residue or a histidine residue. Arginine, lysine and histidine are basic amino acids and have analogous properties. Thus, even if the arginine residue at position 18 in SEQ ID NO: 2 or 57 is substituted with a lysine residue or a histidine residue, the structure of a second helix and interaction with the receptor binding domain (RBD) in SARS-CoV-2 are not significantly affected and the function of the peptide according to the disclosure can be maintained. In particular, lysine and arginine have in common that they do not have aromatic ring. Thus, even if the arginine residue at position 18 is substituted with a lysine residue, the structure of a second helix and the interaction with the receptor binding domain (RBD) in SARS-CoV-2 are not significantly affected and the function of the peptide according to the disclosure can be highly maintained.

As described above, with respect to the sequence of a site in the first region that binds to the receptor binding domain (RBD) in SARS-CoV-2 and the sequence of a site in the second region that binds to the receptor binding domain (RBD) in SARS-CoV-2, defined by the above (d), (d1), (d2) or (d3) and (g), (g1) or (g2), examples of the amino acid residues that can be substituted are described but the sequences of the site in the first region that binds to the receptor binding domain (RBD) in SARS-CoV-2 and the site in the second region that binds to the receptor binding domain (RBD) in SARS-CoV-2 are not limited to the above description.

In the peptide according to the disclosure, the sequence of a site in the first region that binds to the receptor binding domain (RBD) in SARS-CoV-2 may be an amino acid sequence obtained by substituting amino acid residues except for the aspartic acid residue at position 1, the lysine residue at position 8, the glutamic acid residue at position 11 and the glutamic acid residue at position 18 in SEQ ID NO: 1, 59 or 61 with other amino acid residues or an amino acid sequence obtained by deleting and/or inserting of 1 to 4 amino acid residues from/to the amino acid sequence of SEQ ID NO: 1, 59 or 61. More specifically, the sequence of a site in the first region that binds to the receptor binding domain (RBD) in SARS-CoV-2 is not limited to that of SEQ ID NO: 1, 59 or 61 consisting of 20 amino acid residues and may be an amino acid sequence consisting of 16 to 24 amino acid residues and preferably 18 to 22 amino acid residues.

In the peptide according to the disclosure, the sequence of a site in the second region that binds to the receptor binding domain (RBD) in SARS-CoV-2 may be an amino acid sequence obtained by substituting the amino acid residues except for the glycine residue at position 2, the glutamic acid residue at position 14 and the arginine residue at position 18 in SEQ ID NO: 2 or 57 with other amino acid residues or an amino acid sequence obtained by deleting and/or inserting of 1 to 3 amino acid residues from/to the amino acid sequence of SEQ ID NO: 2 or 57. More specifically, the sequence of a site in the second region that binds to the receptor binding domain (RBD) in SARS-CoV-2 is not limited to that of SEQ ID NO: 2 or 57 consisting of 19 amino acid residues and may be an amino acid sequence consisting of 16 to 23 amino acid residues, and preferably 18 to 22 amino acid residues.

The peptide according to the disclosure may be constituted of 36 to 44 amino acid residues as a total number of those in a first region and a second region or most preferably 39 amino acid residues in total. If the total number of amino acid residues in a first region and a second region is 39, a salt bridge can be formed between the lysine residue at the N terminal side in the first region and the aspartic acid residue at the C terminal side in the second region, and the size of the peptide becomes suitable for binding to the receptor binding domain (RBD) in SARS-CoV-2.

As described above, even if the sequence of a site in the first region that binds to the receptor binding domain (RBD) in SARS-CoV-2 is constituted of an amino acid sequence different from that of SEQ ID NO: 1, 59 or 61 and the sequence of a site in the second region that binds to the receptor binding domain (RBD) in SARS-CoV-2 is constituted of an amino acid sequence different from that of SEQ ID NO: 2 or 57, if a salt bridge is formed between an amino acid residue within 5 residues from N terminal (the lysine residue at position 2 from the N terminal in SEQ ID NO: 1) in the sequence of the site binding to the receptor binding domain (RBD) in SARS-CoV-2 and an amino acid residue within 5 residues from C terminal in (the aspartic acid residue at position 1 from the C terminal in SEQ ID NO: 2) of the sequence of the site binding to the receptor binding domain (RBD) in SARS-CoV-2, a desired three-dimensional structure can be maintained. Thus, even if the sequence of a site in the first region that binds to the receptor binding domain (RBD) in SARS-CoV-2 is constituted of an amino acid sequence different from that of SEQ ID NO: 1, 59 or 61 and the sequence of a site in the second region that binds to the receptor binding domain (RBD) in SARS-CoV-2 is constituted of an amino acid sequence different from that of SEQ ID NO: 2 or 57, the peptide according to the disclosure can bind to the receptor binding domain (RBD) in SARS-CoV-2, similarly.

Herein, the roles of individual amino acid residues of the first region consisting of the amino acid sequence of SEQ ID NO: 1 and the second region consisting of the amino acid sequence of SEQ ID NO: 2, are collectively shown in the following Tables 1 and 2.

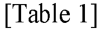

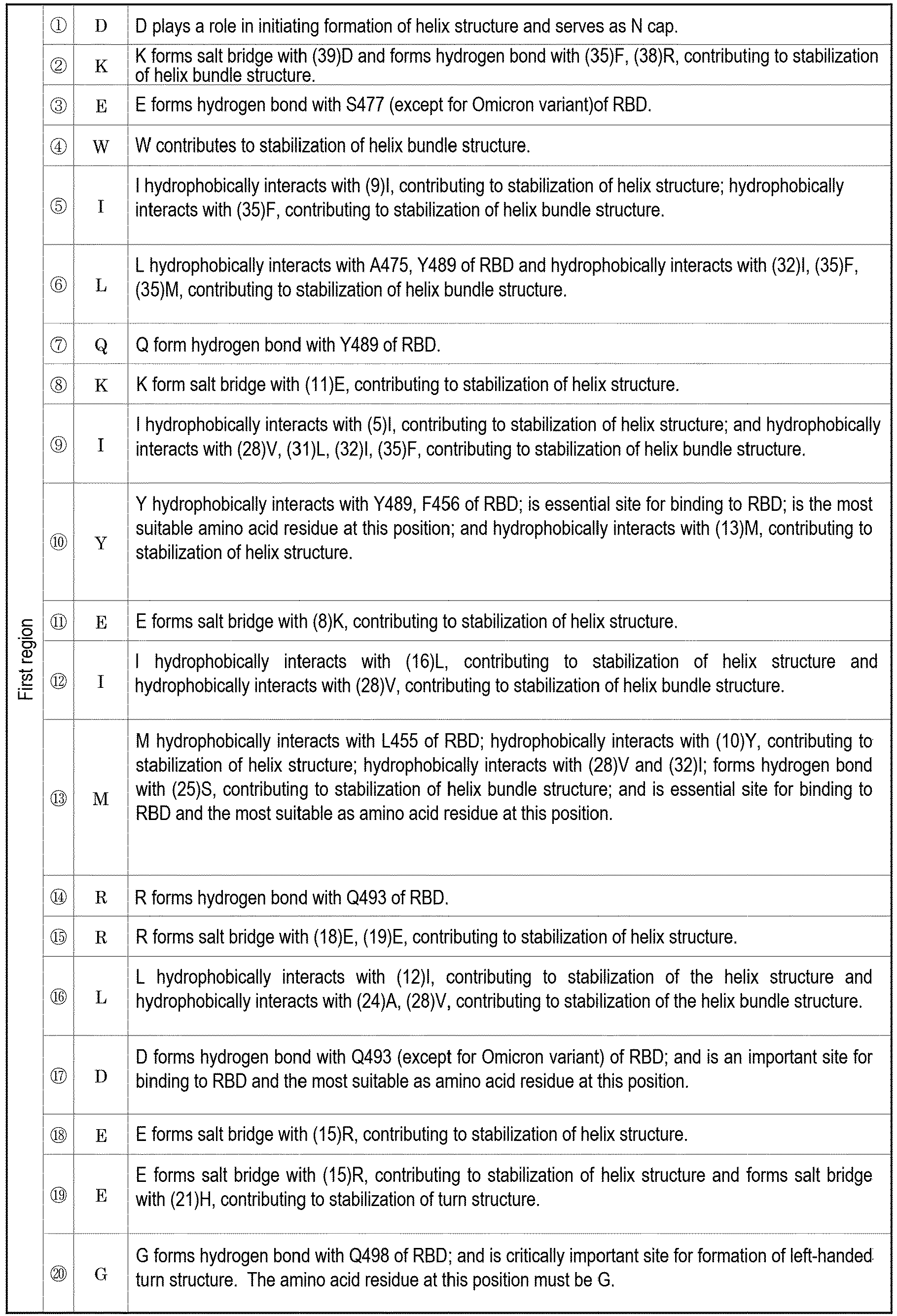

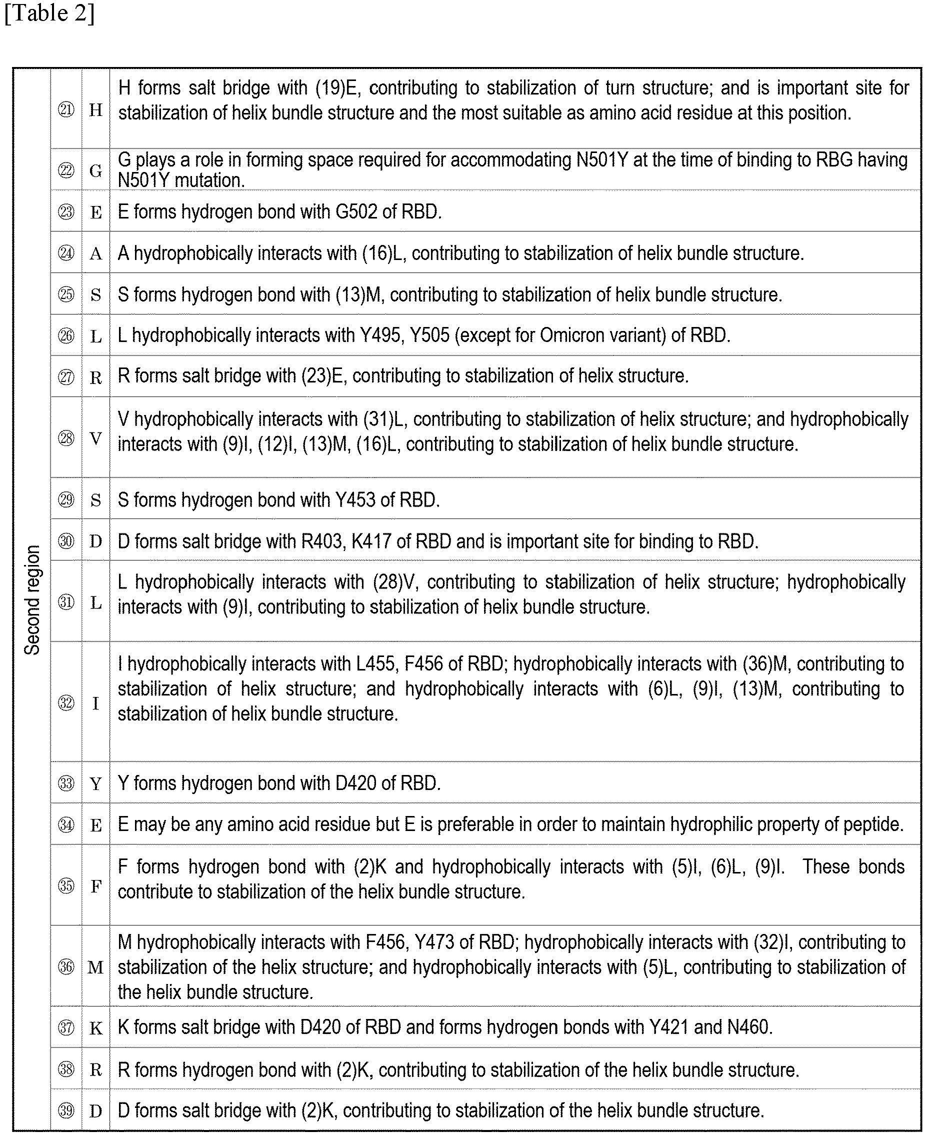

Herein, the roles of individual amino acid residues of the first region consisting of the amino acid sequence of SEQ ID NO: 59 and the second region consisting of the amino acid sequence of SEQ ID NO: 57 are collectively shown in the following Tables 3 and 4.

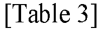

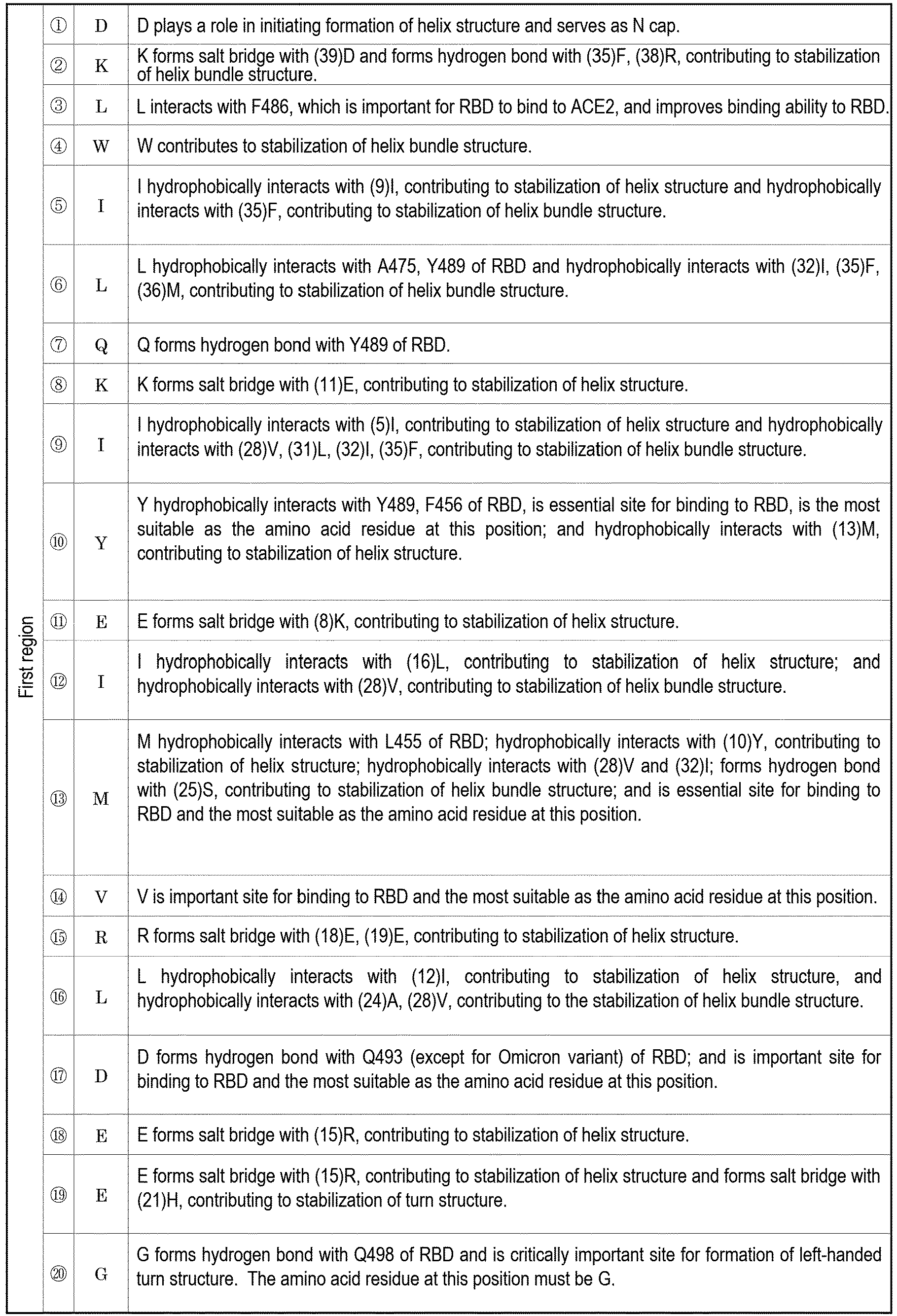

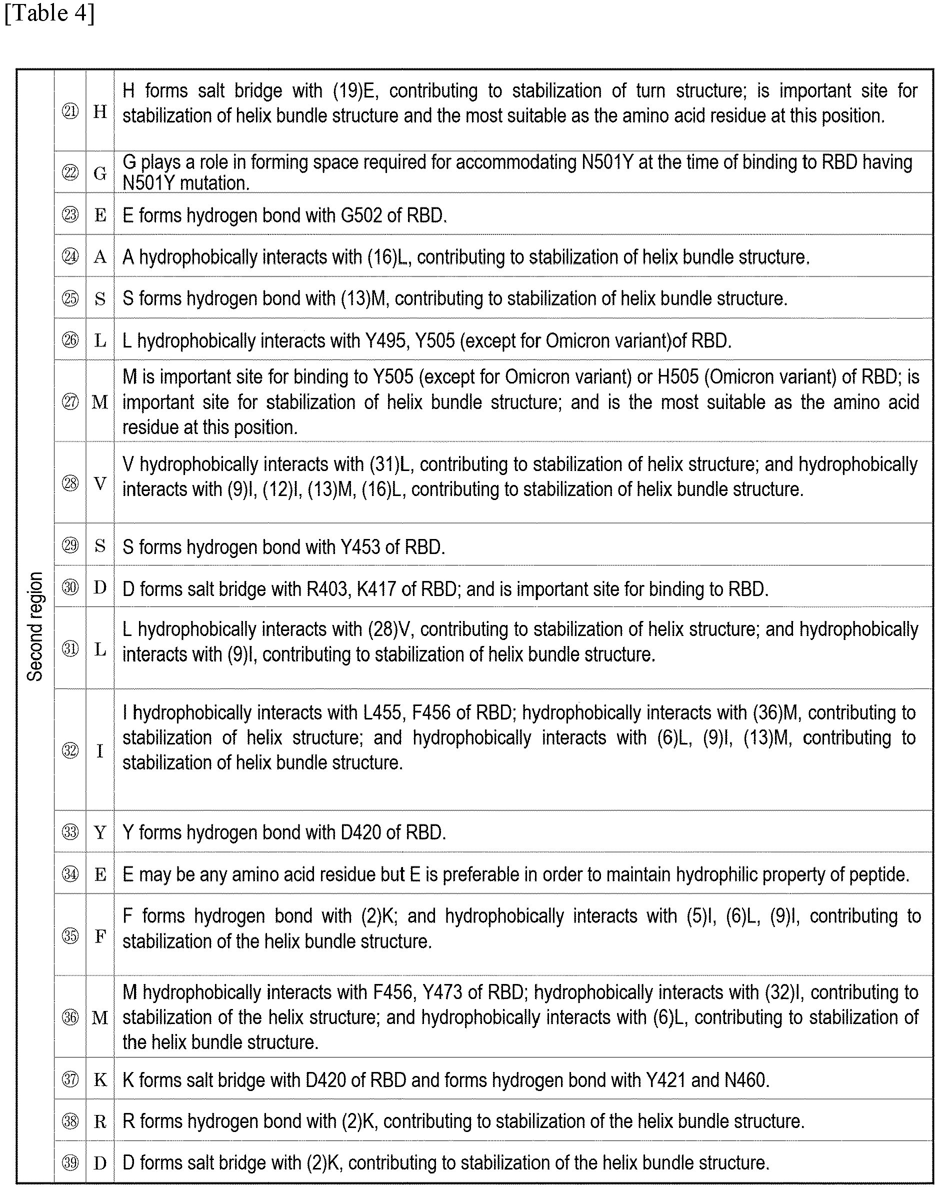

Herein, the roles of individual amino acid residues of the first region consisting of the amino acid sequence of SEQ ID NO: 61 are collectively shown in the following Table 5.

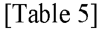

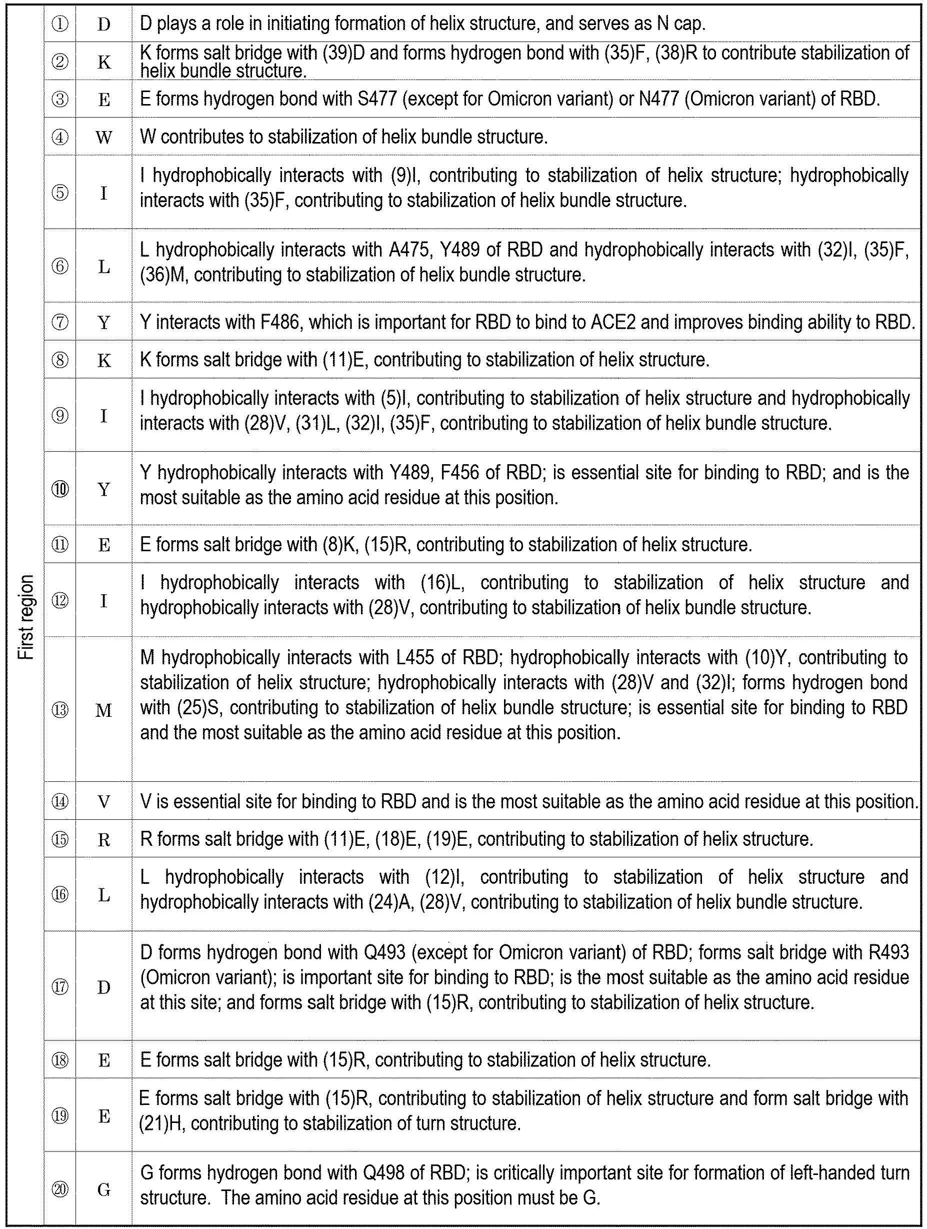

Note that, in the above Tables, for example, the expression "S477 of RBD" means the serine residue at position 477 in the amino acid sequence of the receptor binding domain (RBD) in SARS-CoV-2 (the same applies to the residue other than S477). In the above Tables, the expression "N501Y of RBD having N501Y mutation" means the tyrosine residue at position 501 in the amino acid sequence constituting the receptor binding domain (RBD) in SARS-CoV-2, having a mutation into a tyrosine residue at position 501 (in the receptor binding domain (RBD) in SARS-CoV-2).

As is apparent from the above Tables, the amino acid sequences, that is, the sequence of a site in the first region that binds to the receptor binding domain (RBD) in SARS-CoV-2 and the sequence of a site in the second region that binds to the receptor binding domain (RBD) in SARS-CoV-2, are not limited to SEQ ID NOs: 1, 59 or 61, and 2 or 57, respectively, and can appropriately change depending on the roles of individual amino acid residues.

For example, the aspartic acid residue at position 1 from the N terminal constituting the sequence of a site in the first region that binds to the receptor binding domain (RBD) in SARS-CoV-2 does not specifically interact with the RBD, but if the amino acid residue is removed, the binding ability decreases, and thus, a glutamic acid residue having an analogous chemical structure to aspartic acid can be used in place. The arginine residue at position 15 from the N terminal of the sequence of a site in the first region that binds to the receptor binding domain (RBD) in SARS-CoV-2 forms a salt bridge with the glutamic acid residue at position 19 constituting the sequence of a site in the first region that binds to the receptor binding domain (RBD) in SARS-CoV-2, thereby contributing to the stabilization of a helix structure but the same stability can be realized even if both residues are substituted with a lysine residue and an aspartic acid residue, respectively. The glycine residue at position 2 from the N terminal constituting the sequence of a site in the second region that binds to the receptor binding domain (RBD) in SARS-CoV-2 is extremely important for the receptor binding domain (RBD) containing an N501Y mutation, such as a receptor binding domain (RBD) of the SARS-CoV-2 UK variant (B.1.1.7), but substitution with an alanine residue provides further stronger binding ability to the receptor binding domain (RBD) in wild-type SARS-CoV-2. Furthermore, the glutamic acid residue at position 3 from the N terminal of the sequence of a site in the second region that binds to the receptor binding domain (RBD) in SARS-CoV-2 may be substituted with a glutamine residue. Furthermore, the leucine residue at position 6 from the N terminal of the sequence of a site in the second region that binds to the receptor binding domain (RBD) in SARS-CoV-2 may be substituted with a methionine residue. Furthermore, the methionine residue at position 16 from the N terminal of the sequence of a site in the second region that binds to the receptor binding domain (RBD) in SARS-CoV-2 may be substituted with a tryptophan residue. Moreover, the glutamic acid residue at position 3, leucine residue at position 6, and methionine residue at position 16 from the N terminal of the sequence of a site in the second region that binds to the receptor binding domain (RBD) in SARS-CoV-2 may be substituted at the same time with a glutamine residue, a methionine residue and a tryptophan residue, respectively, to obtain a peptide strongly binding to a SARS-CoV-2 variant and exhibiting antiviral activity.

However, in the sequence of a site in the first region that binds to the receptor binding domain (RBD) in SARS-CoV-2, it is preferable that the amino acid residue at position 1 from the N terminal is an aspartic acid residue. The binding activity of the receptor binding domain (RBD) in SARS-CoV-2 can be improved by employing an aspartic acid residue at position 1 from the N terminal of the sequence of a site in the first region that binds to the receptor binding domain (RBD) in SARS-CoV-2.

In the sequence of a site in the first region that binds to the receptor binding domain (RBD) in SARS-CoV-2, it is preferable that the amino acid residue at position 5 from the N terminal is an isoleucine residue. If the amino acid residue at position 5 from the N terminal of the sequence of a site in the first region that binds to the receptor binding domain (RBD) in SARS-CoV-2 is an isoleucine residue, it is possible to form stable interaction between the isoleucine residue at position 9 from the N terminal of the sequence of a site in the first region that binds to the receptor binding domain (RBD) in SARS-CoV-2 and the phenylalanine residue at position 15 from the N terminal of the sequence of a site in the second region that binds to the receptor binding domain (RBD) in SARS-CoV-2. Furthermore, the amino acid residue at position 9 from the N terminal of the sequence of a site in the first region that binds to the receptor binding domain (RBD) in SARS-CoV-2 is preferably an isoleucine residue. If the amino acid residue at position 9 from the N terminal constituting the sequence of a site in the first region that binds to the receptor binding domain (RBD) in SARS-CoV-2 is an isoleucine residue, it is possible to form stable interaction among the isoleucine at position 5 residue from the N terminal constituting the sequence of a site in the first region that binds to the receptor binding domain (RBD) in SARS-CoV-2, the valine residue at position 8, from the N terminal constituting the sequence of a site in the second region that binds to the receptor binding domain (RBD) in SARS-CoV-2, the leucine residue at position 11 from the N terminal constituting the sequence of a site in the second region that binds to the receptor binding domain (RBD) in SARS-CoV-2, the isoleucine residue at position 12 from the N terminal constituting the sequence of a site in the second region that binds to the receptor binding domain (RBD) in SARS-CoV-2, and the phenylalanine residue at position 15 from the N terminal constituting the sequence of a site in the second region that binds to the receptor binding domain (RBD) in SARS-CoV-2. Moreover, in the sequence of a site in the first region that binds to the receptor binding domain (RBD) in SARS-CoV-2, it is preferable that the amino acid residue at position 10 from the N terminal is a tyrosine residue and the amino acid residue at position 13 is a methionine residue. If the amino acid residue at position 10 from the N terminal of the sequence of a site in the first region that binds to the receptor binding domain (RBD) in SARS-CoV-2 is a tyrosine residue and the amino acid residue at position 13 is a methionine residue, high binding activity to the receptor binding domain (RBD) in SARS-CoV-2 can be realized. The binding activity to the receptor binding domain (RBD) in SARS-CoV-2 significantly decreases if even one of the two residues is substituted with an amino acid residue except for the above amino acid residues. Moreover, it is preferable that, in the sequence of a site in the first region that binds to the receptor binding domain (RBD) in SARS-CoV-2, the amino acid residue at position 12 from the N terminal is an isoleucine residue. If the amino acid residue at position 12 from the N terminal of the sequence of a site in the first region that binds to the receptor binding domain (RBD) in SARS-CoV-2 is an isoleucine residue, stable interaction can be formed between the leucine residue at position 16 from the N terminal of the sequence of a site in the first region that binds to the receptor binding domain (RBD) in SARS-CoV-2 and the valine residue at position 8 from the N terminal of the sequence of a site in the second region that binds to the receptor binding domain (RBD) in SARS-CoV-2. Moreover, it is preferable that in the sequence of a site in the first region that binds to the receptor binding domain (RBD) in SARS-CoV-2, the amino acid residue at position 16 from the N terminal is a leucine residue. If the amino acid residue at position 16 from the N terminal of the sequence of a site in the first region that binds to the receptor binding domain (RBD) in SARS-CoV-2 is a leucine residue, stable interaction can be formed among the isoleucine residue at position 12 from the N terminal of the sequence of a site in the first region that binds to the receptor binding domain (RBD) in SARS-CoV-2, the alanine residue at position 4 from the N terminal of the sequence of a site in the second region that binds to the receptor binding domain (RBD) in SARS-CoV-2, and the valine residue at position 8 from the N terminal of the sequence of a site in the second region that binds to the receptor binding domain (RBD) in SARS-CoV-2.

In the sequence of a site in the second region that binds to the receptor binding domain (RBD) in SARS-CoV-2, it is preferable that the residue at position 1 from the N terminal is a histidine residue. If the residue at position 1 from the N terminal of the sequence of a site in the second region that binds to the receptor binding domain (RBD) in SARS-CoV-2 is a histidine residue as, a helix bundle structure can be stabilized, at the same time, a salt bridge is formed with the glutamic acid residue at position 19 in the sequence of a site in the first region that binds to the receptor binding domain (RBD) in SARS-CoV-2, with the result that a turn structure can be stabilized.

In the sequence of a site in the second region that binds to the receptor binding domain (RBD) in SARS-CoV-2, it is preferable that the amino acid residue at position 10 from the N terminal is an aspartic acid residue. If the amino acid residue at position 10 from the N terminal of the sequence of a site in the second region that binds to the receptor binding domain (RBD) in SARS-CoV-2 is an aspartic acid residue, a salt bridge can be formed between the arginine residue at position 403 and the lysine residue at position 417 in the amino acid sequence of the receptor binding domain (RBD) in SARS-CoV-2. If the amino acid residue at position 10 from the N terminal of the sequence of a site in the second region that binds to the receptor binding domain (RBD) in SARS-CoV-2 is a glutamic acid residue, a salt bridge among the glutamic acid residue, the arginine residue at position 403 and the lysine residue at position 417 in the amino acid sequence of the receptor binding domain (RBD) in SARS-CoV-2 cannot be formed and the binding activity to the receptor binding domain (RBD) in SARS-CoV-2 may decrease.

The amino acid residue at position 13 from the N terminal of the sequence of a site in the second region that binds to the receptor binding domain (RBD) in SARS-CoV-2 is preferably a tyrosine residue. If the amino acid residue at position 13 from the N terminal of the sequence of a site in the second region that binds to the receptor binding domain (RBD) in SARS-CoV-2 is a tyrosine residue, it is possible to form a hydrogen bond with the aspartic acid residue at position 420 in the amino acid sequence of the receptor binding domain (RBD) in SARS-CoV-2.

Furthermore, in the sequence of a site in the second region that binds to the receptor binding domain (RBD) in SARS-CoV-2, the amino acid residue at position 4 from the N terminal is preferably an alanine residue. If the amino acid residue at position 4 from the N terminal of the sequence of a site in the second region that binds to the receptor binding domain (RBD) in SARS-CoV-2 is an alanine residue, a stable interaction can be formed between the alanine residue and the leucine residue at position 16 from the N terminal of the sequence of a site in the first region that binds to the receptor binding domain (RBD) in SARS-CoV-2. Furthermore, in the sequence of a site in the second region that binds to the receptor binding domain (RBD) in SARS-CoV-2, the amino acid residue at position 8 from the N terminal is preferably a valine residue. If the amino acid residue at position 8 from the N terminal of the sequence of a site in the second region that binds to the receptor binding domain (RBD) in SARS-CoV-2 is a valine residue, it is possible to form a stable interaction among the isoleucine residue at position 9 from the N terminal of the sequence of a site in the first region that binds to the receptor binding domain (RBD) in SARS-CoV-2, the isoleucine residue at position 12 from the N terminal of the sequence of a site in the first region that binds to the receptor binding domain (RBD) in SARS-CoV-2, the methionine residue at position 13 from the N terminal of the sequence of a site in the first region that binds to the receptor binding domain (RBD) in SARS-CoV-2, and the leucine residue at position 16 from the N terminal of the sequence of a site in the first region that binds to the receptor binding domain (RBD) in SARS-CoV-2. Moreover, in the sequence of a site in the second region that binds to the receptor binding domain (RBD) in SARS-CoV-2, the amino acid residue at position 11 from the N terminal is preferably a leucine residue. If the amino acid residue at position 11 from the N terminal of the sequence of a site in the second region that binds to the receptor binding domain (RBD) in SARS-CoV-2 is leucine residue, it is possible to form a stable interaction between the isoleucine residue at position 9 from the N terminal of the sequence of a site in the first region that binds to the receptor binding domain (RBD) in SARS-CoV-2 and the valine residue at position 8 from the N terminal of the sequence of a site in the second region that binds to the receptor binding domain (RBD) in SARS-CoV-2. Moreover, in the sequence of a site in the second region that binds to the receptor binding domain (RBD) in SARS-CoV-2, the amino acid residue at position 15 from the N terminal is preferably a phenylalanine residue. If the amino acid residue at position 15 from the N terminal of the sequence of a site in the second region that binds to the receptor binding domain (RBD) in SARS-CoV-2 is a phenylalanine residue, it is possible to form a stable interaction among the isoleucine residue at position 5 from the N terminal of the sequence of a site in the first region that binds to the receptor binding domain (RBD) in SARS-CoV-2, the leucine residue at position 6 from the N terminal of the sequence of a site in the first region that binds to the receptor binding domain (RBD) in SARS-CoV-2, and the isoleucine residue at position 9 from the N terminal of the sequence of a site in the first region that binds to the receptor binding domain (RBD) in SARS-CoV-2.

In addition to these, the amino acid residue at position 14 from the N terminal of the sequence of a site in the second region that binds to the receptor binding domain (RBD) in SARS-CoV-2 may be any amino acid residue but preferably a glutamic acid residue. If the amino acid residue at position 14 from the N terminal of the sequence of a site in the second region that binds to the receptor binding domain (RBD) in SARS-CoV-2 is a glutamic acid residue, it is possible to maintain the hydrophilic property of the peptide can be maintained at a high level.

Furthermore, the amino acid residue at position 17 from the N terminal of the sequence of a site in the second region that binds to the receptor binding domain (RBD) in SARS-CoV-2 is preferably a lysine residue. If the amino acid residue at position 17 from the N terminal of the sequence of a site in the second region that binds to the receptor binding domain (RBD) in SARS-CoV-2 is a lysine residue, a salt bridge can be formed with the aspartic acid residue at position 420 in the amino acid sequence of the receptor binding domain (RBD) in SARS-CoV-2 and a hydrogen bond can be formed between the tyrosine residue at position 421 and the asparagine residue at position 460.

Moreover, the amino acid residue at position 18 from the N terminal of the sequence of a site in the second region that binds to the receptor binding domain (RBD) in SARS-CoV-2 may be any amino acid residue but is preferably an arginine residue. If the amino acid residue at position 18 from the N terminal of the second helix is an arginine residue, it is possible to maintain the hydrophilic property of the peptide at a high level, form a hydrogen bond with the lysine residue at position 2 from the N terminal of the first helix, contributing to the stabilization of a helix bundle structure.

Note that, also if the arginine residue at position 18 is substituted with a lysine residue, it is possible to maintain the hydrophilic property of the peptide at a high level.

In the meantime, one of the characteristics of the peptide according to the disclosure is that the position 15 in the amino acid sequence of SEQ ID NOs: 1, 59 or 61 is an arginine residue and the position 19 is a glutamic acid residue. A salt bridge formed between the arginine residue at position 15 and the glutamic acid residue at position 19 in the amino acid sequence of SEQ ID NO: 1, 59 or 61, contributes to stabilization of the structure of the first helix. In this manner, the binding rate of the peptide according to the disclosure to the receptor binding domain (RBD) in SARS-CoV-2 can be improved.

In the peptide according to the disclosure, the arginine residue at position 15 and the glutamic acid residue at position 19 in the amino acid sequence of SEQ ID NO: 1, 59 or 61 may be substituted with a lysine residue and an aspartic acid residue, respectively. In other words, in the peptide according to the disclosure, as the amino acid residue at position 15 and the amino acid residue at position 19 in the amino acid sequence of SEQ ID NO: 1, 59 or 61, a lysine residue and an aspartic acid residue may be used in a combination or an arginine residue and a glutamic acid residue may be used in combination, particularly, an arginine residue and a glutamic acid residue are desirably used in combination. Even if the amino acid residue at position 15 in the amino acid sequence of SEQ ID NO: 1, 59 or 61 is a lysine residue, and the amino acid residue at position 19 is an aspartic acid residue, a salt bridge can be formed between the lysine residue and the aspartic acid residue, contributing stabilization of a first helix structure. Particularly, if the amino acid residue at position 15 in SEQ ID NO: 1, 59 or 61 is an arginine residue and the amino acid residue at position 19 is a glutamic acid residue, the binding rate of the peptide according to the disclosure to the receptor binding domain (RBD) in SARS-CoV-2 can be further improved, compared to the case where the amino acid residue at position 15 in SEQ ID NO: 1, 59 or 61 is a lysine residue and the amino acid residue at position 19 is an aspartic acid residue.

In the meantime, one of the characteristics of the peptide according to the disclosure is that the amino acid residue at position 2 in the amino acid sequence of SEQ ID NO: 2 or 57 is a glycine residue. The peptide according to the disclosure, since the amino acid residue at position 2 in the amino acid sequence of SEQ ID NO: 2 or 57 is a glycine residue, has excellent binding activity to the receptor binding domain (RBD) in both wild-type SARS-CoV-2 and a SARS-CoV-2 variant.

Wild-type SARS-CoV-2 herein refers to SARS-CoV-2 having a receptor binding domain (RBD) constituted of the amino acid sequence wherein the position 339 is a glycine residue, the position 371 is a serine residue, the position 373 is a serine residue, the position 375 is a serine residue, the position 376 is a threonine residue, the position 405 is an aspartic acid residue, the position 408 is an arginine residue, the position 417 is a lysine residue, the position 440 is an asparagine residue, the position 446 is a glycine residue, the position 452 is a leucine residue, the position 477 is a serine residue, the position 478 is a threonine residue, the position 484 is a glutamic acid residue, the position 493 is a glutamine residue, the position 496 is a glycine residue, the position 498 is a glutamine residue, the position 501 is an asparagine residue, and the position 505 is a tyrosine residue. In contrast, a SARS-CoV-2 variant refers to SARS-CoV-2 having a receptor binding domain (RBD) constituted of the amino acid sequence which has at least one mutation selected from the group consisting of a mutation into an aspartic acid residue at position 339, a mutation into a leucine residue or a phenylalanine residue at position 371, a mutation into a proline residue at position 373, a mutation into a phenylalanine residue at position 375, a mutation into an alanine residue at position 376, a mutation into an asparagine residue at position 405, a mutation into a serine residue at position 408, a mutation into an asparagine residue or threonine residue at position 417, a mutation into a lysine residue at position 440, a mutation into a serine residue at position 446, a mutation into an arginine residue at position 452, a mutation into an asparagine residue at position 477, a mutation into a lysine residue at position 478, a mutation into a lysine residue, a glutamine residue or an alanine residue at position 484, a mutation into an arginine residue at position 493, a mutation into a serine residue at position 496, a mutation into an arginine residue at position 498, a mutation into a tyrosine residue at position 501, and a mutation into a histidine residue at position 505. More specifically, examples of the SARS-CoV-2 variant include SARS-CoV-2 (PANGO lineage: B.1.1.7, sometimes referred to commonly as SARS-CoV-2 UK variant) having a mutation into a tyrosine residue at position 501 and wild-type amino acid residues at positions 339, 371, 373, 375, 376, 405, 408, 417, 440, 446, 452, 477, 478, 484, 493, 496, 498 and 505 in the amino acid sequence constituting the receptor binding domain (RBD); SARS-CoV-2 (PANGO lineage: B.1.351, sometimes referred to commonly as SARS-CoV-2 South African variant), having a mutation into an asparagine residue at position 417, a mutation into a lysine residue at position 484 and a mutation into a tyrosine residue at position 501 and wild-type amino acid residues at positions 339, 371, 373, 375, 376, 405, 408, 440, 446, 452, 477, 478, 493, 496, 498 and 505 in the amino acid sequence constituting the receptor binding domain (RBD); SARS-CoV-2 (PANGO lineage: P.1, sometimes referred to commonly as SARS-CoV-2 Brazilian variant) having a mutation into a threonine at position 417 and a mutation into a lysine residue at position 484 and a mutation into a tyrosine residue at position 501 and wild-type amino acid residues at positions 339, 371, 373, 375, 376, 405, 408, 440, 446, 452, 477, 478, 493, 496, 498 and 505 in the amino acid sequence constituting the receptor binding domain (RBD); SARS-CoV-2 (PANGO lineage: B.1.617.2, sometimes referred to commonly as SARS-CoV-2 Indian variant (δ type)) having a mutation into an arginine residue at position 452 and a mutation into a lysine residue at position 478, and wild-type amino acid residues at positions 339, 371, 373, 375, 376, 405, 408, 417, 440, 446, 477, 484, 493, 496, 498, 501 and 505 in the amino acid sequence constituting the receptor binding domain (RBD); SARS-CoV-2 (PANGO lineage: B.1.617.1, sometimes referred to commonly as SARS-CoV-2 Indian variant (κ type)) having a mutation into an arginine residue at position 452 and a glutamine residue at position 484, and wild-type amino acid residues at positions 339, 371, 373, 375, 376, 405, 408, 417, 440, 446, 477, 478, 493, 496, 498, 501 and 505 in the amino acid sequence constituting the receptor binding domain (RBD); SARS-CoV-2 (PANGO lineage: B.1.1.529BA.1, sometimes referred to commonly as a SARS-CoV-2 Omicron variant (sublineage BA.1)) having a mutation into an aspartic acid residue at position 339, a mutation into a leucine residue at position 371, a mutation into a proline residue at position 373, a mutation into a phenylalanine residue at position 375, a mutation into an asparagine residue at position 417, a lysine residue at position 440, a mutation into a serine residue at position 446, a mutation into an asparagine residue at position 447, a mutation into a lysine residue at position 478, a mutation into an alanine residue at position 484, a mutation into an alanine residue at position 493, a mutation into a serine residue at position 496, a mutation into an arginine residue at position 498, a mutation into a tyrosine residue at position 501 and a mutation into a histidine residue at position 505, and wild-type amino acid residues at positions 376, 405, 408 and 452 in the amino acid sequence constituting the receptor binding domain (RBD); and SARS-CoV-2 (PANGO lineage: B.1.1.529/BA.2, sometimes referred to commonly as a SARS-CoV-2 Omicron variant (sublineage BA.2)) having a mutation into an aspartic acid residue at position 339, a mutation into a phenylalanine residue at position 371, a mutation into a proline residue at position 373, a mutation into a phenylalanine residue at position 375, a mutation into an alanine residue at position 376, a mutation into an asparagine residue at position 405, a mutation into a serine residue at position 408, a mutation into an asparagine residue at position 417, a mutation into a lysine residue at position 440, a mutation into an asparagine residue at position 477, a mutation into a lysine residue at position 478, a mutation into an alanine residue at position 484, a mutation into an arginine residue at position 493, a mutation into an arginine residue at position 498, a mutation into a tyrosine residue at position 501 and a mutation into a histidine residue at position 505, and wild-type amino acid residues at positions 446, 452 and 496 in the amino acid sequence constituting the receptor binding domain (RBD).

Note that, the sequence of a site in the second region that binds to the receptor binding domain (RBD) in SARS-CoV-2 in the peptide according to the disclosure, may be an amino acid sequence obtained by substituting the glycine residue at position 2 in the amino acid sequence of SEQ ID NO: 2 or 57 with an alanine residue. If the amino acid residue at position 2 in SEQ ID NO: 2 or 57 is an alanine residue, excellent binding activity to the receptor binding domain (RBD) in SARS-CoV-2, in which the amino acid residue at position 501 is the same as in wild-type SARS-CoV-2 (of the wild-type SARS-CoV-2 and SARS-CoV-2 variants), can be obtained.

In the meantime, one of the characteristics of the peptide according to the disclosure is that the position 18 in the amino acid sequence of SEQ ID NO: 2 or 57 is an arginine residue. Since the position 18 in the amino acid sequence of SEQ ID NO: 2 or 57 is an arginine residue, the peptide according to the disclosure has excellent binding activity to the receptor binding domains (RBDs) of wild-type SARS-CoV-2 and all SARS-CoV-2 variants mentioned above.

Examples of the structures of the peptides disclosed herein are shown in the following Table 6 but the technical scope of the disclosure herein is not limited to the peptides constituted of the following amino acid sequences.

**[Table 6]**

| | First region | SEQ ID NO: | Second region | SEQ ID NO: |
|---|---|---|---|---|
| Ce59 | DKEWILQKIYEIMRRLDEEG | 1 | HGEASLRVSDLIYEFMKRD | 2 |
| Ce41 | DKEWILQKIYEIMRRLDEEG | 1 | HGEASLRVSDLIYEFMKRD | 3 |
| Ce1 | DKEWILQKIYEIMRLLDELG | 4 | HAEASMRVSDLIYEFMKKD | 5 |
| Ce4 | DKEWILQKIYEIMRKLDEDG | 6 | HAEASMRVSDLIYEFMKKD | 5 |
| Ce9 | DKEWILQKIYEIMRRLDEEG | 1 | HAEASMRVSDLIYEFMKKD | 5 |
| Ce14 | DKEWALQKIYEIMRKLDEDG | 7 | HAEASMRVSDLIYEFMKKD | 5 |
| Ce15 | DKEWILQKIYEVMRKLDEDG | 8 | HAEASMRVSDLIYEFMKKD | 5 |
| Ce16 | DKEWALQKIYEIMRKLDEDG | 7 | HAEASMRASDLIYEFMKKD | 9 |
| Ce113 | DKEWILQKIYEIMQRLDEEG | 56 | HGEASLMVSDLIYEFMKRD | 57 |
| Ce172 | DKLWILQKIYEIMVRLDEEG | 59 | HGEASLMVSDLIYEFMKRD | 57 |
| Ce149 | DKEWILYKIYEIMVRLDEEG | 61 | HGEASLMVSDLIYEFMKRD | 57 |
| Ce173 | DKLWILQKIYEIMQRLDEEG | 63 | HGEASLMVSDLIYEFMKRD | 57 |
| Ce174 | DKEWILYKIYEIMQRLDEEG | 65 | HGEASLMVSDLIYEFMKRD | 57 |

In the peptide according to the disclosure, for example, the sequence of a site in the first region that binds to the receptor binding domain (RBD) in SARS-CoV-2 contains the amino acid sequence (h), (i), (j), (k) or (l):
(h) the amino acid sequence of SEQ ID NO: 56,
(i) the amino acid sequence of SEQ ID NO: 59,
(j) the amino acid sequence of SEQ ID NO: 61,
(k) the amino acid sequence of SEQ ID NO: 63, or
(l) the amino acid sequence of SEQ ID NO: 65,

The sequence of a site in the second region that binds to the receptor binding domain (RBD) in SARS-CoV-2 contains the amino acid sequence of SEQ ID NO: 57.

In the peptide according to the disclosure, the sequence of a site in the first region that binds to the receptor binding domain (RBD) in SARS-CoV-2 contains the amino acid sequence (m), (n), (o) or (p):
(m) the amino acid sequence of SEQ ID NO: 4,
(n) the amino acid sequence of SEQ ID NO: 6
(o) the amino acid sequence of SEQ ID NO: 7, or
(p) the amino acid sequence of SEQ ID NO: 8
the sequence of a site in the second region that binds to the receptor binding domain (RBD) in SARS-CoV-2 contains the amino acid sequence of SEQ ID NO: 2, 3, 5 or 9.

These peptides specifically disclosed are constituted of 2 helixes, unlike LCB1 peptide (SEQ ID NO: 10: DKEWILQKIYEIMRLLDELGHAEASMRVSDLIYEFMKKGDERLLEEAERLLEEVER (having 3 helixes)) disclosed in L Cao et al., Science 370, 426-431 (2020).

The full-length amino acid sequences of these peptides specifically disclosed include peptides consisting of one amino acid sequence selected from the group consisting of SEQ ID NOs: 52 to 55, 58, 60, 62, 64 and 66:
SEQ ID NO: 52: DKEWILQKIYEIMRKLDEDGHAEASMRVSDLIYEFMKKD
SEQ ID NO: 53: DKEWILQKIYEIMRRLDEEGHAEASMRVSDLIYEFMKKD
SEQ ID NO: 54: DKEWILQKIYEIMRRLDEEGHGEASLRVSDLIYEFMKKD
SEQ ID NO: 55: DKEWILQKIYEIMRRLDEEGHGEASLRVSDLIYEFMKRD
SEQ ID NO: 58: DKEWIIQKIYEIMQRLDEEGHGEASLMVSDLIYEFMKRD
SEQ ID NO: 60: DKLWIIQKIYEIMVRLDEEGHGEASLMVSDLIYEFMKRD
SEQ ID NO: 62: DKEWIIYKIYEIMVRLDEEGHGEASLMVSDLIYEFMKRD
SEQ ID NO: 64: DKLWIIQKIYEIMQRLDEEGHGEASLMVSDLIYEFMKRD
SEQ ID NO: 66: DKEWIIYKIYEIMQRLDEEGHGEASLMVSDLIYEFMKRD

It has been found that these peptides specifically disclosed exhibit excellent binding activity to the receptor binding domain (RBD) in SARS-CoV-2, as shown in Examples. The binding activity can be comprehensively determined on the basis of the binding rate and the degree of dissociation. The system for evaluating the binding activity of a predetermined peptide to the receptor binding domain (RBD) in SARS-CoV-2 is not particularly limited but a system using surface plasmon resonance (SPR) can be used. The binding activity can be determined, for example, by immobilizing the receptor binding domain (RBD) in SARS-CoV-2 to the surface of a sensor chip, bringing a sample containing various analytes (peptides) into contact with the surface and measuring a change in SPR angle due to binding of the receptor binding domain (RBD) in SARS-CoV-2 and a peptide. The degree of dissociation can be determined by supplying a buffer containing no analytes to the sensor-chip surface on which the receptor binding domain (RBD) in SARS-CoV-2 and the peptide are present in a mutually binding state, and measuring a change in SPR angle due to dissociation of the receptor binding domain (RBD) in SARS-CoV-2 and the peptide.

Note that, the binding rate and degree of dissociation of a peptide and the receptor binding domain (RBD) in SARS-CoV-2 may be measured not only by the system using surface plasmon resonance (SPR) but also by a system for analyzing the interaction of biomolecules, such as biolayer interferometry (BLI).

The peptide according to the disclosure is not limited as long as it has excellent binding activity to the receptor binding domain (RBD) in SARS-CoV-2. The peptide may be a peptide having 1 or more amino acids bound to at least one of the N terminal and the C terminal; a fusion peptide, i.e., a peptide fused to another peptide or protein, a peptide having an insertion of non-natural amino acid, a peptide attached with a tag or a label and a peptide having a modification other than these. Examples of the fusion peptide include, but are not limited to, a peptide fused to a cell-penetrating peptide (CPP) in order for the peptide to be introduced into a cell when administrated. A peptide having 1 or more amino acids bound to at least one of the N terminal and the C terminal may be acceptable, a fusion peptide, i.e., a peptide fused to another peptide or protein, a peptide having an insertion of non-natural amino acid, a peptide attached with a tag or a label and a peptide having a modification other than these are included in the peptide according to the disclosure as long as they contain the peptide according to the disclosure.

### <Method for producing a peptide>

The peptide according to the disclosure, since it has a full length of about 39 amino acid residues, can be easily produced in accordance with a common chemical synthesis method. Examples of the peptide synthesis method include a solid-phase synthesis method and a liquid-phase synthesis method. Examples of the solid-phase synthesis method include a method using Boc (t-butyloxycarbonyl) or Fmoc (9-fluorenylmethoxycarbonyl) as an amino-group protecting group. Peptides mentioned above can be synthesized by a commercially available peptide synthesizer to which the solid phase synthesis method is applied.

Note that, DNA encoding a peptide as mentioned above is synthesized, introduced in a host cell and expressed in the cell to obtain the peptide. Examples of the host cells include yeasts, bacteria such as *Escherichia coli,* insect cells, animal cells and plant cells. Alternatively, a peptide as mentioned above can be synthesized *in vitro* by use of a cell-free protein synthesis system. However, the peptide according to the disclosure is preferably produced by chemical synthesis. This is because it is easy to chemically synthesize the peptide according to the disclosure since the peptide has a full length of about 39 amino acid residues; and it is easy for the manufacturers and marketing authorization holders to perform chemical synthesis in accordance with the production conditions required by GMP (Good Manufacturing Practice).

### <Pharmaceutical composition, therapeutic drug>

The peptide according to the disclosure can be used as a pharmaceutical composition. The peptide according to the disclosure has an extremely high solubility to ultra-pure water or a buffer solution and excellent binding activity to the receptor binding domain (RBD) in SARS-CoV-2, as mentioned above. Accordingly, the peptide according to the disclosure can be used as a pharmaceutical composition for treating and/or preventing Coronavirus Disease 2019 (COVID-19) caused by SARS-CoV-2. In other words, this disclosure relates to use of the peptide in producing a pharmaceutical composition for treating and/or preventing Coronavirus Disease 2019 (COVID-19).

Examples of a subject to which the pharmaceutical composition is to be administered include, but are not particularly limited to, mammals such as a human, a monkey, a mouse, a rat, a hamster, a rabbit, a guinea pig, a cow, a pig, a dog, a horse, a cat, a goat, and a sheep. Preferably a human is a subject. The route of administration of a pharmaceutical composition disclosed herein may be oral and parenteral route. The dosage of the pharmaceutical composition disclosed herein varies depending on the formulation method, administration method, administration time, the age, body weight, sex, morbid condition, excretion rate, and reaction sensitivity of the subject to be administered. The timing of administering the pharmaceutical composition disclosed herein is not particularly limited. The pharmaceutical composition may be administered at any time, i.e., before meals, after meals or between meals. The administration/intake period is not particularly limited.

The pharmaceutical composition disclosed herein can be produced by a method routinely carried out by those having common knowledge in the technical field. The pharmaceutical composition can be produced in the form of unit dosage using a pharmaceutically acceptable carrier and/or excipient or contained in a multi-dose container. The pharmaceutical composition disclosed herein can be appropriately prepared into a desired dosage form depending on the administration method. The pharmaceutical composition disclosed herein can be produced in accordance with a known method disclosed in, for example, Japanese Pharmacopoeia (JP), United States Pharmacopoeia (USP) or European Pharmacopoeia (EP). Also, an existing medicine or a future medicine (including mRNA vaccine) for treating and/or preventing COVID-19 and medicines for treating or preventing other diseases can be used in combination.

### Examples

Now, the present invention will be more specifically described with reference to Examples but the technical scope of the present invention is not limited to Examples.

### [Example 1]

In this Example, a part of LCB1 peptide (SEQ ID NO: 10: DKEWILQKIYEIMRLLDELGHAEASMRVSDLIYEFMKKGDERLLEEAERLLEEVER) disclosed in L Cao et al., Science 370, 426-431 (2020), that is, a peptide (referred to as Ca1) consisting of DKEWILQKIYEIMRLLDELGHAEASMRVSDLIYEFMKKG (SEQ ID NO: 11) was used as Comparative Example. Ca1 was modified to develop a peptide having excellent binding activity to the receptor binding domain (RBD) in wild-type SARS-CoV-2. LCB1 is a peptide having 3 helixes but Ca1 is a peptide having 2 helixes from the N terminal of LCB1. The region of Ca1 consisting of DKEWILQKIYEIMRLLDELG (SEQ ID NO: 4) is referred to as a first region, the region consisting of HAEASMRVSDLIYEFMKKG (SEQ ID NO: 12) is referred to as a second region. The first region and the second region were modified.

The amino acid sequences of Ca1 peptide and peptides designed in Examples were collectively listed in Table 7. Note that, in the amino acid sequences shown in Table 7, the amino acid residues changed from those of the amino acid sequence of Ca1 peptide were each surrounded by a frame.

**[Table 7]**

| | First region | SEQ ID NO: | Second region | SEQ ID NO: |
|---|---|---|---|---|
| Ca1 | DKEWILQKIYEIMRLLDELG | 4 | HAEASMRVSDLIYEFMKKG | 12 |
| Cb1 | DKEWILQKIYENMRLLDELG | 13 | HAEASMRVSDNIYEFMKKG | 14 |
| Cb2 | DKEWILQKIYENMRKLDEDG | 15 | HAEASMRVSDLIYEFMKKG | 12 |
| Cb3 | DKEWILQKIYEAMRKLDEDG | 16 | HAEASMRVSDAIYEFMKKG | 17 |
| Cb4 | DKEWILQKIYENMRKLDEDG | 15 | HAEASMRKSDDIYEFMKKG | 18 |
| Cb5 | DKEWILQKIYENMRNLDELG | 19 | HAEASMRVSDNIYEFMKKG | 14 |
| Cb6 | DKEWILQKIYETMRKLDEDG | 20 | HAEASMRVSDAIYEFMKKG | 17 |
| Cb7 | DKEWILQKIYETMRNLDELG | 21 | HAEASMRVSDNIYEFMKKG | 14 |
| Ce1 | DKEWILQKIYEIMRLLDELG | 4 | HAEASMRVSDLIYEFMKKD | 5 |

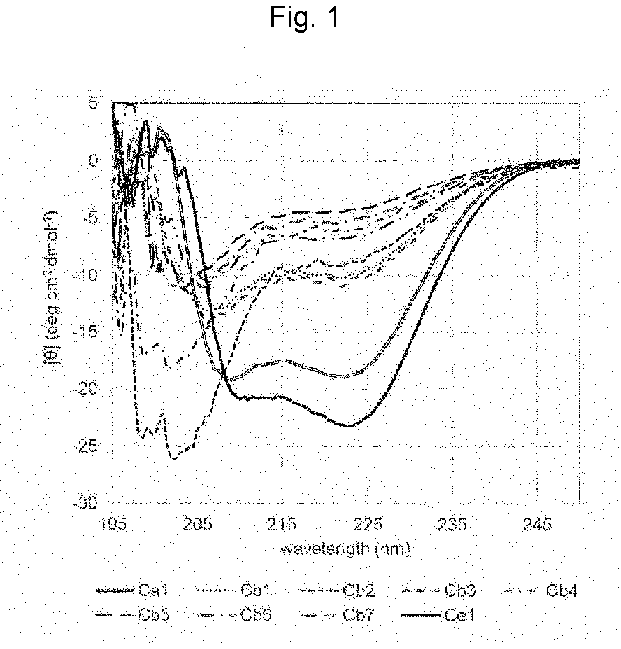

All peptides listed in Table 7 were subjected to measurement of CD spectrum in accordance with the protocol (CD spectral measurement protocol) later described in detail. The results were shown in Figure 1. As is apparent from Figure 1, Cb1, Cb2, Cb3, Cb4, Cb5, Cb6 and Cb7 are peptides in which the amino acid sequence of the first region and the amino acid sequence of the second region do not form helix structures. It was found that Ca1 has a helix structure as predicted from the results of LCB1 peptide disclosed in L Cao et al., Science 370, 426-431 (2020).

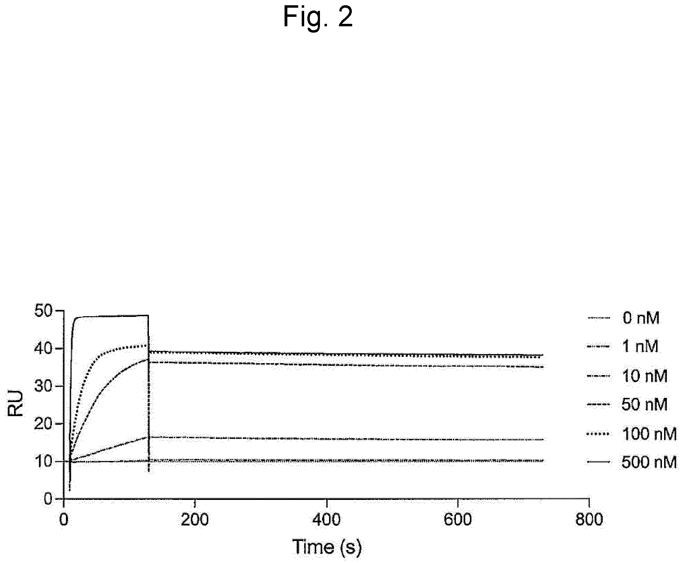

In contrast, Ce1 has an aspartic acid residue at the position in the C terminal region of the second region, which is a position at which a salt bridge can be formed with the lysine residue at the second position from the N terminal of the first region. In Ce1, it was found that a helix bundle structure is formed of the first helix contained in the first region and the second helix contained in the second region, thereby providing a more stable structure than Ca1, as shown in Figure 1. Then, the binding activity of Ce1 to the receptor binding domain RBD in wild-type SARS-CoV-2 was measured in accordance with the protocol (molecular interaction analysis protocol) later described in detail. As a result, as shown in Figure 2, it was found that Ce1 has excellent features, that is, high binding rate and a low degree of dissociation.

### [Example 2]

In this Example, Ce1 designed in Example 1 was subjected to plaque reduction assay according to the protocol (plaque reduction assay protocol) later described in detail to measure the effect of reducing infection with wild-type SARS-CoV-2. Based on the results, the IC₅₀ value thereof was calculated. For comparison, LCB1 peptide and Ca1 were subj ected to plaque reduction assay using wild-type SARS-CoV-2 and the IC₅₀ values of them were calculated.

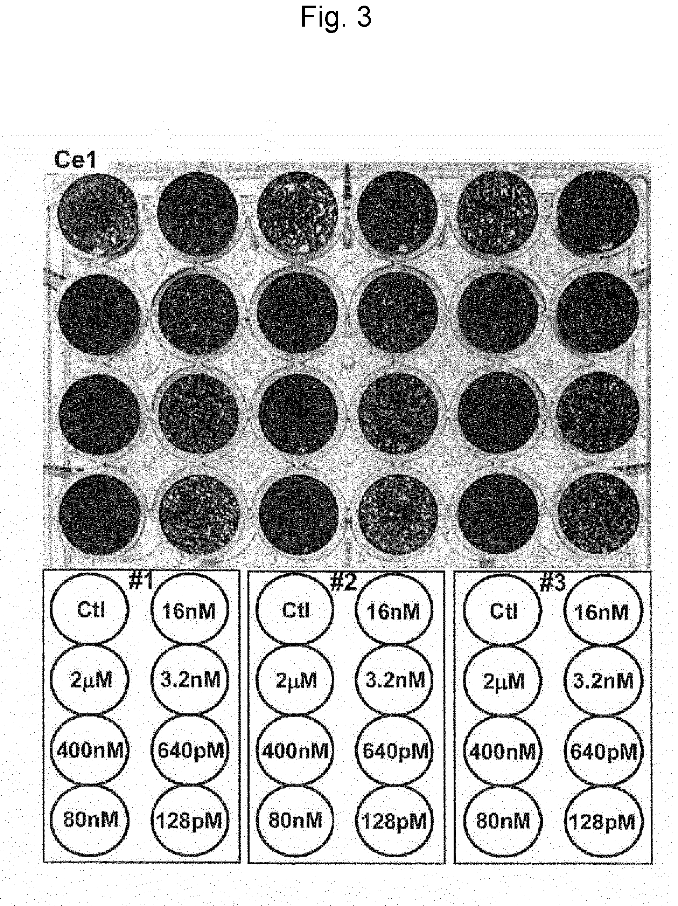

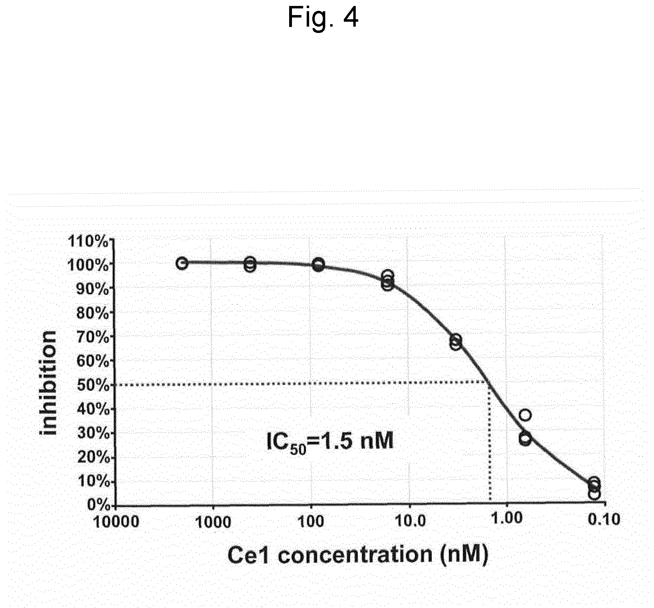

The results of the plaque reduction assay carried out for Ce1 were shown in Figure 3. The inhibition rates were calculated based on the numbers of plaques formed in individual plates. The relationship between the inhibition rate and the concentration of Ce1 peptide was shown in Figure 4. As a result of the calculation, the IC₅₀ value of Ce1 was determined as 1.5 nM.

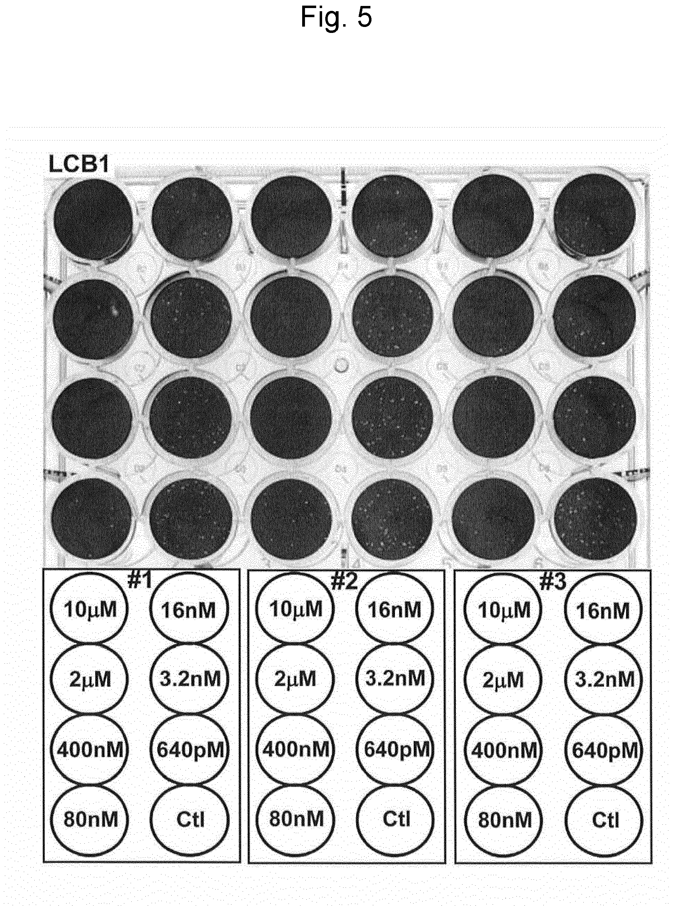

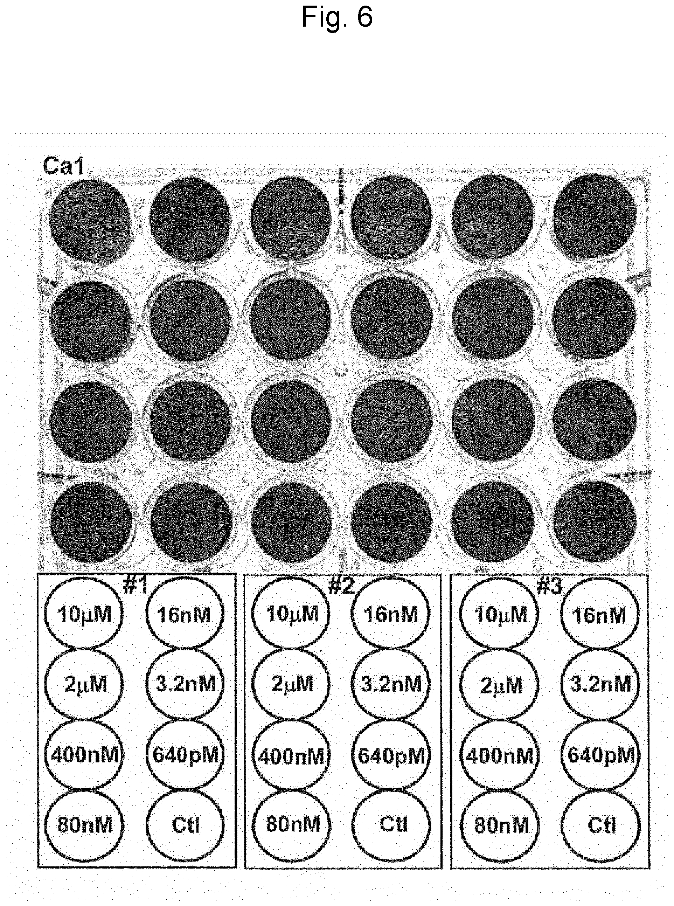

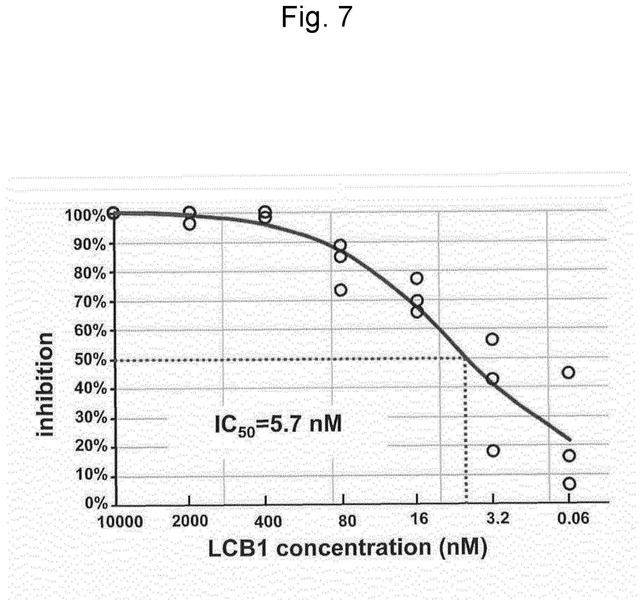

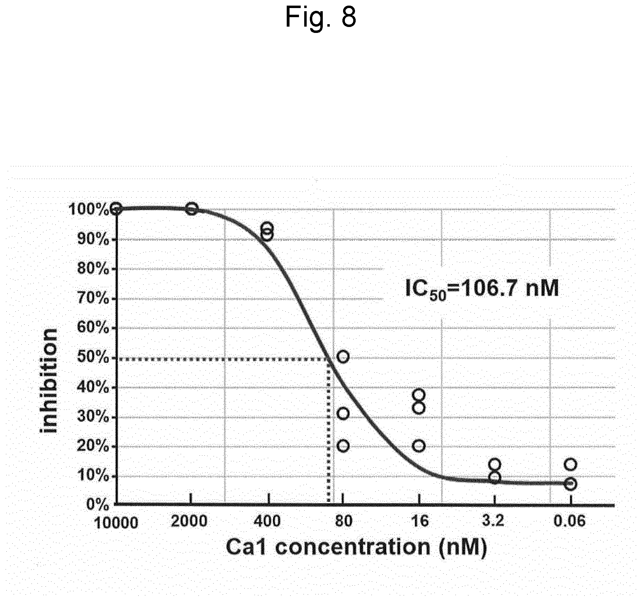

The results of the plaque reduction assay carried out for LCB1 peptide and Ca1 were shown in Figures 5 and 6, respectively, and their relationships between the inhibition rate and peptide concentration were shown in Figures 7 and 8, respectively. As a result of these calculations, the IC₅₀ values of LCB1 peptide and Ca1 were determined as 5.7 nM and 106.7 nM, respectively.

From these results, it was found that Ce1 designed in Example 1 exhibits more excellent antiviral activity against wild-type SARS-CoV-2 than LCB1 peptide and Ca1.

### [Example 3]

In this Example, Ce1 designed in Example 1 was further improved to develop a peptide having more excellent binding activity to the receptor binding domain (RBD) in wild-type SARS-CoV-2.

The amino acid sequences of the peptides designed in this Example were collectively shown in Table 8. Note that, in the amino acid sequences shown in Table 8, amino acid residues changed from those of the amino acid sequence of Ce1 peptide were each surrounded by a frame.

**[Table 8]**

| | First region | SEQ ID NO: | Second region | SEQ ID NO: |
|---|---|---|---|---|
| Ce1 | DKEWILQKIYEIMRLLDELG | 4 | HAEASMRVSDLIYEFMKKD | 5 |
| Ge2 | DREWILQKIYEIMRLLDELG | 22 | HAEASMRVSDLIYEFMKKD | 5 |
| Ce3 | DKEWILQKIYENMRKLDEDG | 15 | HAEASMRVSDLIYEFMKKD | 5 |
| Ce4 | DKEWILQKIYEIMRKLDEDG | 6 | HAEASMRVSDLIYEFMKKD | 5 |
| Ce5 | DKEWILQKIYEIMRLLDELG | 4 | HAESSMRVSDLIYEFMKKD | 23 |
| Ce6 | DKEWILQKIYEIMRLLDELG | 4 | HAEASMRVSDLIYEYMKKD | 24 |
| Ce7 | DKEWILQKIYEEMRKLDEDG | 25 | HAEASMRKSDLIYEFMKKD | 26 |
| Ce8 | DKEWILQKIYEIMRLLDELG | 4 | HAEASMRASDLIYEFMKKD | 9 |
| Ce9 | DKEWILQKIYEIMRRLDEEG | 1 | HAEASMRVSDLIYEFMKKD | 5 |
| Ce10 | DKEWILQKIYEIMRRLDELG | 27 | HAEASMRVSDLIYEFMKKD | 5 |
| Ce11 | DKEWILQRIYEIMRLLDELG | 28 | HAEASMRVSDLIYEFMKKD | 5 |
| Ce12 | DKEWILQKIYEIMRKLDEDG | 6 | HAEASMRASDLIYEFMKKD | 9 |
| Ce13 | DKEWALQKIYEVMRKLDEDG | 29 | HAEASMRASDLIYEFMKKD | 9 |
| Ce14 | DKEWALQKIYEIMRKLDEDG | 7 | HAEASMRVSDLIYEFMKKD | 5 |
| Ce15 | DKEWILQKIYEVMRKLDEDG | 8 | HAEASMRVSDLIYEFMKKD | 5 |
| Ce16 | DKEWALQKIYEIMRKLDEDG | 7 | HAEASMRASDLIYEFMKKD | 9 |
| Ce17 | DKEWILQKIYEIMRKLDEDG | 6 | HAEASMRASDLIYEFMKKD | 9 |
| Ce18 | DKEWILQKIYEVMRKLDEDG | 8 | HAEASMRASDLIYEFMKKD | 9 |

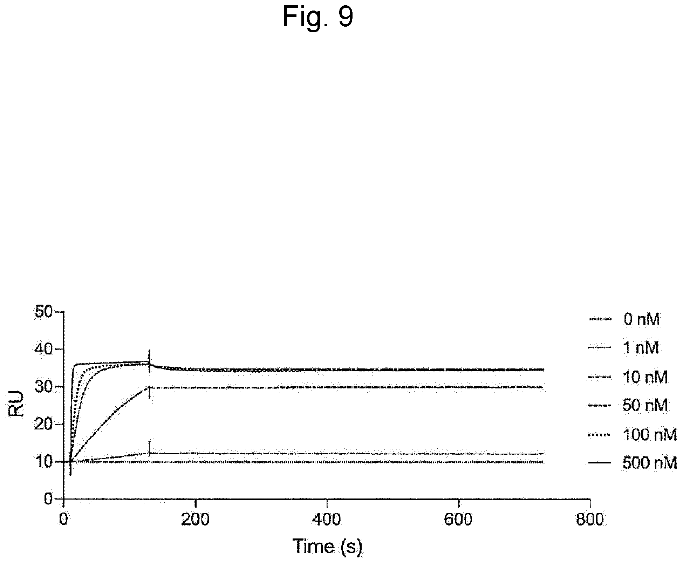

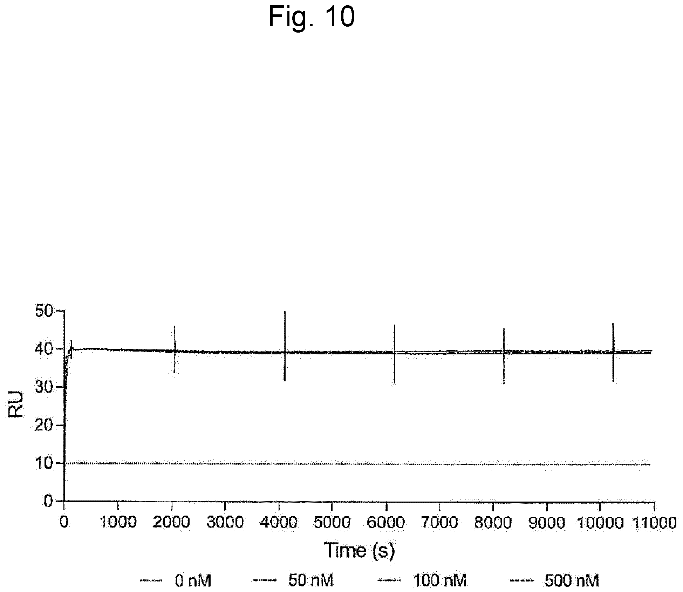

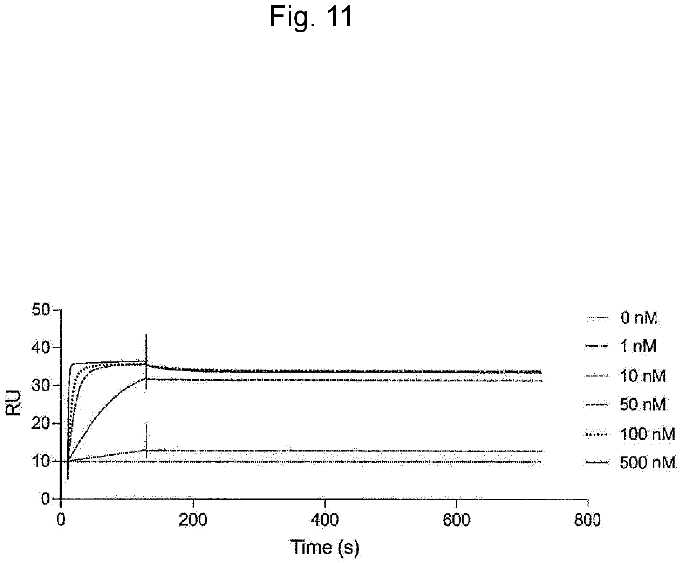

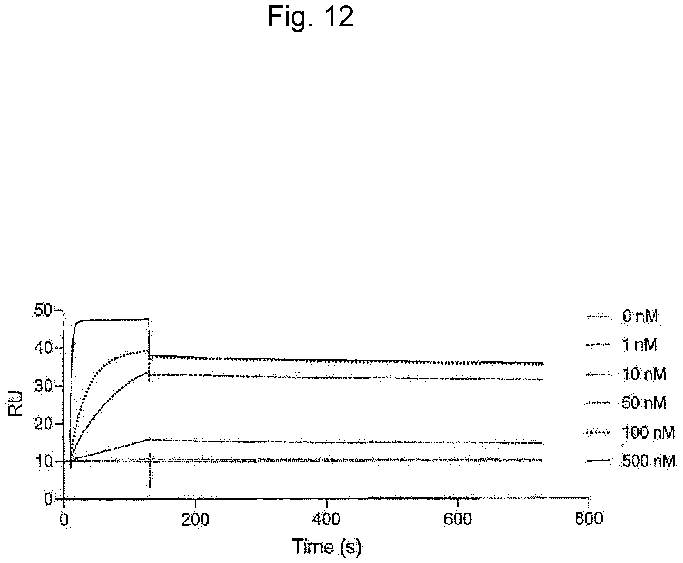

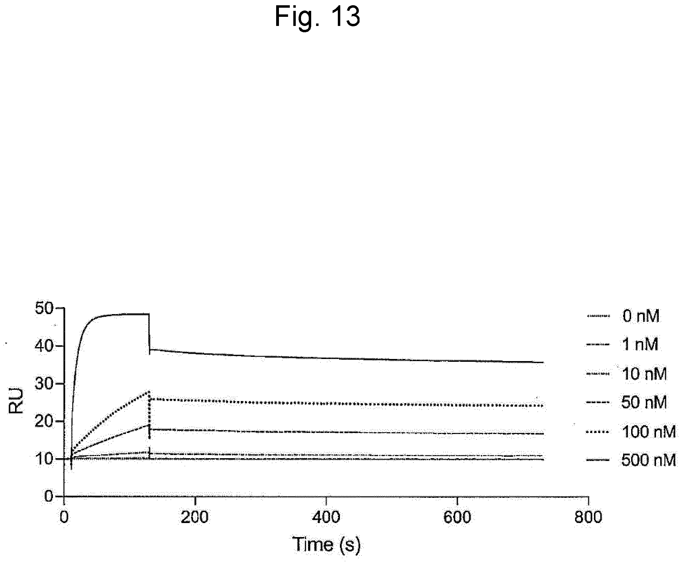

All peptides shown in Table 8 were measured for binding activity to the receptor binding domain (RBD) in wild-type SARS-CoV-2. As a result, it was found that the peptides are divided into a group of peptides (Ce4, Ce9, Ce14, Ce15 and Ce16), having higher binding activity than Ce1; a group of peptides (Ce6, Ce8, Ce10 and Ce11) having the same binding activity as Ce1; and group of peptides (Ce2, Ce3, Ce5, Ce7, Ce12, Ce13, Ce17 and Ce18) having lower binding activity than Ce1. Ce4 and Ce9 were selected as representative examples of the group having higher binding activity to the receptor binding domain (RBD) in wild-type SARS-CoV-2 than Ce1, the measurement results of the binding activity of Ce4 were shown in Figures 9 and 10, and the measurement results of the binding activity of Ce9 were shown in Figure 11. Ce6 was selected as a representative example of the group having the same binding activity to the receptor binding domain (RBD) in wild-type SARS-CoV-2 as Ce1, and the measurement results of the binding activity of Ce6 were shown in Figure 12. Ce5 was selected as a representative example of the group having a lower binding activity to the receptor binding domain (RBD) in wild-type SARS-CoV-2 as Ce1, and the measurement results of the binding activity of Ce5 were shown in Figure 13.

Note that, the pairs of the amino acid residue at position 15 and the amino acid residue at position 19 in the first helix of Ce4 and Ce9 are a pair of a lysine residue and an aspartic acid residue and a pair of an arginine residue and a glutamic acid residue, respectively. Owing to this, a salt bridge is formed between the amino acid residue at position 15 and the amino acid residue at position 19 in the first helix. Stabilization of the structure of the first helix is intended by the salt bridge. The full-length amino acid sequences of Ce4 and Ce9 were shown in SEQ ID NOs: 52 and 53, respectively.

The measurement results of the binding activity of Ce4 to the receptor binding domain (RBD) in wild-type SARS-CoV-2 were shown in Figures 9 and 10. The measurement results of the binding activity of Ce9 to the receptor binding domain (RBD) in wild-type SARS-CoV-2 were shown in Figure 11. Note that, Figures 9 and 10 show the measurement results of binding activity of Ce4 to the receptor binding domain (RBD) in wild-type SARS-CoV-2. Figure 9 shows the measurement results from the initiation of binding to 720 seconds, whereas, Figure 10 shows the measurement results from the initiation of binding to 3 hours and 2 minutes.

As is apparent from Figures 9 and 11, Ce4 and Ce9 were able to greatly improve the binding rate, compared to Ce1 (the results were shown in Figure 2). From the results, it was considered that the structure of the first region is stabilized by the formation of the salt bridge within the first region to thereby improve the binding activity to the receptor binding domain (RBD) in wild-type SARS-CoV-2. In particular, Ce9 using an arginine residue and a glutamic acid residue as the amino acid residue at position 15 and the amino acid residue at position 19 in the first region was found to have further excellent binding activity than Ce4.

From the results, it was found that the pair of the amino acid residue at position 15 and the amino acid residue at position 19 in the first region of Ce1 designed in Example 1 is preferably a pair of a lysine residue and an aspartic acid residue or a pair of the arginine residue and a glutamic acid residue, and particularly more preferably a pair of the arginine residue and a glutamic acid residue.

In particular, as shown in Figure 10, it can be found that even if Ce4 was washed with a buffer for 3 hours, it does not dissociate at all from the receptor binding domain (RBD) in wild-type SARS-CoV-2 to which Ce4 once bound. The same results were obtained in the case of Ce9. It was found that Ce4 and Ce9 prepared in the Example can maintain excellent binding activity to the receptor binding domain (RBD) in wild-type SARS-CoV-2 for a long term.

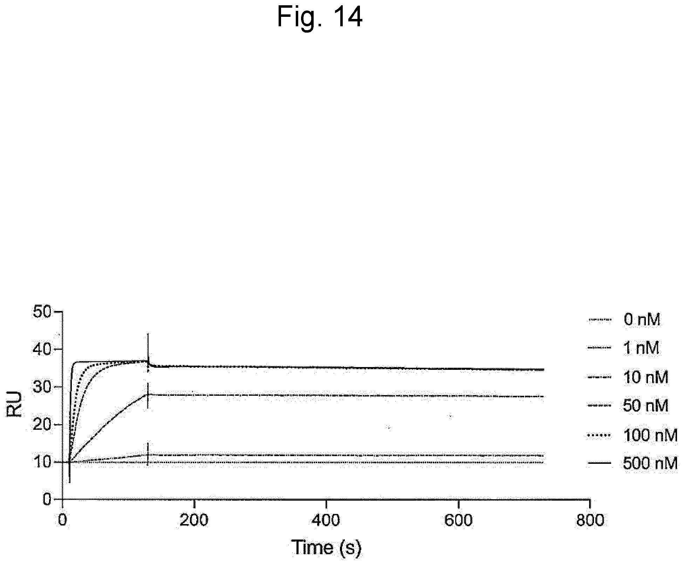

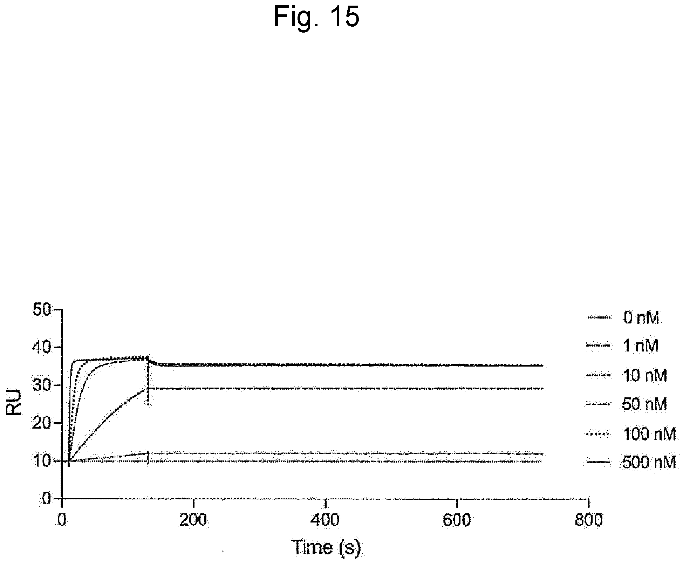

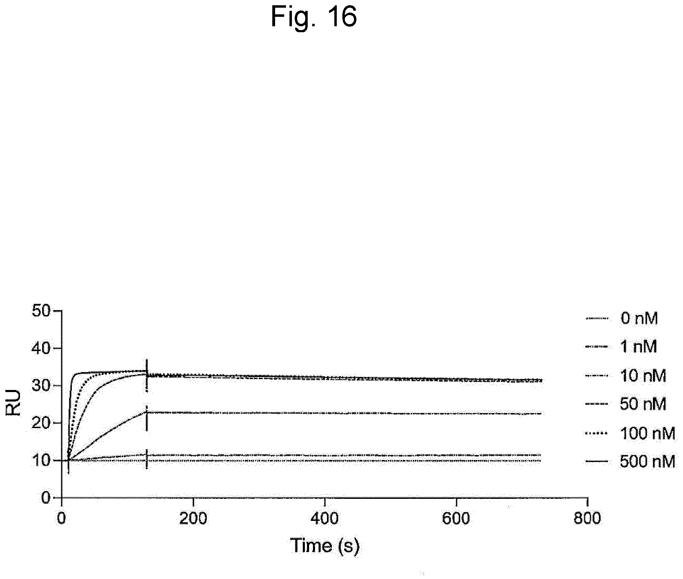

Ce14, Ce15, and Ce16 prepared in the Example were measured for the binding activity to the receptor binding domain (RBD) in wild-type SARS-CoV-2. The measurement results of them were shown in Figure 14, 15 and 16, respectively. As shown in Figures 14 to 16, it was found that Ce14, Ce15 and Ce16 have high binding activity compared to Ce1 (the results were shown in Figure 2).

### [Example 4]

In this Example, peptides having excellent binding activity to the SARS-CoV-2 variants were developed. It was found that Ce9, which shows the most excellent binding activity to the receptor binding domain (RBD) in wild-type SARS-CoV-2 in Example 3, has extremely excellent antiviral activity (IC₅₀ value: 0.4 nM) based on the results of the plaque reduction assay using wild-type SARS-CoV-2 (Figure not shown).

However, as a result of plaque reduction assay using SARS-CoV-2 variants and carried out for Ce9, IC₅₀ value of Ce9 was 13 nM for the SARS-CoV-2 UK variant (B.1.1.7) and IC₅₀ value of Ce9 was 1195 nM for the SARS-CoV-2 Brazilian variant (P.1). In short, Ce9 has extremely excellent antiviral activity specifically to wild-type SARS-CoV-2. In this Example, Ce9 was modified to develop peptides also having excellent antiviral activity against SARS-CoV-2 variants. The amino acid sequences of the peptides designed in this Example were shown in Table 9. Note that, in the amino acid sequences shown in Table 9, the amino acid residues changed from those of the amino acid sequence of the Ce9 peptide were each surrounded by a frame.

**[Table 9]**

| | First region | SEQ ID NO: | Second region | SEQ ID NO: |
|---|---|---|---|---|
| Ce9 | DKEWILQKIYEIMRRLDEEG | 1 | HAEASMRVSDLIYEFMKKD | 5 |
| Ce19 | DKEWILQKIYEIMRRLDEEG | 1 | HAEASMRVSELIYEFMKKD | 30 |
| Ce20 | DKEWILQKIYEIMRRLDEEG | 1 | HAEASMRVSELIKEFMKKD | 31 |
| Ce21 | DKEWILQKIYEIMRRLDEEG | 1 | HGEASMRVSDLIYEFMKKD | 32 |
| Ce22 | DKEWILQKIYEIMRRLDEEG | 1 | HAQASMRVSDLIYEFMKKD | 33 |
| Ce23 | DKEWILQKIYEIMRRLDEEG | 1 | HALASMRVSDLIYEFMKKD | 34 |
| Ce24 | DKEWILQKIYEIMRRLDEEG | 1 | HAEASLRVSDLIYEFMKKD | 35 |
| Ce25 | DKEWILQKIYEIMRRLDEEG | 1 | HAEASIRVSDLIYEFMKKD | 36 |
| Ce26 | DKEWILQKIYEIMRRLDEEG | 1 | HAEASMRVADLIYEFMKKD | 37 |
| Ce27 | DKEWILQKIYEIMRRLDEEG | 1 | HAEASMRVVDLIYEFMKKD | 38 |
| Ce28 | DKEWILQKIYEIMRRLDEEG | 1 | HAEASMRVLDLIYEFMKKD | 39 |
| Ce29 | DKEWILQKIYEIMRRLDEEG | 1 | HAEASMRVNDLIYEFMKKD | 40 |
| Ce30 | DKEWILQKIYEIMRRLDEEG | 1 | HAEASMRVSALIYEFMKKD | 41 |
| Ce31 | DKEWILQKIYEIMRRLDEEG | 1 | HAEASMRVSLLIYEFMKKD | 42 |
| Ce32 | DKEWILQKIYEIMRRLDEEG | 1 | HAEASMRVSMLIYEFMKKD | 43 |
| Ce33 | DKEWILQKIYEIMRRLDEEG | 1 | HAEASMRVSNLIYEFMKKD | 44 |
| Ce34 | DKEWILQKIYEIMRRLDEEG | 1 | HAEASMRVSQLIYEFMKKD | 45 |
| Ce35 | DKEWILQKIYEIMRRLDEEG | 1 | HAEASMRVSKLIYEFMKKD | 46 |
| Ce36 | DKEWILQKIYEIKRRLDEEG | 47 | HGEASMRVSDLIYEFMKKD | 32 |
| Ce37 | DKEWILQKIYEIWRRLDEEG | 48 | HGEASMRVSDLIYEFMKKD | 32 |
| Ce38 | DKEWILQKIYEIMRRLDEEG | 1 | HGEASMRVSDLIYEFKKKD | 49 |
| Ge39 | DKEWILQKIYEIMRRLDEEG | 1 | HGEASMRVSDLIYEFWKKD | 50 |
| Ce40 | DKEWILQKIYEIMRRLDEEG | 1 | HGQASMRVSDLIYEFMKKD | 51 |
| Ce41 | DKEWILQKIYEIMRRLDEEG | 1 | HGEASLRVSDLIYEFMKKD | 3 |

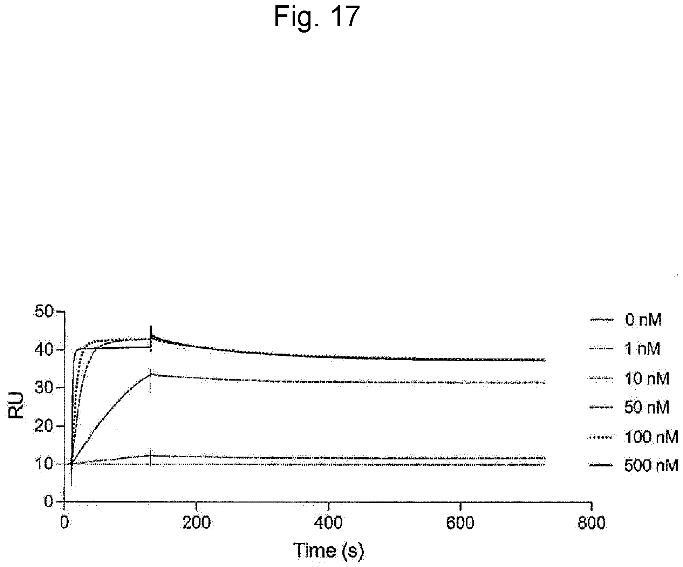

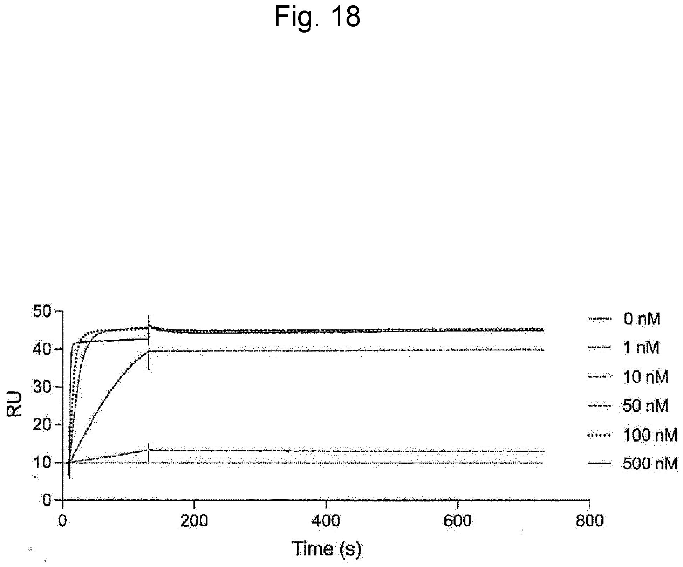

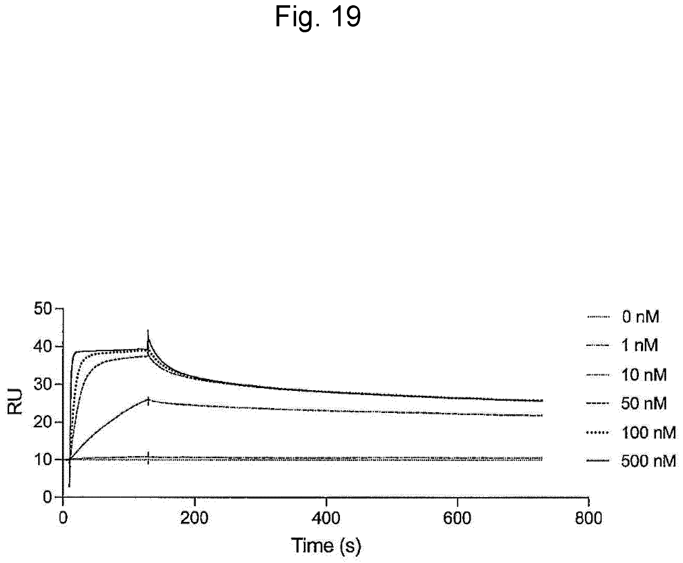

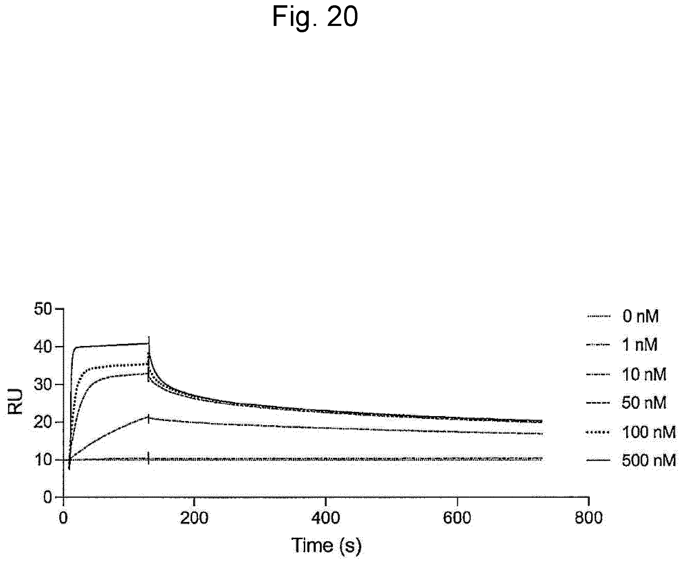

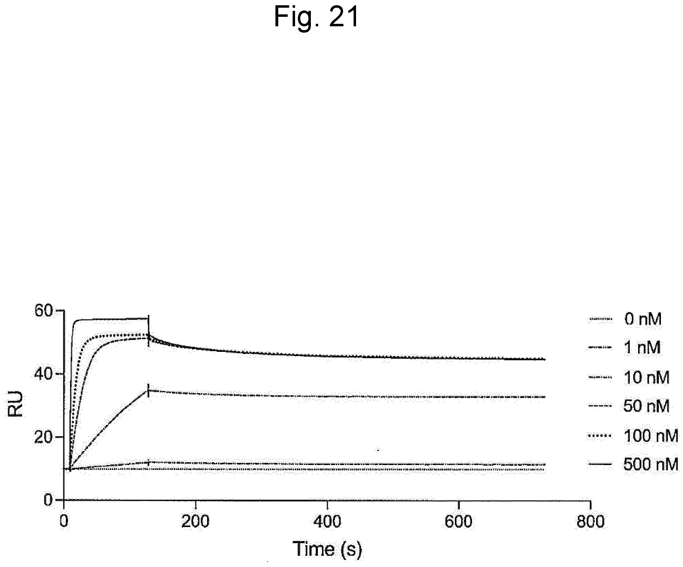

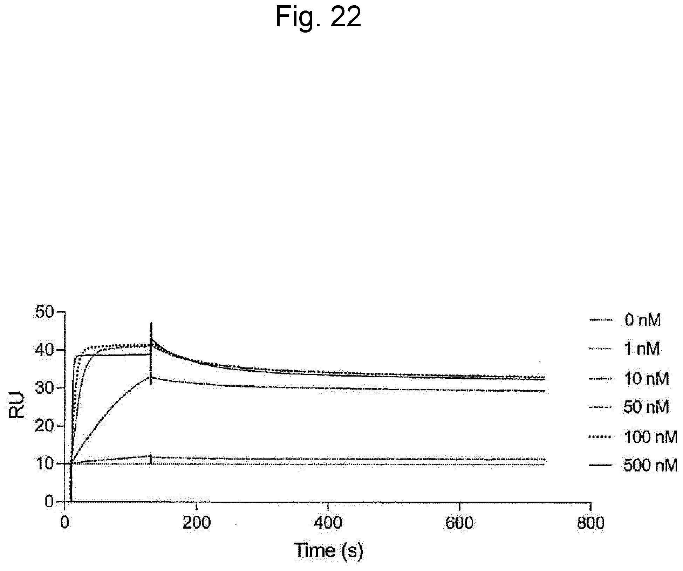

All peptides shown in Table 9 were studied. It was found that Ce41 is high binding activity to all of the receptor binding domains (RBD) of wild-type SARS-CoV-2, the SARS-CoV-2 UK variant (B.1.1.7), the SARS-CoV-2 Brazilian variant (P.1), the SARS-CoV-2 South African variant (B.1.351), the SARS-CoV-2 Indian variant (δ type (B.1.617.2)) and the SARS-CoV-2 Indian variant (κ type (B.1.617.1)). The measurement results of binding activities of Ce41 to receptor binding domains (RBD) of wild-type SARS-CoV-2, the SARS-CoV-2 UK variant (B.1.1.7), the SARS-CoV-2 Brazilian variant (P.1), the SARS-CoV-2 South African variant (B.1.351), the SARS-CoV-2 Indian variant (δ type (B.1.617.2)) and the SARS-CoV-2 Indian variant (κ type (B.1.617.1)) were shown in Figures 17, 18, 19, 20, 21 and 22, respectively. Note that, the full-length amino acid sequence of Ce41 was shown in SEQ ID NO: 54.

### [Example 5]

In this Example, Ce41 designed in Example 4 was subjected to plaque reduction assay using the SARS-CoV-2 UK variant (B.1.1.7) to measure a viral infectivity reduction effect. Based on the results, the IC₅₀ value was calculated.

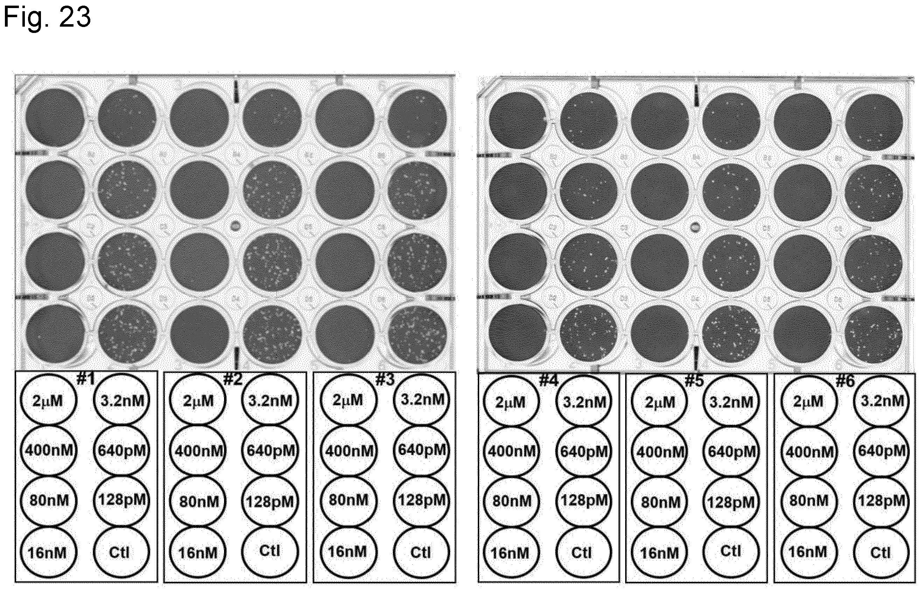

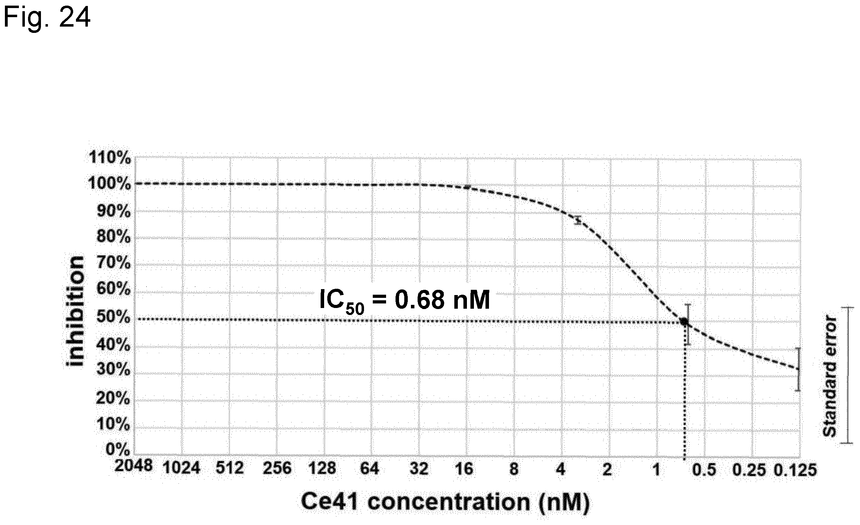

The results of the plaque reduction assay using the SARS-CoV-2 UK variant (B.1.1.7) for Ce41 were shown in Figure 23. The inhibition rates were calculated based on the numbers of plaques formed in individual plates. The relationship between peptide concentration and the inhibition rate was shown in Figure 24. As a result, the IC₅₀ value of Ce41 to the SARS-CoV-2 UK variant (B.1.1.7) calculated was determined as 0.68 nM.

From the results, it was found that Ce41 designed in Example 4 has an extremely excellent antiviral activity against the SARS-CoV-2 UK variant (B.1.1.7).

### [Example 6]

In this Example, Ce41 prepared in the Example 4 was measured for the binding activity to the receptor binding domain (RBD) in the SARS-CoV-2 UK variant (B.1.1.7), and the binding state of Ce41 to a receptor binding domain (RBD) in the SARS-CoV-2 UK variant (B.1.1.7) was studied.

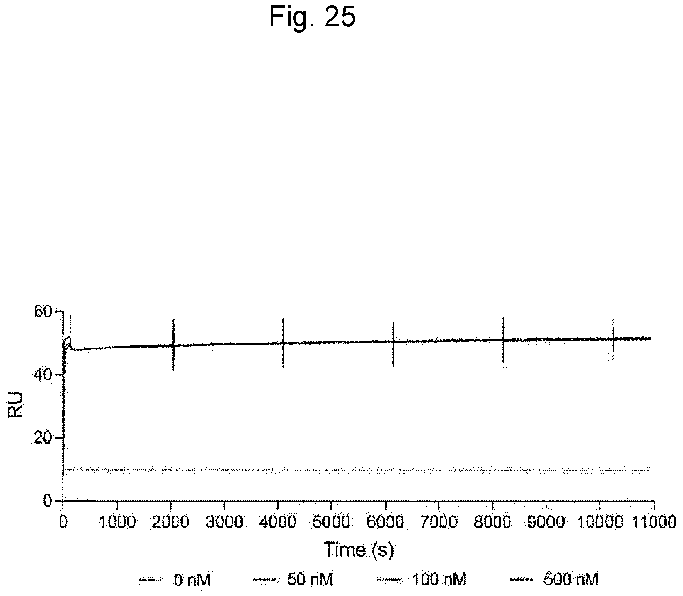

The binding activity of Ce 41 to the receptor binding domain (RBD) in the SARS-CoV-2 UK variant (B.1.1.7) was measured for 3 hours and 2 minutes from the initiation of binding and the results were shown in Figure 25.

As a result, it was found that Ce41 does not dissociate at all from the receptor binding domain (RBD) in the SARS-CoV-2 UK variant (B.1.1.7) once bound, even if it was washed with a buffer for 3 hours; and that Ce41 can maintain excellent binding activity to the receptor binding domain (RBD) in the SARS-CoV-2 UK variant (B.1.1.7) for a long term. The property (of maintaining excellent binding activity to the receptor binding domain (RBD) in the SARS-CoV-2 UK variant (B.1.1.7) for a long term) of Ce41 was considered extremely important for exhibiting significantly high antiviral activity against the SARS-CoV-2 UK variant (B.1.1.7) as shown in Example 5. Note that, LCB1 peptide cannot maintain the binding activity to the receptor binding domain (RBD) in the SARS-CoV-2 UK variant (B.1.1.7) even for a short time.

### [Example 7]

In this Example, a peptide having further excellent binding activity was developed from Ce41 developed in the above Example. In this Example, Ce59 was designed by substituting the amino acid residue at position 18 (lysine residue) in the second region of Ce41 with an arginine residue (surrounded by a frame) as shown in Table 10. Note that, the full-length amino acid sequence of Ce59 was shown in SEQ ID NO: 55.

**[Table 10]**

| | First region | SEQ ID NO: | Second region | SEQ ID NO: |
|---|---|---|---|---|
| Ce41 | DKEWILQKIYEIMRRLDEEG | 1 | HGEASLRVSDLIYEFMKKD | 3 |
| Ce59 | DKEWILQKIYEIMRRLDEEG | 1 | HGEASLRVSDLIYEFMKRD | 2 |

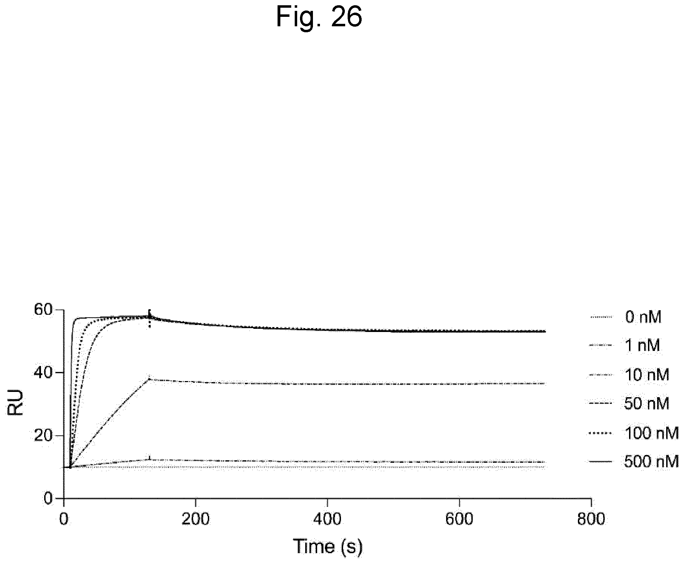

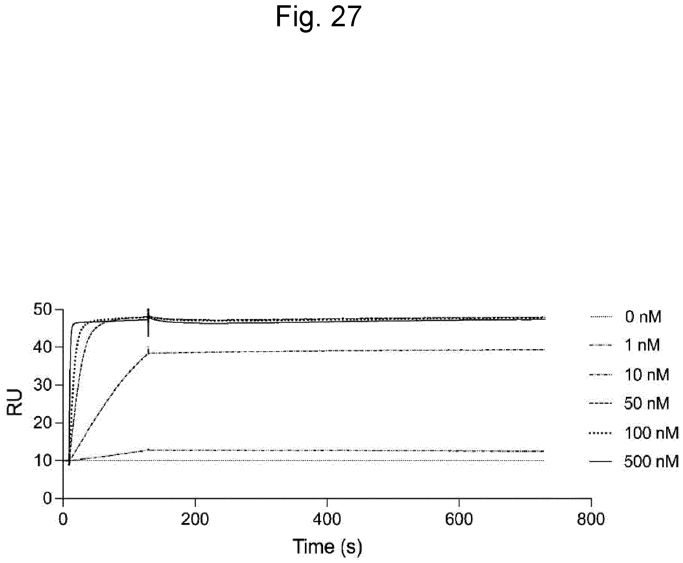

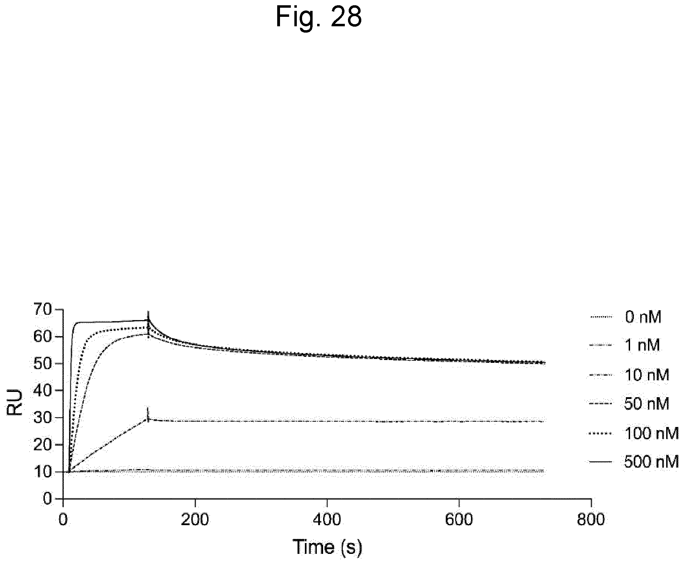

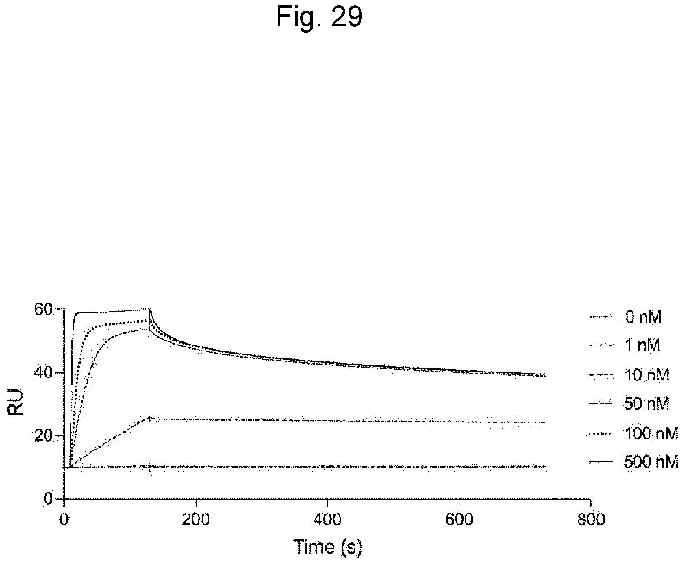

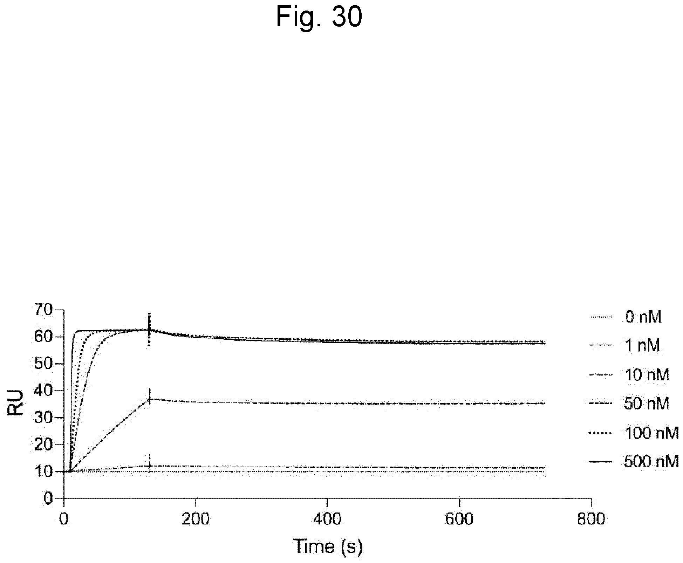

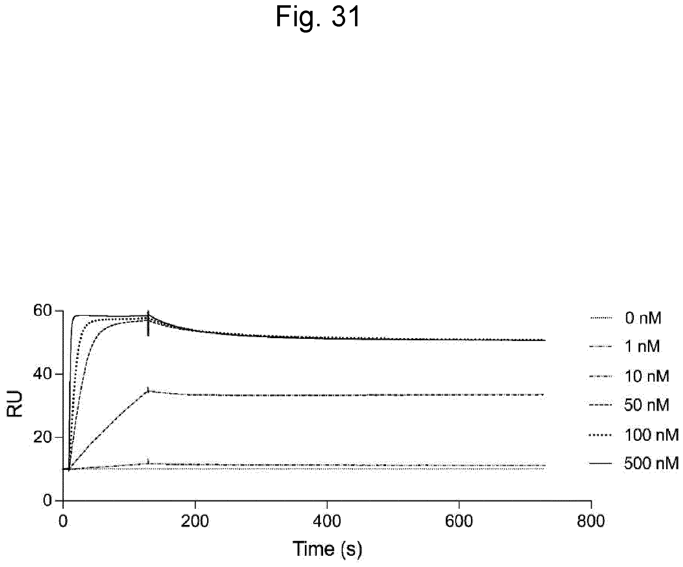

The binding activities of Ce59 to the receptor binding domains (RBD) of wild-type SARS-CoV-2, the SARS-CoV-2 UK variant (B.1.1.7), the SARS-CoV-2 Brazilian variant (P.1), the SARS-CoV-2 South African variant (B.1.351), the SARS-CoV-2 Indian variant (δ type (B.1.617.2)) and the SARS-CoV-2 Indian variant (κ type (B.1.617.1)) were measured. The results of these were shown in Figures 26, 27, 28, 29, 30 and 31, respectively. From the experimental results, it was found that Ce59 is a peptide having excellent binding activity to the receptor binding domains (RBD) in SARS-CoV-2 of wild-type SARS-CoV-2 and all variants tested herein. It was also found that Ce 59 has further excellent binding activity, particularly to the receptor binding domains (RBD) of the SARS-CoV-2 Brazilian variant (P.1) and the SARS-CoV-2 South African variant (B.1.351), compared to Ce41 (Figures 28 and 29).

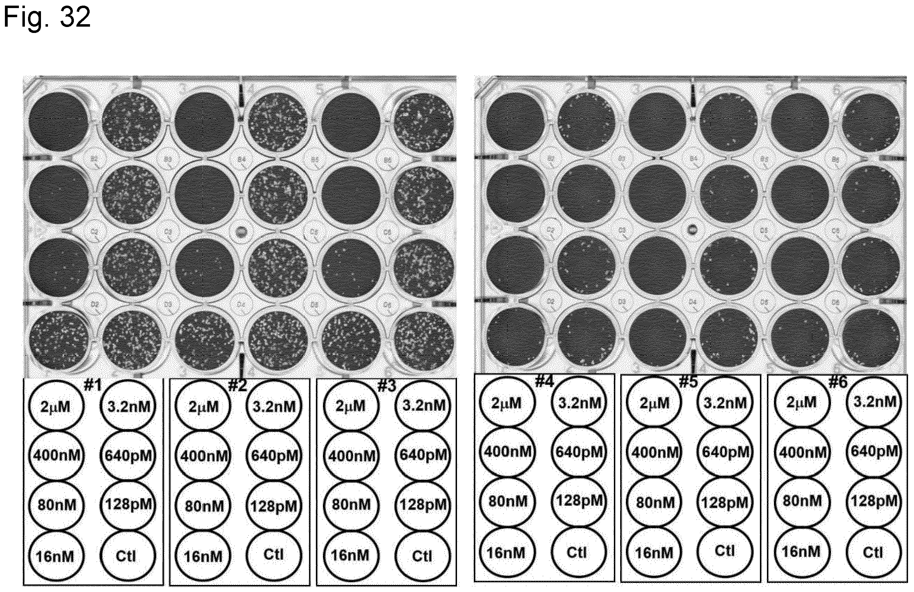

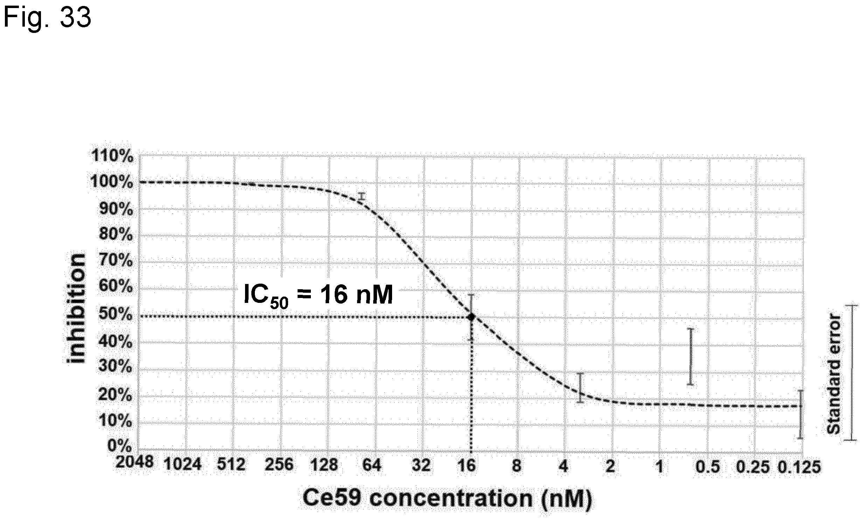

The viral infectivity reduction effect of Ce59 was measured by the plaque reduction assay using the SARS-CoV-2 Indian variant (δ type (B.1.617.2)) and the SARS-CoV-2 UK variant (B.1.1.7). Based on the results, IC₅₀ values were calculated. The results of the plaque reduction assay carried out using the SARS-CoV-2 Indian variant (δ type) (B.1.617.2) were shown in Figure 32. The inhibition rates were calculated based on the numbers of plaques formed in individual plates. The relationship between the peptide concentration and the inhibition rate was shown in Figure 33. As a result, the IC₅₀ value of Ce59 to the SARS-CoV-2 Indian variant (δ type) (B.1.617.2) calculated was 16 nM. As mentioned above, it was found that Ce59 has also excellent antiviral activity against the SARS-CoV-2 Indian variant (δ type) (B.1.617.2).

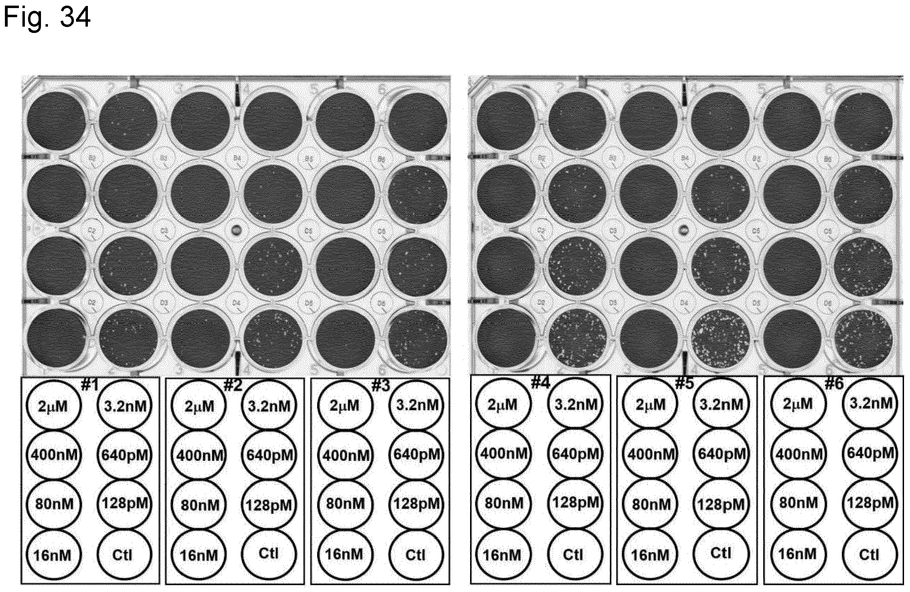

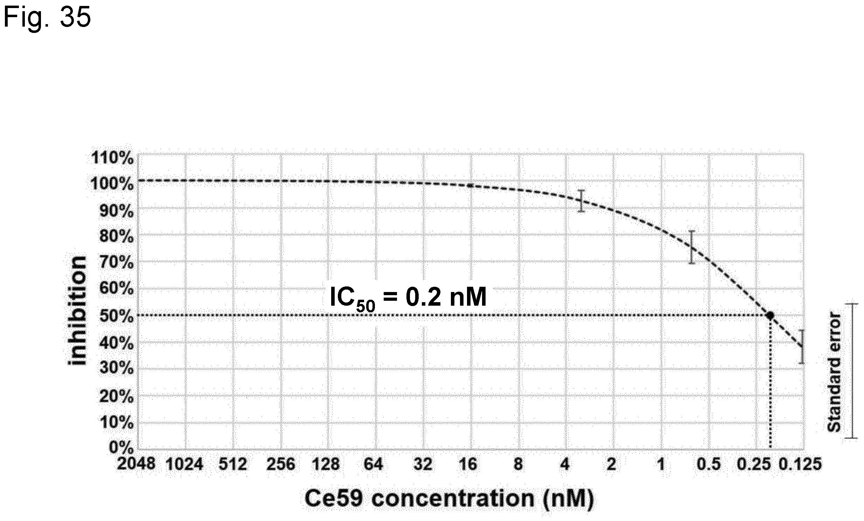

Similarly, the results of the plaque reduction assay using the SARS-CoV-2 UK variant (B.1.1.7) were shown in Figure 34. Also, the inhibition rates were calculated based on the numbers of plaques formed in individual plates. The relationship between the peptide concentration and the inhibition rate was shown in Figure 35. As a result, the IC₅₀ value of Ce59 to the SARS-CoV-2 UK variant (B.1.1.7) calculated was 0.2 nM. The IC₅₀ value of Ce41 to the SARS-CoV-2 UK variant (B.1.1.7), was 0.68 nM, as shown in Example 5. Compared to this, the IC₅₀ value of Ce59 was 0.2 nM. Thus, it became apparent that Ce59 has more excellent antiviral activity against the SARS-CoV-2 UK variant (B.1.1.7) than Ce41.

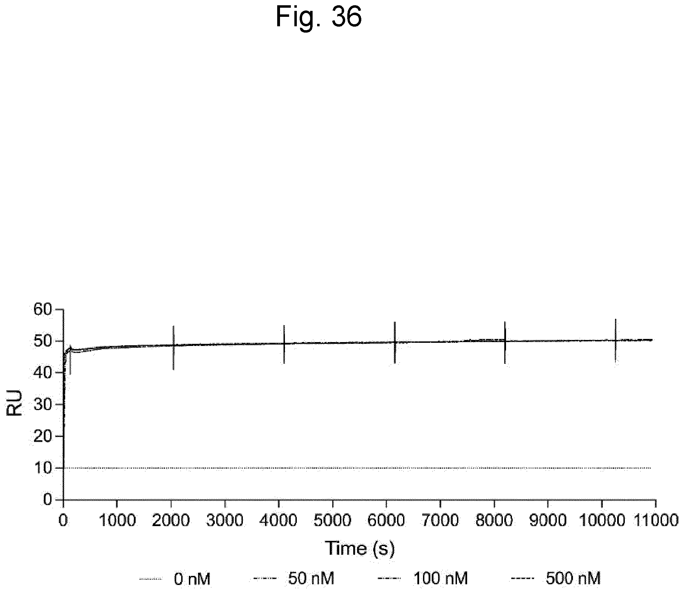

The binding activity of Ce 59 to the receptor binding domain (RBD) in the SARS-CoV-2 UK variant (B.1.1.7) was measured for 3 hours and 2 minutes from the initiation of binding and the results were shown in Figure 36. As a result, it was found that Ce59 does not dissociate at all from the receptor binding domain (RBD) in the SARS-CoV-2 UK variant (B.1.1.7) once bound, even if it was washed with a buffer for 3 hours, and that Ce59 can maintain excellent binding activity to the receptor binding domain (RBD) in the SARS-CoV-2 UK variant (B.1.1.7) for a long term.

### [Example 8]

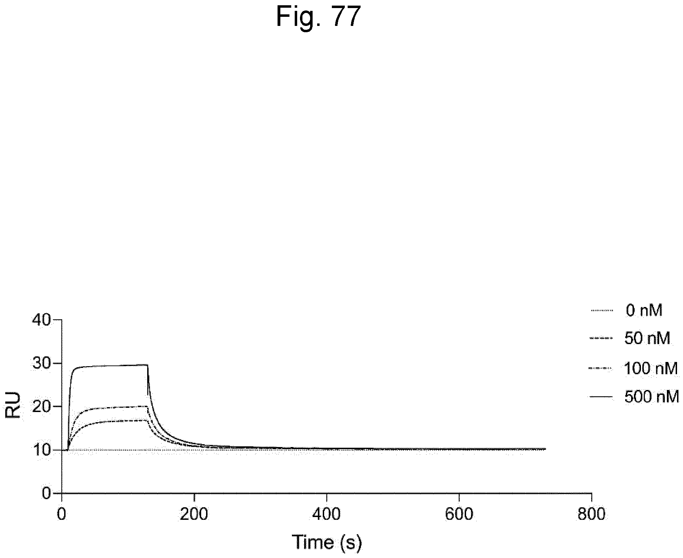

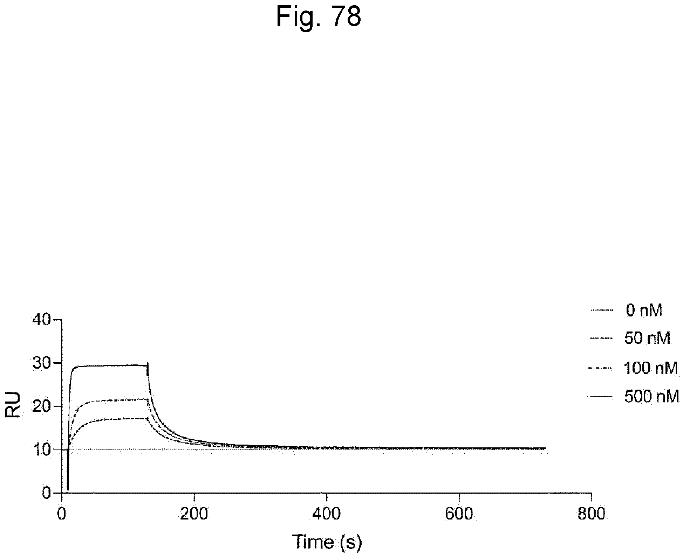

The binding activities of Ce41 and Ce59 developed in the aforementioned Examples to the SARS-CoV-2 Omicron (sublineage BA.1) variant (B.1.1.529BA.1) were measured. As a result, it was found that Ce41 and Ce59 cannot maintain binding to the receptor binding domain (RBD) in the SARS-CoV-2 Omicron (sublineage BA.1) variant (B.1.1.529/BA.1). The experimental results were shown in Figure 77 and Figure 78. Because of this, in this Example, a peptide having further excellent binding activity was developed from Ce59 developed in the aforementioned Example. Ce113 was designed by substituting the amino acid residue at position 14 (arginine residue) in a first region and the amino acid residue at position 7 (arginine residue) in a second region of Ce59 with a glutamine residue and a methionine residue, respectively. The amino acid sequence of Ce113 was shown in Table 11. The amino acid residues substituted for those of the amino acid sequence of Ce59 were each surrounded by a frame. Note that, the full-length amino acid sequence of Ce113 was shown in SEQ ID NO: 58.

**[Table 11]**

| | First region | SEQ ID NO: | Second region | SEQ ID NO: |
|---|---|---|---|---|
| Ce59 | DKEWILQKIYEIMRRLDEEG | 1 | HGEASLRVSDLIYEFMKRD | 2 |
| Ce113 | DKEWILQKIYEIMQRLDEEG | 56 | HGEASLMVSDLIYEFMKRD | 57 |

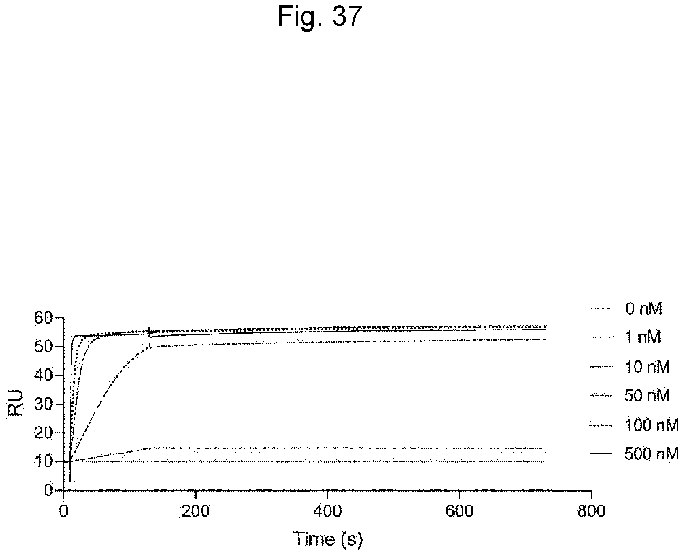

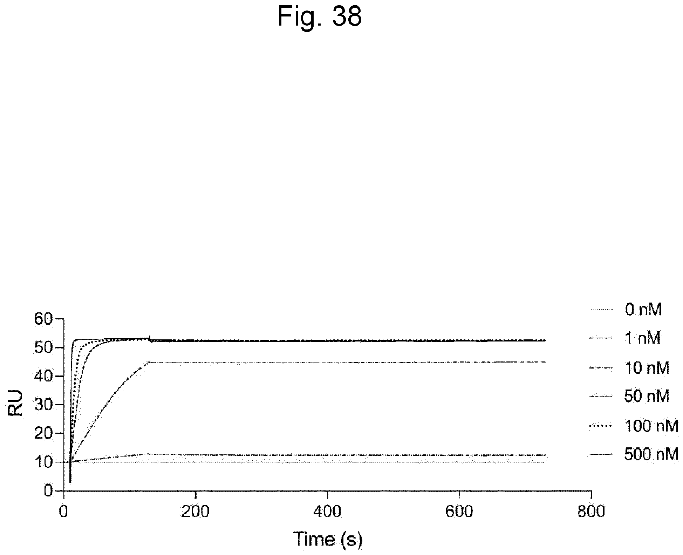

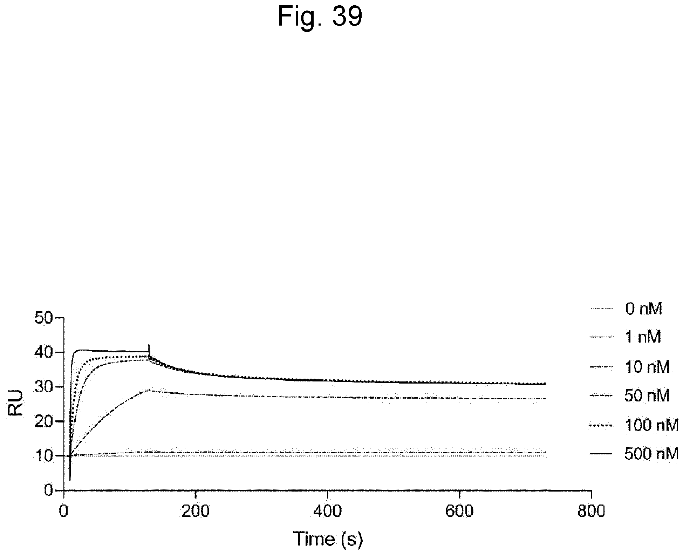

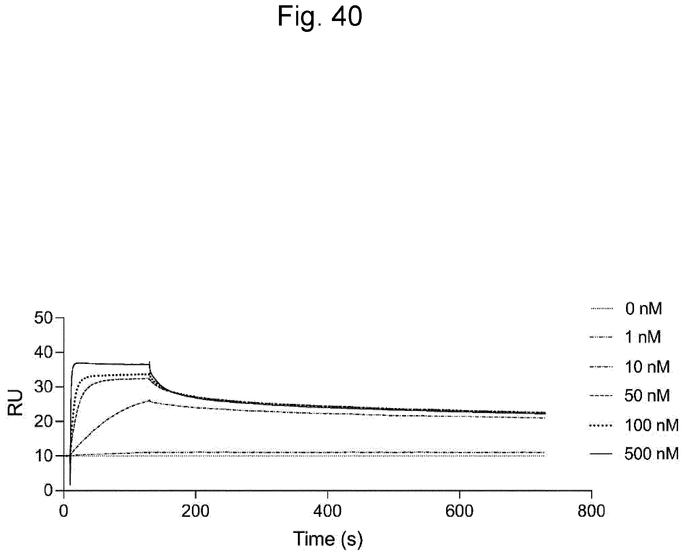

The binding activities of Ce113 to the receptor binding domains (RBD) of the SARS-CoV-2 UK variant (B.1.1.7), the SARS-CoV-2 Indian variant (δ type (B.1.617.2)), the SARS-CoV-2 Omicron (sublineage BA.1) variant (B.1.1.529/BA.1) and the SARS-CoV-2 Omicron (sublineage BA.2) variant (B.1.1.529BA.2) were measured. The results of these were shown in Figures 37, 38, 39 and 40, respectively. From the experimental results, it was found that Ce113 is a peptide having excellent binding activity to the receptor binding domains (RBD) in all SARS-CoV-2 variants tested. It was also found that Ce113 has significantly excellent binding activity, particularly to the receptor binding domains (RBD) of the SARS-CoV-2 Omicron (sublineage BA.1) variant (B.1.1.529/BA.1) and the SARS-CoV-2 Omicron (sublineage BA.2) variant (B.1.1.529BA.2) compared to Ce59.

### [Example 9]

In this Example, a peptide having further excellent binding activity was developed from Ce59 developed in the aforementioned Example. Ce172 was designed by substituting the amino acid residue at position 3 (glutamic acid residue), the amino acid residue at position 14 (arginine residue) in the first region and the amino acid residue at position 7 (arginine residue) in the second region of Ce59 with a leucine residue, a valine residue and a methionine residue, respectively. The amino acid sequence of Ce172 was shown in Table 12. The amino acid residues substituted for those of the amino acid sequence of Ce59 were each surrounded by a frame. Note that, the full-length amino acid sequence of Ce172 was shown in SEQ ID NO: 60.

**[Table 12]**

| | First region | SEQ ID NO: | Second region | SEQ ID NO: |
|---|---|---|---|---|
| Ce59 | DKEWILQKIYEIMRRLDEEG | 1 | HGEASLRVSDLIYEFMKRD | 2 |
| Ce172 | DKLWILQKIYEIMVRLDEEG | 59 | HGEASLMVSDLIYEFMKRD | 57 |

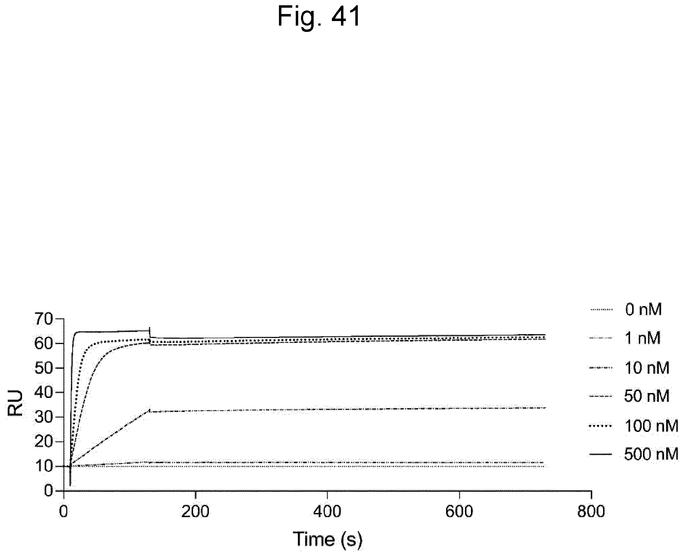

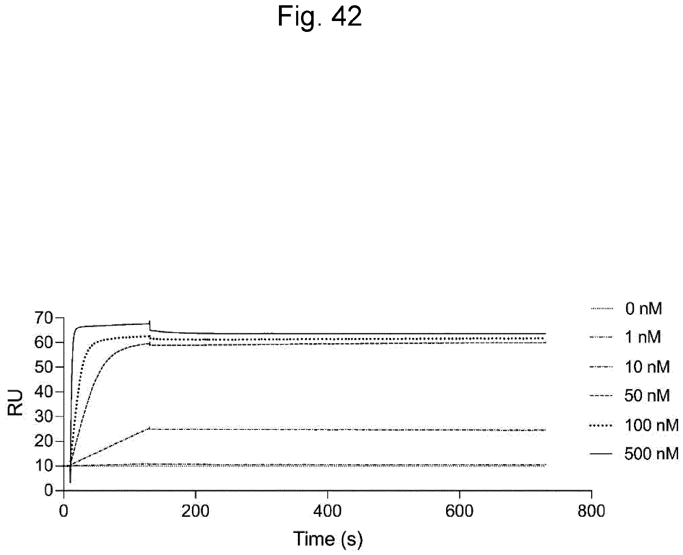

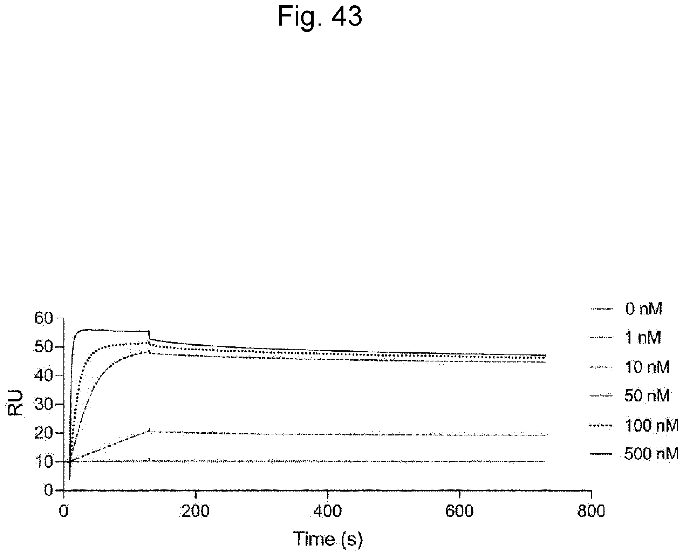

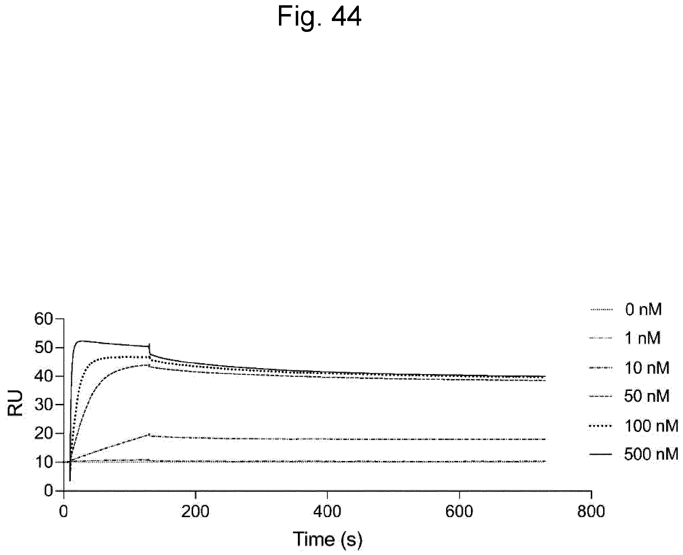

The binding activities of Ce172 to the receptor binding domains (RBD) of the SARS-CoV-2 UK variant (B.1.1.7), the SARS-CoV-2 Indian variant (δ type (B.1.617.2)), the SARS-CoV-2 Omicron (sublineage BA.1) variant (B.1.1.529/BA.1) and the SARS-CoV-2 Omicron (sublineage BA.2) variant (B.1.1.529BA.2) were measured. The results of these were shown in Figures 41, 42, 43 and 44, respectively. From the experimental results, it was found that Ce172 is a peptide having excellent binding activity to the receptor binding domains (RBD) in all SARS-CoV-2 variants tested. It was also found that Ce172 has significantly high binding activity, particularly to the receptor binding domains (RBD) of the SARS-CoV-2 Omicron (sublineage BA.1) variant (B.1.1.529/BA.1) and the SARS-CoV-2 Omicron (sublineage BA.2) variant (B.1.1.529BA.2), compared to Ce59.

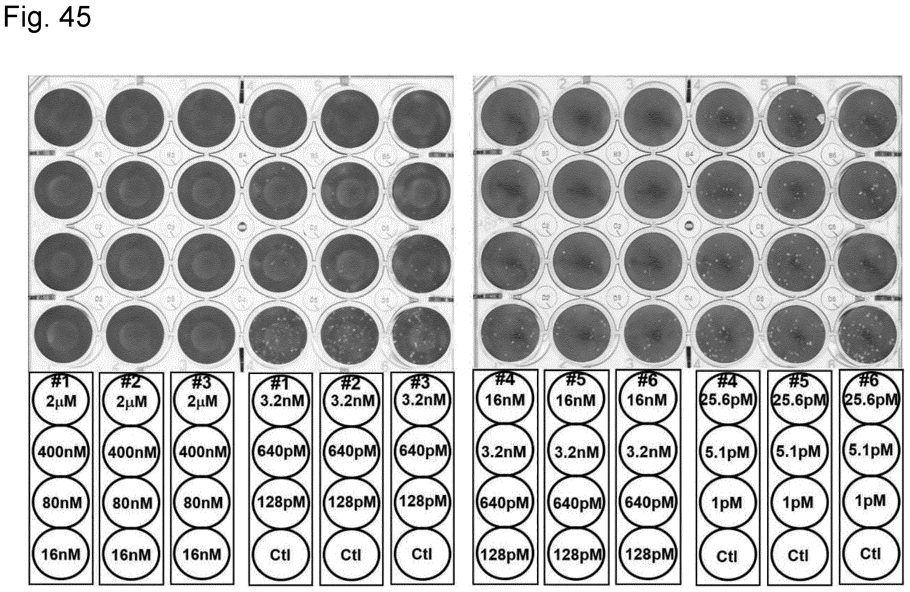

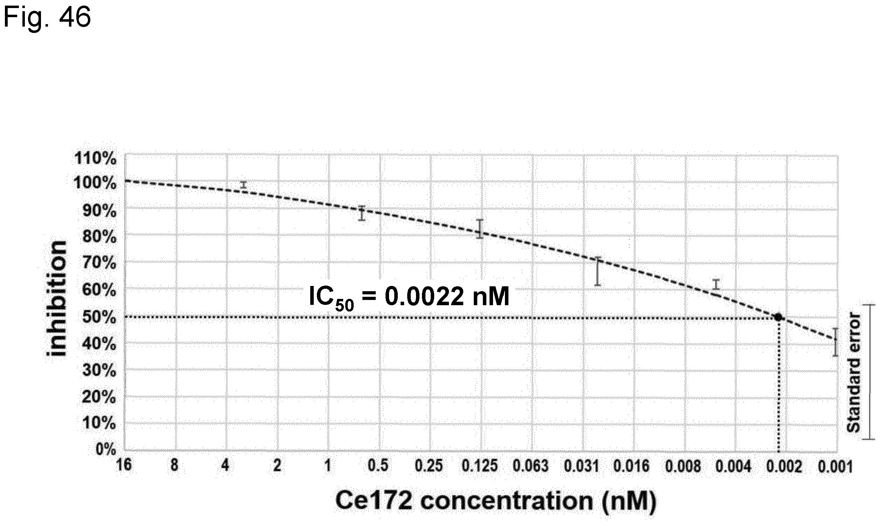

The viral infectivity reduction effect of Ce172 was measured by the plaque reduction assay using the SARS-CoV-2 UK variant (B.1.1.7), the SARS-CoV-2 Indian variant (δ type (B.1.617.2)) and the SARS-CoV-2 Omicron (sublineage BA.1) variant (B.1.1.529/BA.1). Based on the results, IC₅₀ values were calculated. The results of the plaque reduction assay carried out using the SARS-CoV-2 UK variant (B.1.1.7) were shown in Figure 45. The inhibition rates were calculated based on the numbers of plaques formed in individual plates. The relationship between the peptide concentration and the inhibition rate was shown in Figure 46. As a result, the IC₅₀ value of Ce172 to the SARS-CoV-2 UK variant (B.1.1.7) calculated was 0.0022 nM. The IC₅₀ value of Ce41 to the SARS-CoV-2 UK variant (B.1.1.7) was 0.68 nM, as shown in Example 5, and the IC₅₀ value of Ce59 to the SARS-CoV-2 UK variant (B.1.1.7) was 0.2 nM as shown in Example 7. Compared to these, the IC₅₀ value of Ce172 was 0.0022 nM. Thus, it becomes apparent that Ce172 has more excellent antiviral activity against the SARS-CoV-2 UK variant (B.1.1.7) than Ce41 and Ce59.

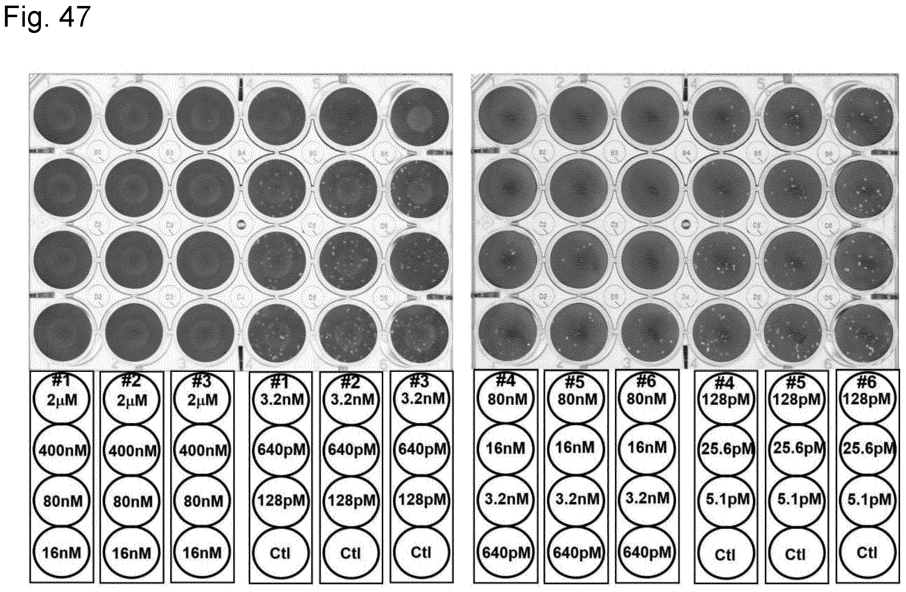

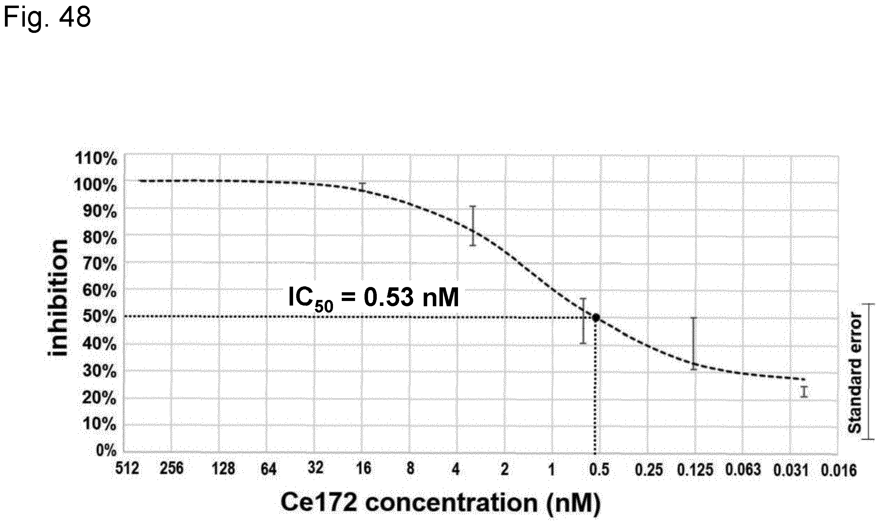

The results of the plaque reduction assay carried out using the SARS-CoV-2 Indian variant (δ type (B.1.617.2)) were similarly shown in Figure 47. The inhibition rates were calculated based on the numbers of plaques formed in individual plates. The relationship between the peptide concentration and the inhibition rate was shown in Figure 48. As a result, the IC₅₀ value of Ce172 to the SARS-CoV-2 Indian variant (δ type) (B.1.617.2) calculated as 0.53 nM. As shown in Example 7, the IC₅₀ value of Ce59 to the SARS-CoV-2 Indian variant (δ type) (B.1.617.2) was 16 nM, whereas the IC₅₀ value of Ce172 of 0.53 nM. From the comparison, it becomes apparent that Ce172 has extremely excellent antiviral activity against the SARS-CoV-2 Indian variant (δ type) (B.1.617.2) than Ce59.

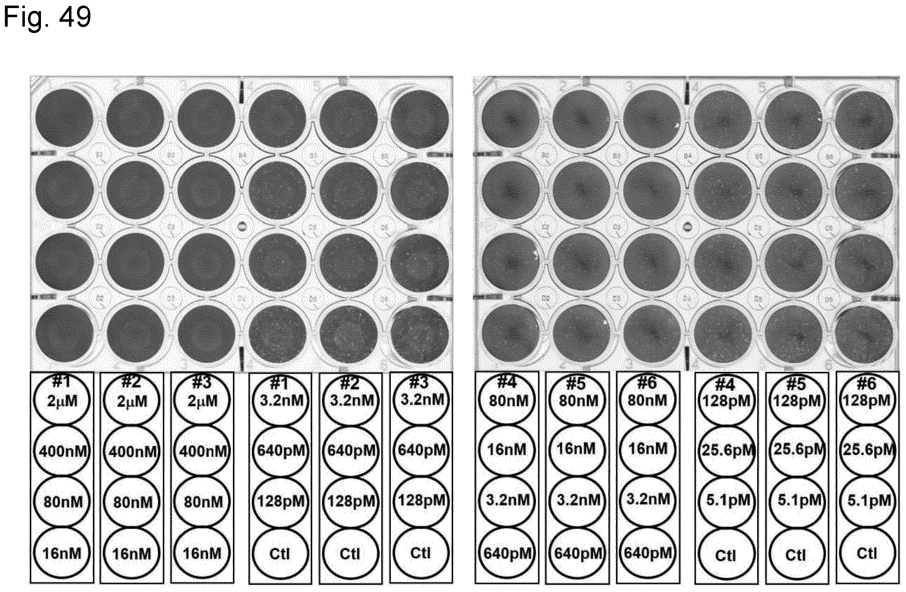

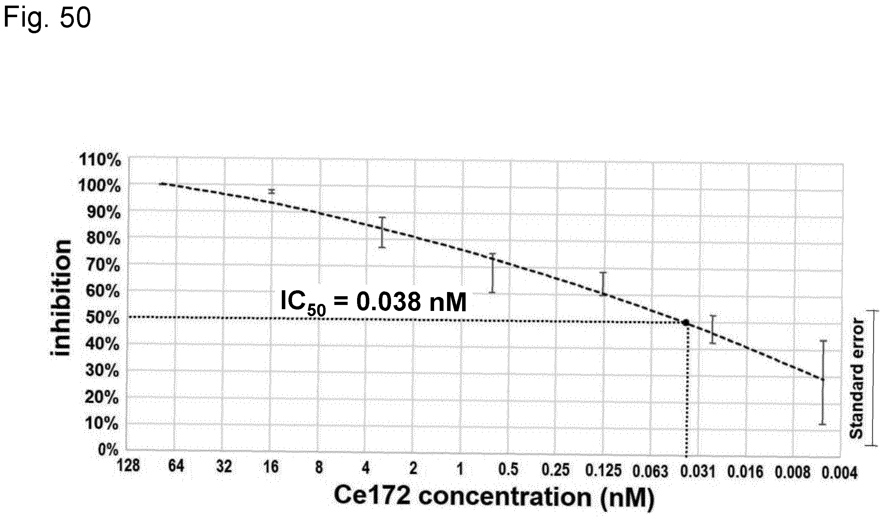

The results of the plaque reduction assay carried out using the SARS-CoV-2 Omicron (sublineage BA.1) variant (B.1.1.529/BA.1) were similarly shown in Figure 49. The inhibition rates were calculated based on the numbers of plaques formed in individual plates. The relationship between the peptide concentration and the inhibition rate was shown in Figure 50. As a result, the IC₅₀ value of Ce172 to the SARS-CoV-2 Omicron (sublineage BA. 1) variant (B.1.1.529/BA.1) calculated was 0.038 nM. As shown above, it became apparent that Ce172 has excellent antiviral activity against the SARS-CoV-2 Omicron (sublineage BA.1) variant (B.1.1.529/BA.1).

### [Example 10]

In this Example, a peptide having further excellent binding activity was developed from Ce59 developed in the aforementioned Example. Ce173 was designed by substituting the amino acid residue at position 3 (glutamic acid residue) and the amino acid residue at position 14 (arginine residue) in the first region, and the amino acid residue at position 7 (arginine residue) in the second region of Ce59 with a leucine residue, a glutamine residue and a methionine residue, respectively. The amino acid sequence of Ce173 was shown in Table 13. The amino acid residues substituted for those of the amino acid sequence of Ce59 were each surrounded by a frame. Note that, the full-length amino acid sequence of Ce173 was shown in SEQ ID NO: 64.

**[Table 13]**

| | First region | SEQ ID NO: | Second region | SEQ ID NO: |
|---|---|---|---|---|
| Ce59 | DKEWILQKIYEIMRRLDEEG | 1 | HGEASLRVSDLIYEFMKRD | 2 |
| Ce173 | DKLWILQKIYEIMQRLDEEG | 63 | HGEASLMVSDLIYEFMKRD | 57 |

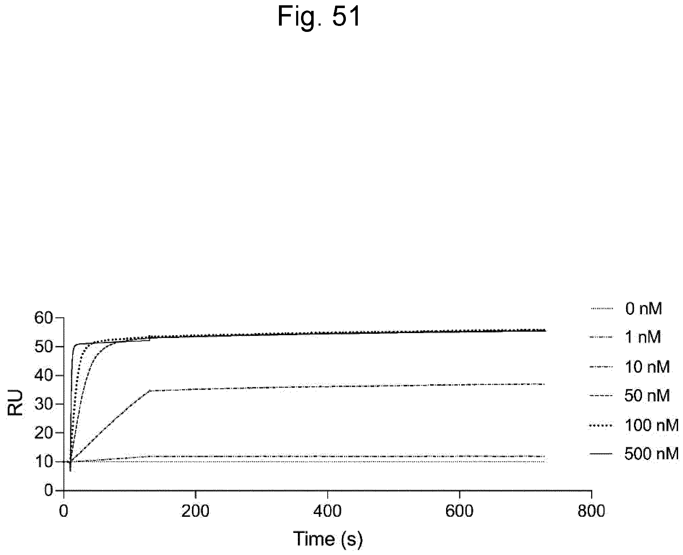

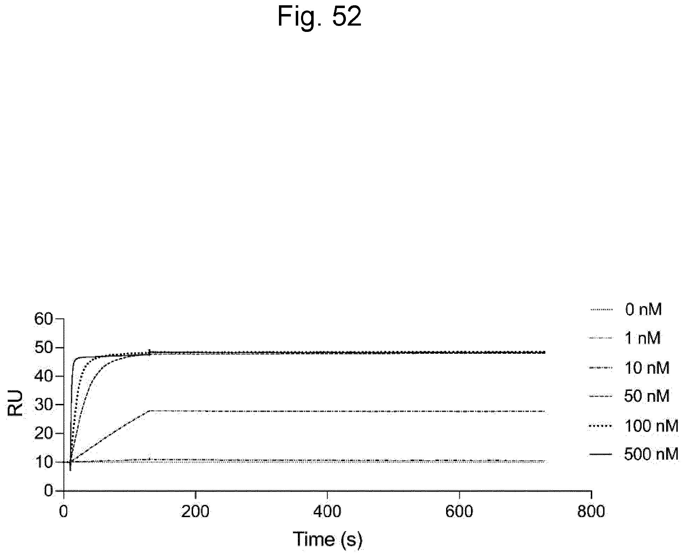

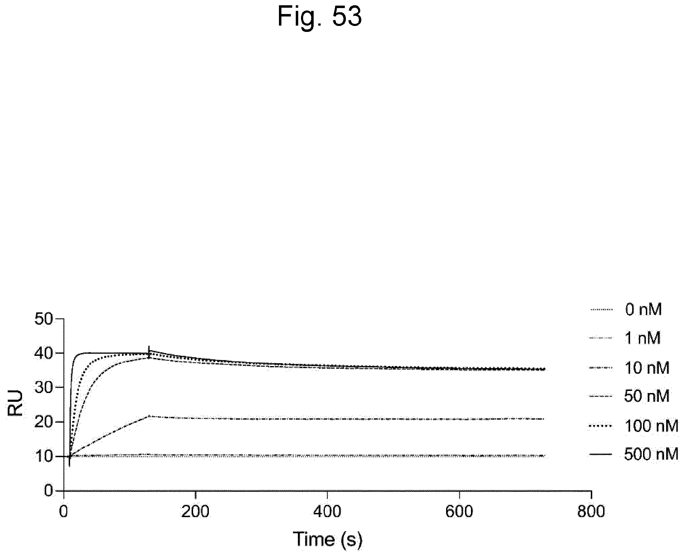

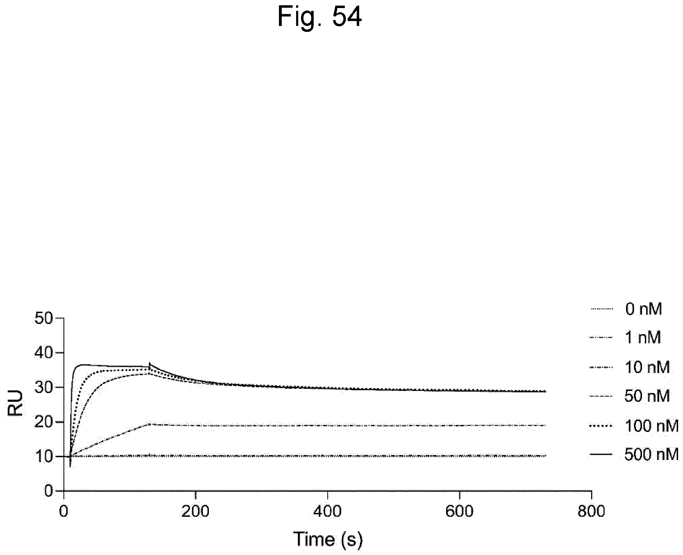

The binding activities of Ce173 to the receptor binding domains (RBD) of the SARS-CoV-2 UK variant (B.1.1.7), the SARS-CoV-2 Indian variant (δ type (B.1.617.2)), the SARS-CoV-2 Omicron (sublineage BA.1) variant (B.1.1.529/BA.1) and the SARS-CoV-2 Omicron (sublineage BA.2) variant (B.1.1.529BA.2) were measured. The results of these were shown in Figures 51, 52, 53 and 54, respectively. From the experimental results, it was found that Ce173 is a peptide having excellent binding activity to the receptor binding domains (RBD) in all SARS-CoV-2 variants tested. It was also found that Ce173 has significantly high binding activity particularly to the receptor binding domains (RBD) of the SARS-CoV-2 Omicron (sublineage BA.1) variant (B.1.1.529/BA.1) and the SARS-CoV-2 Omicron (sublineage BA.2) variant (B.1.1.529BA.2) compared to Ce59.

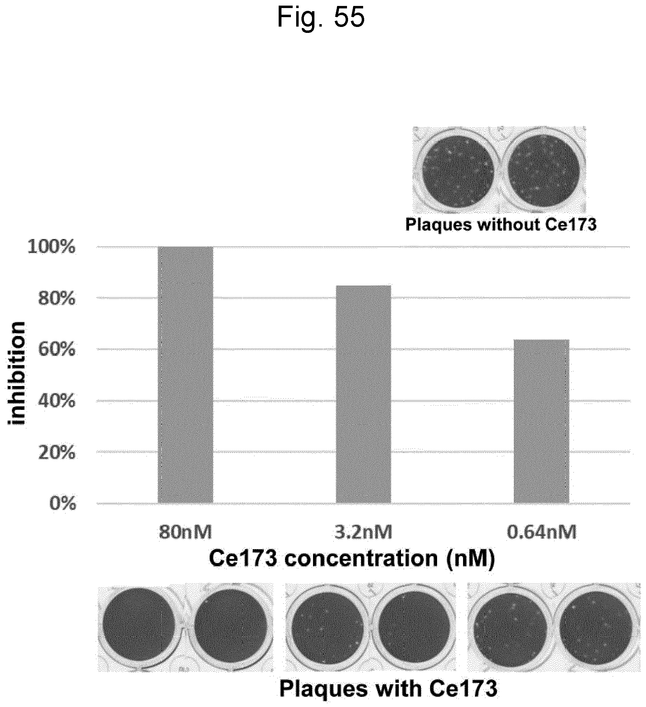

The viral infectivity reduction effect of Ce173 was measured by the plaque reduction assay using the SARS-CoV-2 UK variant (B.1.1.7), the SARS-CoV-2 Indian variant (δ type (B.1.617.2)) and the SARS-CoV-2 Omicron (sublineage BA.1) variant (B.1.1.529/BA.1). Based on the results, inhibition rates were calculated. The results of the plaque reduction assay carried out using the SARS-CoV-2 UK variant (B.1.1.7) and the inhibition rates calculated based on the numbers of plaques formed in individual plates were shown in Figure 55. As a result, it was found that Ce173 has excellent antiviral activity against the SARS-CoV-2 UK variant (B.1.1.7).

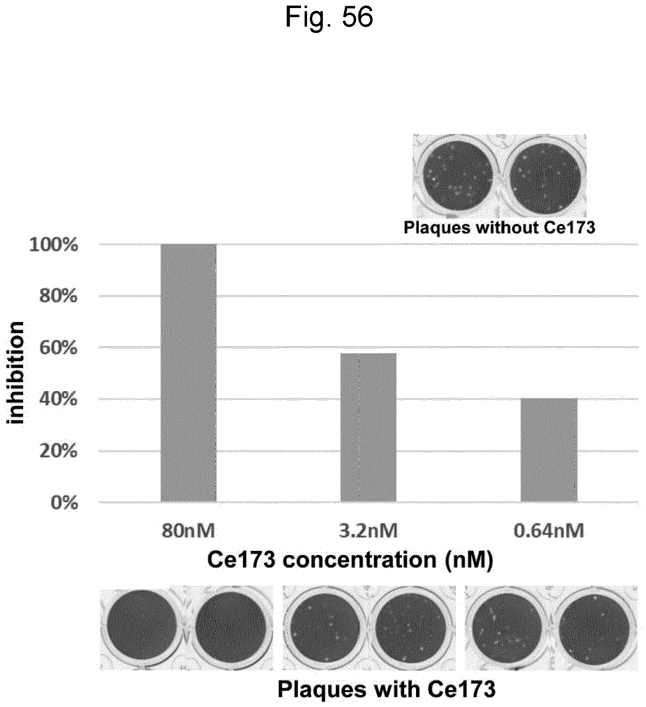

Similarly, the results of the plaque reduction assay carried out using the SARS-CoV-2 Indian variant (δ type (B.1.617.2)) and the inhibition rates calculated based on the numbers of plaques formed in individual plates were shown in Figure 56. As a result, it was found that Ce173 has excellent antiviral activity against the SARS-CoV-2 Indian variant (δ type (B.1.617.2)).

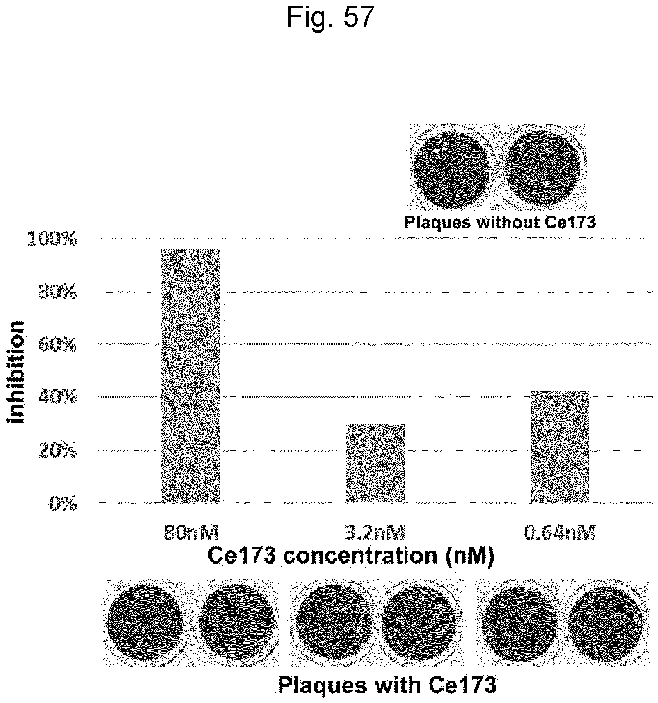

Similarly, the results of the plaque reduction assay carried out using the SARS-CoV-2 Omicron (sublineage BA. 1) variant (B.1.1.529/BA.1) and the inhibition rates calculated based on the numbers of plaques formed in individual plates were shown in Figure 57. As a result, it was found that Ce173 has excellent antiviral activity against the SARS-CoV-2 Omicron (sublineage BA. 1) variant (B.1.1.529/BA.1).

### [Example 11]

In this Example, a peptide having further excellent binding activity was developed from Ce59 developed in the aforementioned Example. Ce174 was designed by substituting the amino acid residue at position 7 (glutamine residue) and the amino acid residue at position 14 (arginine residue) in the first region, and the amino acid residue at position 7 (arginine residue) in the second region of Ce59 with a tyrosine residue, a glutamine residue and a methionine residue, respectively. The amino acid sequence of Ce174 was shown in Table 14. The amino acid residues substituted for those of the amino acid sequence of Ce59 were each surrounded by a frame. Note that, the full-length amino acid sequence of Ce174 was shown in SEQ ID NO: 66.

**[Table 14]**

| | First region | SEQ ID NO: | Second region | SEQ ID NO: |
|---|---|---|---|---|
| Ce59 | DKEWILQKIYEIMRRLDEEG | 1 | HGEASLRVSDLIYEFMKRD | 2 |
| Ce174 | DKEWILYKIYEIMQRLDEEG | 65 | HGEASLMVSDLIYEFMKRD | 57 |

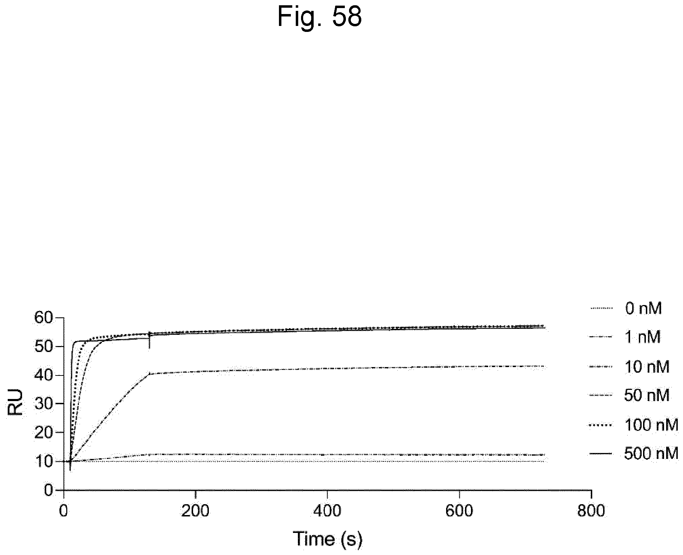

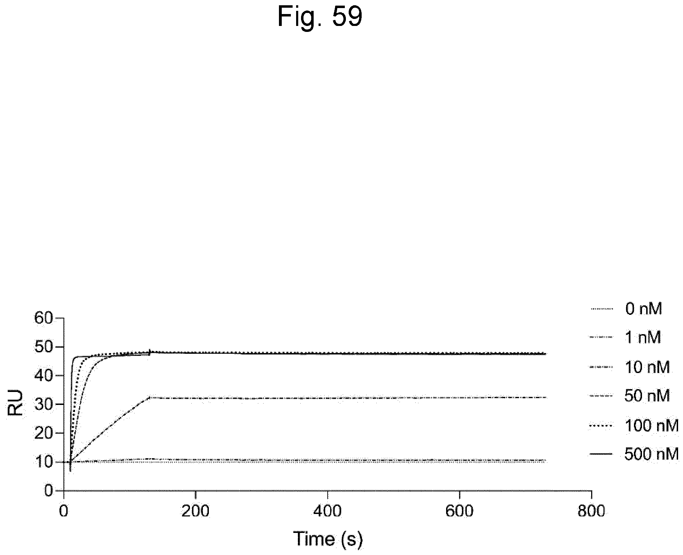

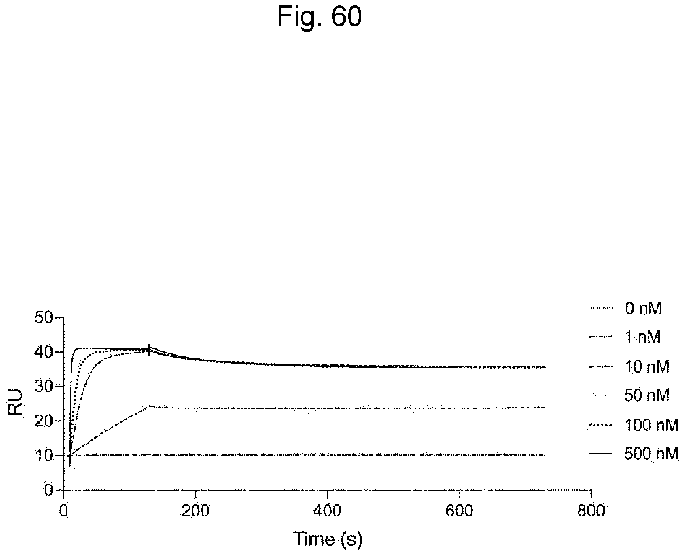

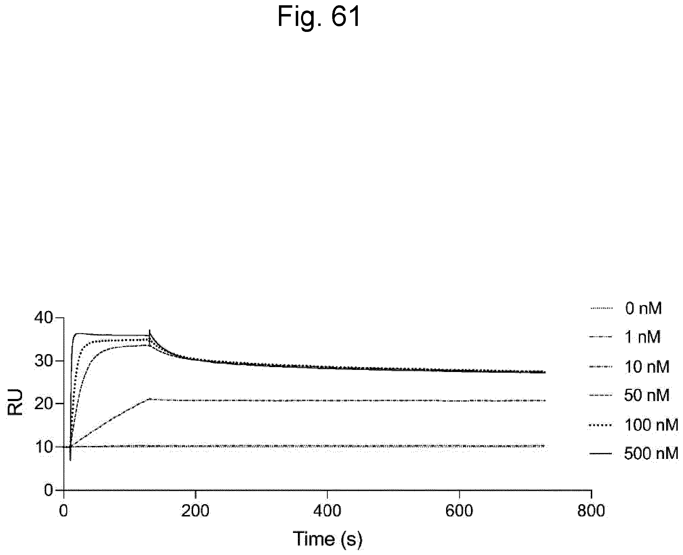

The binding activities of Ce174 to the receptor binding domains (RBD) of the SARS-CoV-2 UK variant (B.1.1.7), the SARS-CoV-2 Indian variant (δ type (B.1.617.2)), the SARS-CoV-2 Omicron (sublineage BA.1) variant (B.1.1.529/BA.1) and the SARS-CoV-2 Omicron (sublineage BA.2) variant (B.1.1.529BA.2) were measured. The results of these were shown in Figures 58, 59, 60 and 61, respectively. From the experimental results, it was found that Ce174 is a peptide having excellent binding activity to the receptor binding domains (RBD) in all SARS-CoV-2 variants tested. It was also found that Ce174 has significantly high binding activity, particularly to the receptor binding domains (RBD) of the SARS-CoV-2 Omicron (sublineage BA.1) variant (B.1.1.529/BA.1) and the SARS-CoV-2 Omicron (sublineage BA.2) variant (B.1.1.529BA.2) compared to Ce59.

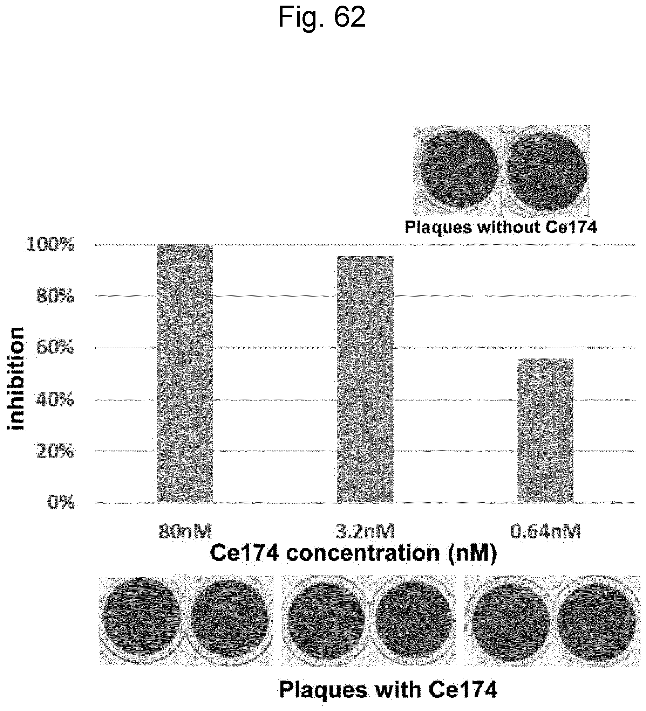

The viral infectivity reduction effect of Ce174 was measured by the plaque reduction assay using the SARS-CoV-2 UK variant (B.1.1.7), the SARS-CoV-2 Indian variant (δ type (B.1.617.2)) and the SARS-CoV-2 Omicron (sublineage BA.1) variant (B.1.1.529/BA.1). Based on the results, IC₅₀ values were calculated. The results of the plaque reduction assay carried out using the SARS-CoV-2 UK variant (B.1.1.7) and the inhibition rates calculated based on the numbers of plaques formed in individual plates were shown in Figure 62. As a result, it became apparent that Ce174 has excellent antiviral activity against the SARS-CoV-2 UK variant (B.1.1.7).

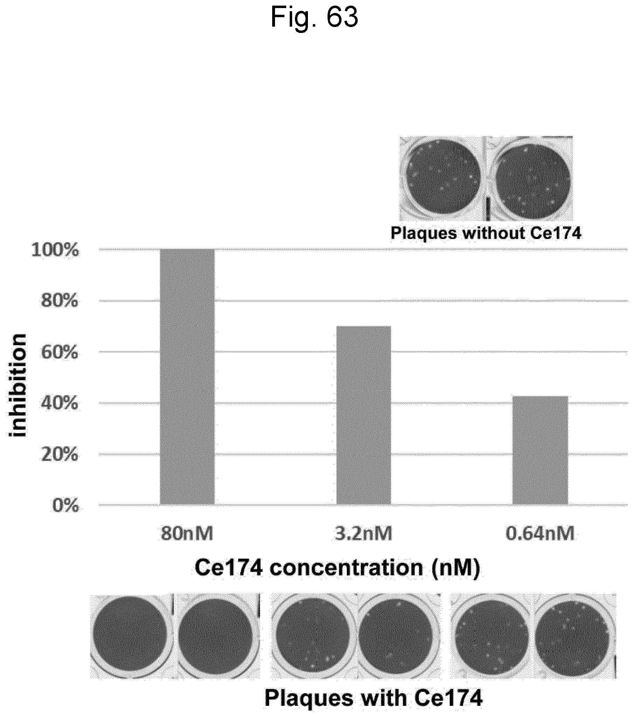

Similarly, the results of the plaque reduction assay carried out using the SARS-CoV-2 Indian variant (δ type (B.1.617.2)) and the inhibition rates calculated based on the numbers of plaques formed in individual plates were shown in Figure 63. As a result, it became apparent that Ce174 has excellent antiviral activity against the SARS-CoV-2 Indian variant (δ type (B.1.617.2)).

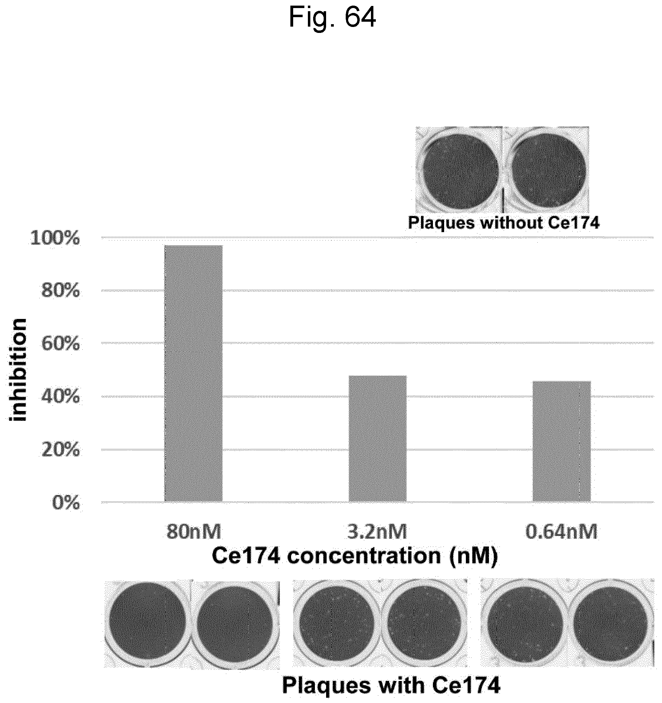

Similarly, the results of the plaque reduction assay carried out using the SARS-CoV-2 Omicron (sublineage BA.1) variant (B.1.1.529/BA.1) and the inhibition rates calculated based on the numbers of plaques formed in individual plates were shown in Figure 64. As a result, it became apparent that Ce174 has excellent antiviral activity against the SARS-CoV-2 Omicron (sublineage BA.1) variant (B.1.1.529/BA.1).

### [Example 12]

In this Example, a peptide having further excellent binding activity was developed from Ce59 developed in the aforementioned Example. Ce149 was designed by substituting the amino acid residue at position 7 (glutamine residue) and amino acid residue at position 14 (arginine residue) in the first region, and the amino acid residue at position 7 (arginine residue) in the second region of Ce59 with a tyrosine residue, a valine residue and a methionine residue, respectively. The amino acid sequence of Ce149 was shown in Table 15. The amino acid residues substituted for those of the amino acid sequence of Ce59 were each surrounded by a frame. Note that, the full-length amino acid sequence of Ce149 was shown in SEQ ID NO: 62.

**[Table 15]**

| | First region | SEQ ID NO: | Second region | SEQ ID NO: |
|---|---|---|---|---|
| Ce59 | DKEWILQKIYEIMRRLDEEG | 1 | HGEASLRVSDLIYEFMKRD | 2 |
| Ce149 | DKEWILYKIYEIMVRLDEEG | 61 | HGEASLMVSDLIYEFMKRD | 57 |

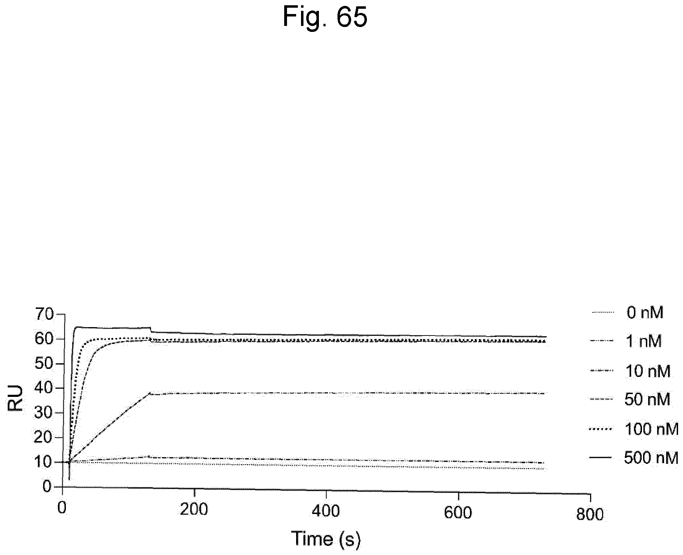

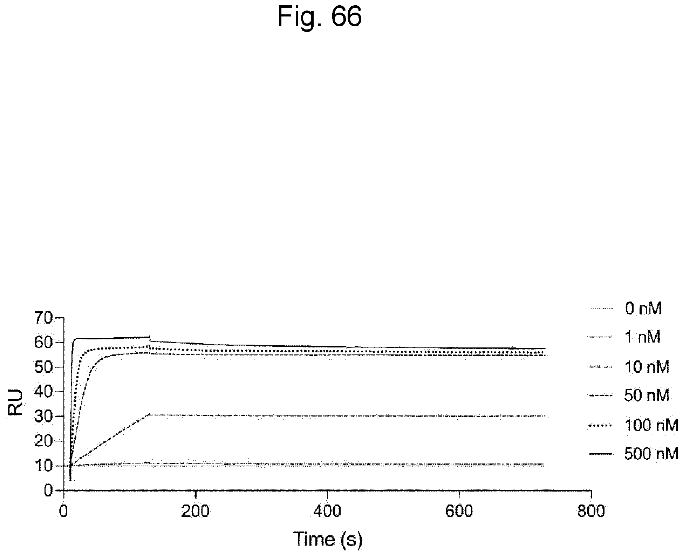

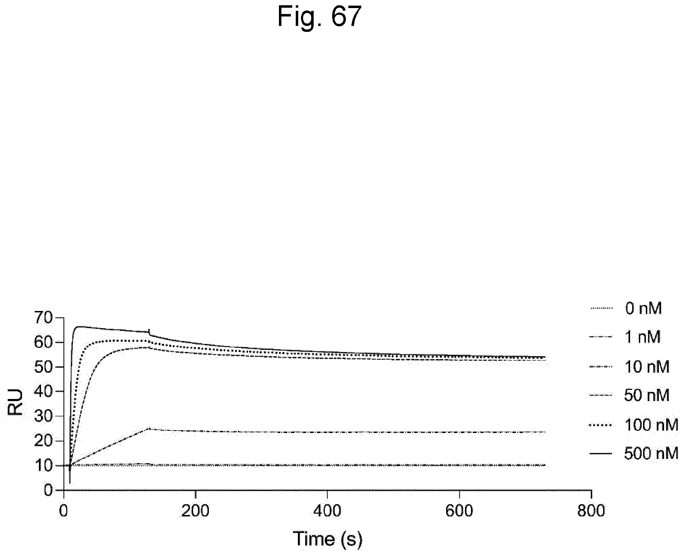

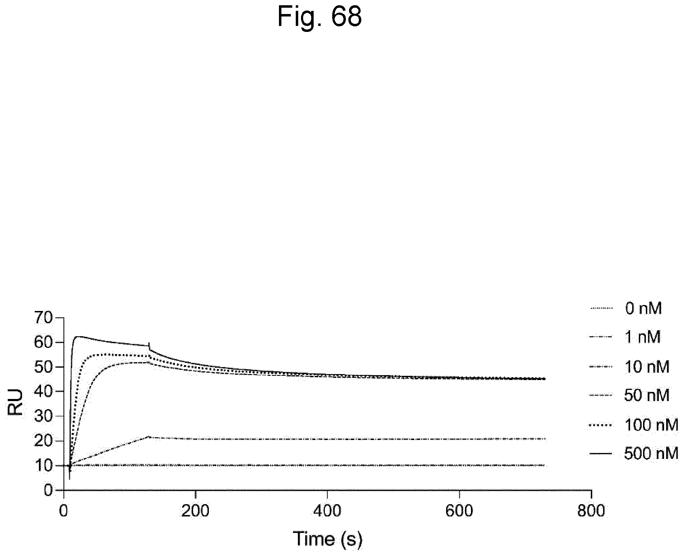

The binding activities of Ce149 to the receptor binding domains (RBD) of the SARS-CoV-2 UK variant (B.1.1.7), the SARS-CoV-2 Indian variant (δ type (B.1.617.2)), the SARS-CoV-2 Omicron (sublineage BA.1) variant (B.1.1.529/BA.1) and the SARS-CoV-2 Omicron (sublineage BA.2) variant (B.1.1.529BA.2) were measured. The results of these were shown in Figures 65, 66, 67 and 68, respectively. From the experimental results, it was found that Ce149 is a peptide having excellent binding activity to the receptor binding domains (RBD) in all SARS-CoV-2 variants tested. It was also found that Ce149 has significantly high binding activity, particularly to the receptor binding domains (RBD) of the SARS-CoV-2 Omicron (sublineage BA.1) variant (B.1.1.529/BA.1) and the SARS-CoV-2 Omicron (sublineage BA.2) variant (B.1.1.529BA.2) compared to Ce59.

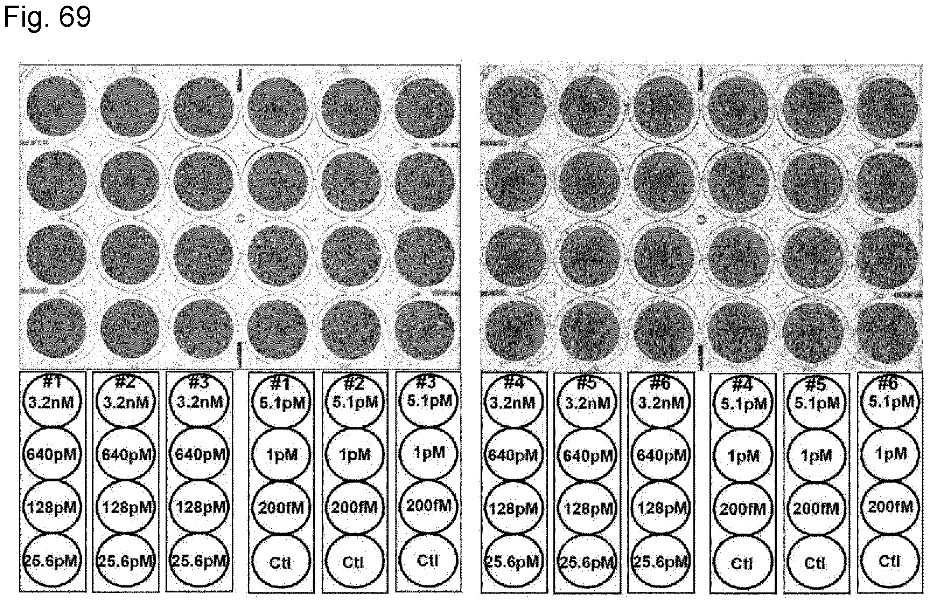

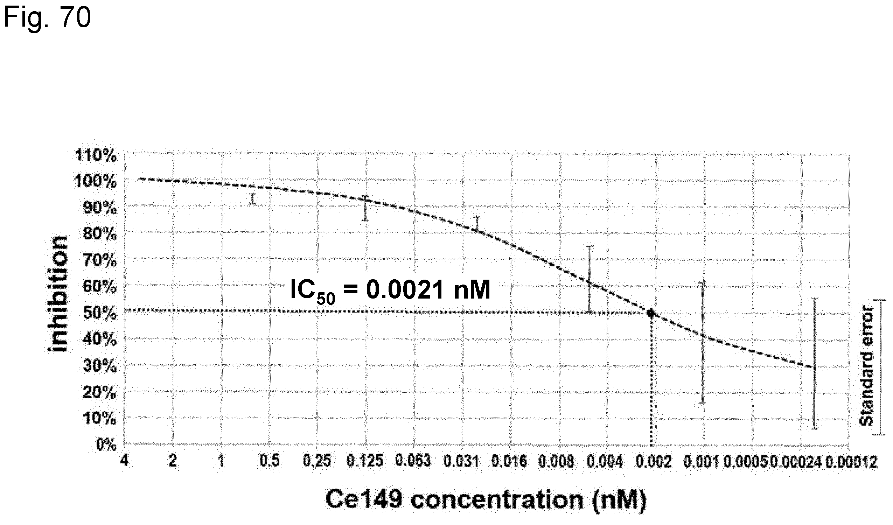

The viral infectivity reduction effect of Ce149 was measured by the plaque reduction assay using the SARS-CoV-2 UK variant (B.1.1.7), the SARS-CoV-2 Indian variant (δ type (B.1.617.2)) and the SARS-CoV-2 Omicron (sublineage BA.1) variant (B.1.1.529/BA.1). Based on the results, IC₅₀ values were calculated. The results of the plaque reduction assay carried out using the SARS-CoV-2 UK variant (B.1.1.7) were shown in Figure 69. The inhibition rates were calculated based on the numbers of plaques formed in individual plates. The relationship between the peptide concentration and the inhibition rate was shown in Figure 70. As a result, the IC₅₀ value of Ce149 to the SARS-CoV-2 UK variant (B.1.1.7) calculated was 0.0021 nM. The IC₅₀ value of Ce59 to the SARS-CoV-2 UK variant (B.1.1.7) was 0.68 nM as shown in Example 5 and the IC₅₀ value of Ce59 to the SARS-CoV-2 UK variant (B.1.1.7) was 0.2 nM as shown in Example 7. As compared to these, the IC₅₀ value ofCe149 was 0.0021 nM. Thus, it becomes apparent that Ce149 has more excellent antiviral activity against the SARS-CoV-2 UK variant (B.1.1.7) than Ce41 and Ce59.

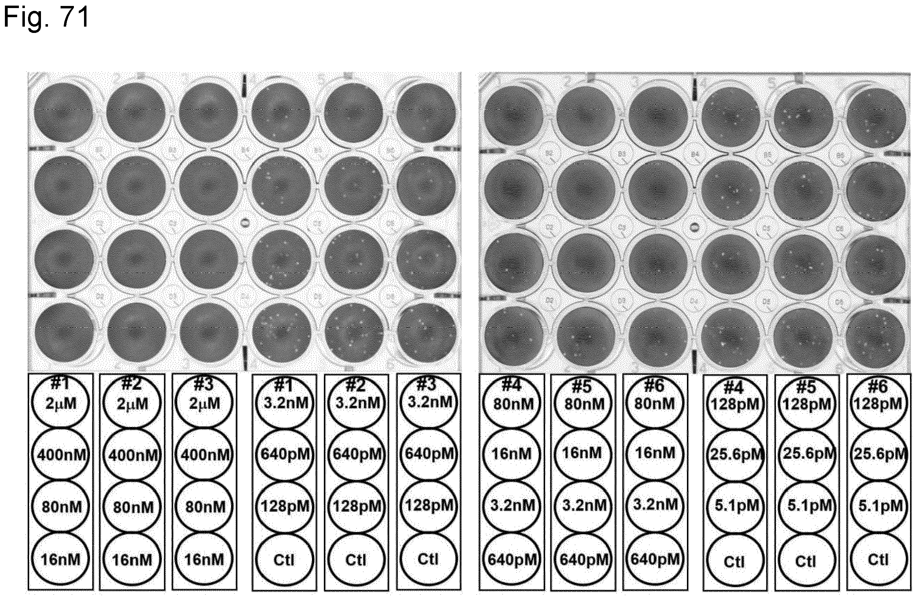

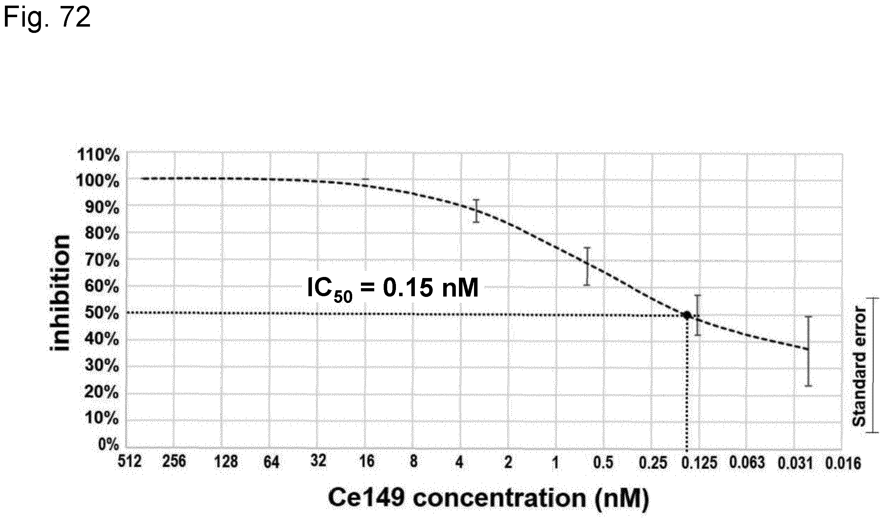

Similarly, the results of the plaque reduction assay similarly carried out using the SARS-CoV-2 Indian variant (δ type (B.1.617.2)) were shown in Figure 71. The inhibition rates were calculated based on the numbers of plaques formed in individual plates. The relationship between the peptide concentration and the inhibition rate was shown in Figure 72. As a result, the IC₅₀ value of Ce149 to the SARS-CoV-2 Indian variant (δ type) (B.1.617.2) calculated was 0.15 nM. The IC₅₀ value of Ce59 to the SARS-CoV-2 Indian variant (δ type) (B.1.617.2) was 16 nM, as shown in Example 7. Compared to this, the IC₅₀ value of Ce149 was 0.15 nM. Thus, it becomes apparent that Ce149 has extremely excellent antiviral activity against the SARS-CoV-2 Indian variant (δ type) (B.1.617.2) than Ce59.

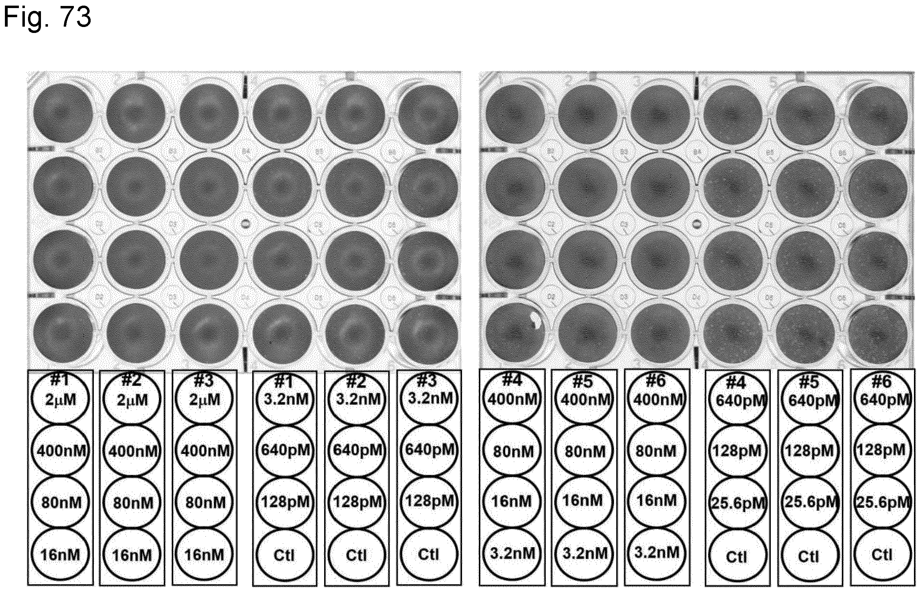

Similarly, the results of the plaque reduction assay carried out using the SARS-CoV-2 Omicron (sublineage BA.1) variant (B.1.1.529/BA.1) were shown in Figure 73. The inhibition rates were calculated based on the numbers of plaques formed in individual plates. As a result, the IC₅₀ value of Ce149 to the SARS-CoV-2 Omicron (sublineage BA.1) variant (B.1.1.529/BA.1) calculated was 1 nM. As shown above, it became apparent that Ce149 has excellent antiviral activity against the SARS-CoV-2 Omicron (sublineage BA.1) variant (B.1.1.529/BA.1).

### [Example 13]

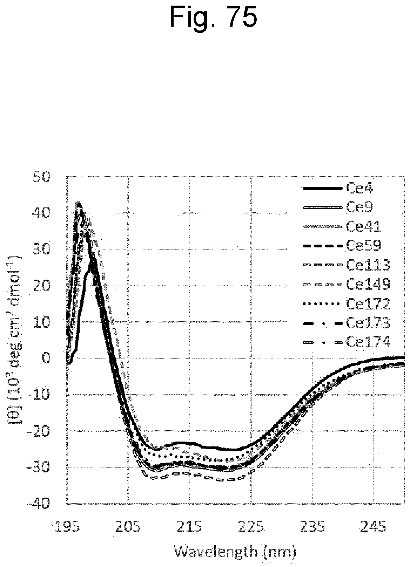

In this Example, Ce4, Ce9, Ce41, Ce59, Ce113, Ce149, Ce172, Ce173 and Ce174 developed in the above Examples were subj ected to measurement of CD spectrum. The results were shown in Figure 75. As is apparent from Figure 75, in all of Ce4, Ce9, Ce41, Ce59, Ce113, Ce149, Ce172, Ce173 and Ce174, the first helix contained in the first region and the second helix contained in the second region form a helix bundle structure to provide a stable structure.

### [Example 14]

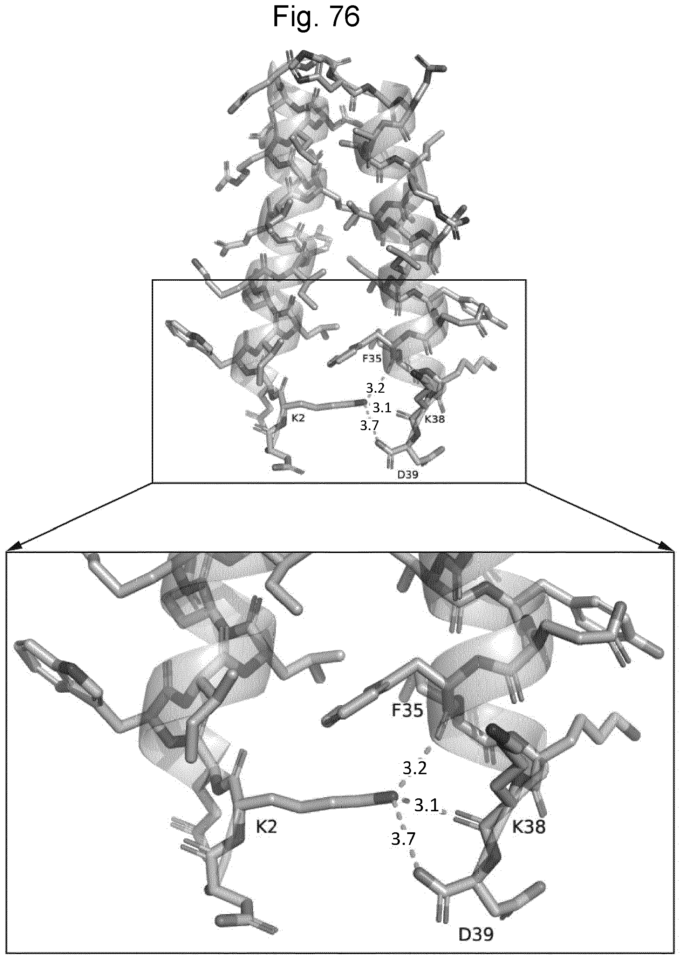

In this Example, a three-dimensional structure of a complex of Ce41 developed in the aforementioned Example and the receptor binding domain (RBD) of the SARS-CoV-2 UK variant (B.1.1.7) was analyzed. A purified protein solution of a complex of Ce41 and the receptor binding domain (RBD) of the SARS-CoV-2 UK variant (B.1.1.7) was concentrated by Amicon ultra manufactured by MerckMillipore. The purified protein solution obtained was set in mosquito LCP manufactured by SPT Labtech and a crystallized drop was prepared by a crystallization plate manufactured by Violamo company. The three-dimensional crystal obtained was treated by anti-freeze treatment and frozen with liquid nitrogen. An X-ray diffraction experiment was carried out by the automatic measurement beam line in the large synchrotron radiation facility SPring-8 (Sayo district, Hyogo prefecture) and diffraction data using X-ray having a wavelength of 1 angstrom were collected. With respect to the data set processed by the automatic data processing system KAMO, the initial phase was determined by Phaser. An atomic model was constructed by use of COOT and refinement was carried out by Refmac5 and PHENIX. In this manner, the three-dimensional structures of Ce41 and the receptor binding domain (RBD) in the SARS-CoV-2 UK variant (B.1.1.7) were determined. The Ce41 structure model was displayed by PyMOL and the interaction between the lysine residue at position 2 in the first region and the C-terminal 5 residues in the second region was analyzed. The results were shown in Figure 76. As a result of the analysis, it was found that a hydrogen bond is formed between the lysine residue at position 2 in the first region and each of the phenylalanine residue at position 15 in the second region (the amino acid residue at position 35 in the whole region of Ce41) and the lysine residue at position 18 of the second region (the amino acid residue at position 38 in the whole region of Ce41). It was also found that a salt bridge was formed between the lysine residue at position 2 in the first region and the aspartic acid residue at position 19 (the amino acid residue at position 39 in the whole region of Ce41) in the second region. It was apparent that these interactions contribute to the stabilization of the helix bundle structure of Ce41.

### [Example 15]

In this Example, Ce4, Ce9, Ce41 and Ce59 developed in the aforementioned Examples were each dissolved in ultra-pure water and the degrees of solubility of them to ultra-pure water were computationally obtained.

Ce4, Ce9, Ce41 and Ce59 having the aforementioned amino acid sequences were chemically synthesized and each dissolved in ultra-pure water. To describe more specifically, the peptides chemically synthesized were each added to ultra-pure water of 250 µL and stirred by pipetting using a micropipette a sufficient number of times and allowed to stand still at room temperature. When undissolved residue was not present, the peptide was further added and the same operation was repeated. When undissolved residue was present, the sample was allowed to stand still for 10 minutes, again stirred by pipetting using a micropipette a sufficient number of times and allowed to stand still at room temperature. After the operation in this step was repeated further twice, concentration was measured by NanoPhotometer NP80 manufactured by IMPLEN to evaluate the degrees of solubility to ultra-pure water. The results were shown in Table 16.

**[Table 16]**

| | Solubility (mM) |
|---|---|
| Ce4 | 4.4 |
| Ce9 | 5.2 |
| Ce41 | 6.1 |
| Ce59 | 6.1 |

From the results, it was found that Ce4, Ce9, Ce41 and Ce59 have extremely high degrees of solubility to ultra-pure water and extremely high binding activity to the receptor binding domain (RBD) in SARS-CoV-2.

### [Example 16]

In this Example, Ce113, Ce149, Ce172, Ce173 and Ce174 developed in the aforementioned Examples were each dissolved in 50 mM HEPES buffer solution (pH 8.0) and the degrees of solubility of them to 50 mM HEPES buffer solution (pH 8.0) were computationally obtained.

Ce113, Ce149, Ce172, Ce173 and Ce174 having the aforementioned amino acid sequences were chemically synthesized and each dissolved in a 50 mM HEPES buffer solution (pH 8.0). To describe more specifically, the peptides chemically synthesized were each added to the 50 mM HEPES buffer solution (pH 8.0) of 250 µL and stirred by pipetting using a micropipette a sufficient number of times and allowed to stand still at room temperature. When undissolved residue was not present, the peptide was further added and the same operation was repeated. When undissolved residue was present, the sample was allowed to stand still for 10 minutes, stirred by pipetting using a micropipette a sufficient number of times and allowed to stand still at room temperature. The operation of this step was repeated further twice and then concentration was measured by NanoPhotometer NP80 manufactured by EMPLEN to evaluate the degrees of solubility to the 50 mM HEPES buffer solution (pH 8.0). The results were shown in Table 17.

**[Table 17]**

| | Solubility (mM) |
|---|---|
| Ce113 | 2.5 |
| Ce149 | 1.3 |
| Ce172 | 1.1 |
| Ce173 | 1.5 |
| Ce174 | 1.6 |

From the results, it was found that Ce113, Ce149, Ce172, Ce173 and Ce174 have extremely high degrees of solubility to the 50 mM HEPES buffer solution (pH 8.0) and extremely high binding activity to the receptor binding domain (RBD) in SARS-CoV-2.

### [Experimental protocol]

### <Protocol for Molecular interaction analysis>

The protocol is used for measuring the binding rate and degree of dissociation of predetermined peptides, which were designed in Examples and chemically synthesized, to the receptor binding domain (RBD) in SARS-CoV-2. In this Example, the molecular interactions between the receptor binding domain (RBD) in SARS-CoV-2 and peptides of interest were analyzed using Biacore S200 manufactured by Cytiva and Biotin CAPture Kit manufactured by Cytiva, and Series S (contents: Sensor Chip CAP, Biotin CAPture Reagent, Regeneration solution). Measurement data were recorded by use of Kinetics multi-cycle using Biotin CAP program of Biacore S200 Control Software manufactured by Cytiva.

First, Sensor Chip CAP was inserted in Biacore S200 and Running Buffer was supplied at a flow rate of 65 mL/day for 12 hours. Running Buffer used herein was prepared by diluting HB S-EP+ buffer 10x manufactured by Cytiva 10 fold with ultra-pure water. Subsequently, the Regeneration solution was injected for one minute and repeated three times. Thereafter, Biotin CAPture Reagent was supplied at a flow rate of 2 µL/min for 300 seconds; biotinylated RBD prepared so as to have a concentration of 1.0 µg/mL at a flow rate of 10 µL/min for 80 seconds; Running Buffer at a flow rate 30 µL/min for 720 seconds (3 hours and 2 minutes when the long-time binding state was studied); and Regeneration solution at a flow rate of 10 µL/min for 120 seconds. The series of four steps was repeated three times.

Subsequently, Biotin CAPture Reagent was supplied at a flow rate of 2 µL/min for 300 seconds; biotinylated RBD prepared so as to have a concentration of 1.0 µg/mL at a flow rate of 10 µL/min for 80 seconds; a peptide of interest at a flow rate 30 µL/min for 120 seconds; Running Buffer at a flow rate 30 µL/min for 600 seconds (3 hours when the long-time binding state was studied); and Regeneration solution at a flow rate of 10 µL/min for 120 seconds. The series of five steps was repeated a number of times depending on the number of peptides to be measured. In this manner, the molecular interaction between the receptor binding domain (RBD) in SARS-CoV-2 and each of the peptides was analyzed.

A peptide of interest was diluted with a solution (dilution Buffer) containing dimethyl sulfoxide in Running Buffer so as to have a final concentration of 0.1% (v/v) to prepare a solution containing the peptide of interest at arbitral concentration. A dilution Buffer was used as the solution containing 0 nM of the peptide of interest.

### <CD spectral measurement protocol>

The protocol is used for measuring a far-ultraviolet circular dichroism (CD) spectrum of a predetermined peptide designed in Examples and chemically synthesized. Based on the CD spectrum measured in accordance with the protocol, it is possible to evaluate whether or not the peptide measured forms a desired helix structure and helix bundle structure.

After a peptide powder of interest was dissolved in TBS (20 mM Tris-HCl pH 8.0, 150 mM NaCl) and the concentration was controlled to be 20 µM, the peptide solution (200 µL) was added in a 1 mm-cuvette. A CD spectrum was measured by a circular dichroism dispersion meter J-720 manufactured by JASCO Corporation and recorded at 25°C at intervals of 0.5 nm in the wavelength range of 250 nm to 195 nm at a scan speed of 20 nm/min.

### <Protocol for plaque reduction assay>

The protocol is used for counting cells infected with SARS-CoV-2. The infection suppression effects of peptides prepared in Examples on SARS-CoV-2 can be evaluated based on the number of cells measured in accordance with the protocol.

First, one day before the measurement date, virus-sensitive cells VeroE6/TMPRSS2 (Matsuyama et al., Proc Natl Acad Sci USA 117: 7001-7003, 2020) were seeded in the wells of a 24-well plate at a density of 1 × 10⁵ cells/well.

At the measurement date, SARS-CoV-2 NIID strain was diluted with a culture medium containing neither serum nor an antibiotic substance so as to be 100 TCID₅₀/180 µL. Subsequently, an undiluted peptide solution was serially diluted 10-fold with TBS (20 mM Tris-HCl pH 8.0, 150 mM NaCl) and 20 µL of each of the dilution solutions was added to the virus solution (as a control, a solution to which 20 µL TBS was added was used). For example, solutions having a peptide final concentration of 2 µM, 400 nM, 80 nM, 16 nM, 3.2 nM, 640 pM and 128 pM were prepared. As the control, a 2% DMSO solution was used.

Subsequently, the culture medium of VeroE6/TMPRSS2, to which the cells were seeded the day before the measurement date, was picked up. A virus/peptide solution (200 µL) was added to the culture medium, and the suspension was incubated at 37°C for one hour. Thereafter, the supernatant was removed and the cells were washed twice with PBS. To the cells, 500 µL of a methylcellulose viscous medium (E-MEM culture medium containing 1% methylcellulose and 2% bovine serum) was added. The suspension was cultured in a CO₂ incubator for 2 days.

After culture, the methylcellulose viscous medium was removed. To the cells, 3.7% formaldehyde was added to fix the cells for 2 hours. Thereafter, formaldehyde was removed and the place was washed with running water. Subsequently, living cells were stained with 1% crystal violet and the number of plaques (mass of cells killed by virus infection) was counted. The numbers of plaques in a viral infection area treated only with TBS and in areas treated with a peptide solution different in concentration were calculated to determine a 50% inhibition concentration (IC₅₀ value).

### Sequence Listing Free Text

SEQ ID NOs: 1 to 66 Synthetic peptides
All publications, patents and patent applications cited herein are incorporated in their entirety by reference.

## Claims

1. A peptide comprising a first region comprising a first helix and a second region comprising a second helix in the direction from an N terminal to a C terminal, wherein a sequence of a site in the first region that binds to the receptor binding domain (RBD) in SARS-CoV-2 comprises the following amino acid sequence (a), (b), (c) or (d):
(a) an amino acid sequence of SEQ ID NO: 1,
(b) an amino acid sequence of SEQ ID NO: 61,
(c) an amino acid sequence of SEQ ID NO: 59, or
(d) an amino acid sequence having a sequence identity of 80% or more with the amino acid sequence of SEQ ID NO: 1, 61 or 59;
a sequence of a site in the second region that binds to the receptor binding domain (RBD) in SARS-CoV-2 comprises the following amino acid sequence (e), (f) or (g):
(e) an amino acid sequence of SEQ ID NO: 2,
(f) an amino acid sequence of SEQ ID NO: 57, or
(g) an amino acid sequence having a sequence identity of 80% or more with the amino acid sequence of SEQ ID NO: 2 or 57; and
a bond is formed between an amino acid residue within 5 residues from N terminal in the sequence of the site in the first region that binds to the receptor binding domain (RBD) in SARS-CoV-2 and an amino acid residue within 5 residues from C terminal in the sequence of the site in the second region that binds to the receptor binding domain (RBD) in SARS-CoV-2, whereby the peptide binds to the receptor binding domain (RBD) in SARS-CoV-2.

2. The peptide according to claim 1, comprising the first region comprising the first helix and the second region comprising the second helix in the direction from the N terminal to the C terminal, wherein the sequence of the site in the first region that binds to the receptor binding domain (RBD) in SARS-CoV-2 comprises the following amino acid sequence (a) or (d1):
(a) the amino acid sequence of SEQ ID NO: 1, or
(d1) an amino acid sequence having a sequence identity of 80% or more with the amino acid sequence of SEQ ID NO: 1;
the sequence of the site in the second region that binds to the receptor binding domain (RBD) in SARS-CoV-2 comprises the following amino acid sequence (e) or (g1):
(e) the amino acid sequence of SEQ ID NO: 2, or
(g1) an amino acid sequence having a sequence identity of 80% or more with the amino acid sequence of SEQ ID NO: 2; and
a bond is formed between an amino acid residue within 5 residues from N terminal in the sequence of the site in the first region that binds to the receptor binding domain (RBD) in SARS-CoV-2, and an amino acid residue within 5 residues from C terminal in the sequence of the site in the second region that binds to the receptor binding domain (RBD) in SARS-CoV-2, whereby the peptide binds to the receptor binding domain (RBD) in SARS-CoV-2.

3. The peptide according to claim 1, comprising the first region comprising the first helix and the second region comprising the second helix in the direction from the N terminal to the C terminal, wherein the sequence of the site in the first region that binds to the receptor binding domain (RBD) in SARS-CoV-2 comprises the following amino acid sequence (b) or (d2):
(b) the amino acid sequence of SEQ ID NO: 61, or
(d2) an amino acid sequence having a sequence identity of 80% or more with the amino acid sequence of SEQ ID NO: 61;
the sequence of the site in the second region that binds to the receptor binding domain (RBD) in SARS-CoV-2 comprises the following amino acid sequence (f) or (g2):
(f) the amino acid sequence of SEQ ID NO: 57, or
(g2) an amino acid sequence having a sequence identity of 80% or more with the amino acid sequence of SEQ ID NO: 57; and
a bond is formed between an amino acid residue within 5 residues from N terminal in the sequence of the site in the first region that binds to the receptor binding domain (RBD) in SARS-CoV-2 and an amino acid residue within 5 residues from C terminal in the sequence of the site in the second region that binds to the receptor binding domain (RBD) in SARS-CoV-2, whereby the peptide binds to the receptor binding domain (RBD) in SARS-CoV-2.

4. The peptide according to claim 1, comprising the first region comprising the first helix and the second region comprising the second helix in the direction from the N terminal to the C terminal, wherein the sequence of the site in the first region that binds to the receptor binding domain (RBD) in SARS-CoV-2 comprises the following amino acid sequence (c) or (d3):
(c) the amino acid sequence of SEQ ID NO: 59, or
(d3) an amino acid sequence having a sequence identity of 80% or more with the amino acid sequence of SEQ ID NO: 59;
the sequence of the site in the second region that binds to the receptor binding domain (RBD) in SARS-CoV-2 comprises the following amino acid sequence (f) or (g2):
(f) the amino acid sequence of SEQ ID NO: 57, or
(g2) an amino acid sequence having a sequence identity of 80% or more with the amino acid sequence of SEQ ID NO: 57; and
a bond is formed between an amino acid residue within 5 residues from N terminal in the sequence of the site in the first region that binds to the receptor binding domain (RBD) in SARS-CoV-2 and an amino acid residue within 5 residues from C terminal in the sequence of the site in the second region that binds to the receptor binding domain (RBD) in SARS-CoV-2, whereby the peptide binds to the receptor binding domain (RBD) in SARS-CoV-2.

5. The peptide according to any one of claims 1 to 4, wherein, as the amino acid residue within 5 residues from N terminal and the amino acid residue within 5 residues from C terminal, a lysine residue and an aspartic acid residue, a lysine residue and a glutamic acid residue, an arginine residue and a glutamic acid residue, or an arginine residue and an aspartic acid residue are used in combination.

6. The peptide according to claim 1 or 2, wherein the amino acid sequence (d) or (d1) is an amino acid sequence obtained by substituting at least one amino acid residue selected from the group consisting of the amino acid residue at position 1, the amino acid residue at position 8, the amino acid residue at position 11 and the amino acid residue at position 18 in the amino acid sequence of SEQ ID NO: 1, with another amino acid residue.

7. The peptide according to claim 1 or 2, wherein the amino acid sequence (g) or (g1) is an amino acid sequence obtained by substituting at least one amino acid residue selected from the group consisting of the amino acid residue at position 2, the amino acid residue at position 14 and the amino acid residue at position 18 in the amino acid sequence of SEQ ID NO: 2, with another amino acid residue.

8. The peptide according to claim 1 or 2, wherein the amino acid sequence (d) and (d1) are amino acid sequences obtained by substituting the arginine residue at position 15 and the glutamic acid residue at position 19 in the amino acid sequence of SEQ ID NO: 1 with a lysine residue and an aspartic acid residue, respectively.

9. The peptide according to claim 1 or 2, wherein the amino acid sequence (g) or (g1) is an amino acid sequence obtained by substituting the glycine residue at position 2 in the amino acid sequence of SEQ ID NO: 2 with an alanine residue.

10. The peptide according to claim 1 or 2, wherein the amino acid sequence (g) or (g1) is an amino acid sequence obtained by substituting the arginine residue at position 18 in the amino acid sequence of SEQ ID NO: 2 with a lysine residue.

11. The peptide according to claim 1 or 2, wherein the amino acid sequence (d) or (d1) is an amino acid sequence in which the tyrosine residue at position 10 and the methionine acid residue at position 13 in the amino acid sequence of SEQ ID NO: 1 are further conserved.

12. The peptide according to claim 1 or 2, wherein the amino acid sequence (g) or (g1) is an amino acid sequence in which the histidine residue at position 1, the serine residue at position 9, the aspartic acid residue at position 10 and the tyrosine residue at position 13 in the amino acid sequence of SEQ ID NO: 2 are further conserved.

13. The peptide according to any one of claims 1 to 4, wherein the sequence of the site in the first region that binds to the receptor binding domain (RBD) in SARS-CoV-2 and/or the sequence of the site in the second region that binds to the receptor binding domain (RBD) in SARS-CoV-2 is constituted of 18 to 22 amino acid residues.

14. The peptide according to any one of claims 1 to 4, consisting of the first region and the second region and having a full-length sequence of 39 amino acid residues.

15. The peptide according to any one of claims 1 to 4, consisting of a single amino acid sequence selected from the group consisting of SEQ ID NOs: 52 to 55.

16. A peptide that binds to a receptor binding domain (RBD) in SARS-CoV-2, wherein the peptide has a first region and a second region in the direction from an N terminal to a C terminal, wherein
the first region comprises an amino acid sequence selected from the following (h), (i), (j), (k) or (l):
(h) an amino acid sequence of SEQ ID NO: 56,
(i) an amino acid sequence of SEQ ID NO: 59,
(j) an amino acid sequence of SEQ ID NO: 61,
(k) an amino acid sequence of SEQ ID NO: 63, or
(l) an amino acid sequence of SEQ ID NO: 65; and
the second region comprises an amino acid sequence of SEQ ID NO: 57.

17. The peptide according to claim 16, wherein the first region consists of the amino acid sequence of SEQ ID NO: 56.

18. The peptide according to claim 16, wherein the first region consists of the amino acid sequence of SEQ ID NO: 59.

19. The peptide according to claim 16, wherein the first region consists of the amino acid sequence of SEQ ID NO: 61.

20. The peptide according to claim 16, wherein the first region consists of the amino acid sequence of SEQ ID NO: 63.

21. The peptide according to claim 16, wherein the first region consists of the amino acid sequence of SEQ ID NO: 65.

22. The peptide according to any one of claims 16 to 21, wherein the second region consists of the amino acid sequence of SEQ ID NO: 57.

23. A peptide that binds a receptor binding domain (RBD) in SARS-CoV-2, the peptide consisting of an amino acid sequence of SEQ ID NO: 58.

24. A peptide that binds to a receptor binding domain (RBD) in SARS-CoV-2, the peptide consisting of an amino acid sequence of SEQ ID NO: 60.

25. A peptide that binds to a receptor binding domain (RBD) in SARS-CoV-2, the peptide consisting of an amino acid sequence of SEQ ID NO: 62.

26. A peptide that binds to a receptor binding domain (RBD) in SARS-CoV-2, the peptide consisting of an amino acid sequence of SEQ ID NO: 64.

27. A peptide that binds to a receptor binding domain (RBD) in SARS-CoV-2, the peptide consisting of an amino acid sequence of SEQ ID NO: 66.

28. A pharmaceutical composition comprising the peptide according to any one of claims 1 to 27 as an active ingredient.

29. The pharmaceutical composition according to claim 28, for treating COVID-19.

30. The pharmaceutical composition for treating COVID-19 according to claim 29, wherein the COVID-19 is caused by infection with any one of wild-type SARS-CoV-2 having a receptor binding domain (RBD) in which an amino acid residue at position 339 is a glycine residue, an amino acid residue at position 371 is a serine residue, an amino acid residue at position 373 is a serine residue, an amino acid residue at position 375 is a serine residue, an amino acid residue at position 376 is a threonine residue, an amino acid residue at position 405 is an aspartic acid residue, an amino acid residue at position 408 is an arginine residue, an amino acid residue at position 417 is a lysine residue, an amino acid residue at position 440 is an asparagine residue, an amino acid residue at position 446 is a glycine residue, an amino acid residue at position 452 is a leucine residue, an amino acid residue at position 477 is a serine residue, an amino acid residue at position 478 is a threonine residue, an amino acid residue at position 484 is a glutamic acid residue, an amino acid residue at position 493 is a glutamine residue, an amino acid residue at position 496 is a glycine residue, an amino acid residue at position 498 is a glutamine residue, an amino acid residue at position 501 is an asparagine residue, and an amino acid residue at position 505 is a tyrosine residue; a first SARS-CoV-2 variant in which the amino acid residue at position 501 is a tyrosine residue; a second SARS-CoV-2 variant in which the amino acid residue at position 484 is a lysine residue; a third SARS-CoV-2 variant in which the amino acid residue at position 417 is an asparagine residue, the amino acid residue at position 484 is a lysine residue, and the amino acid residue at position 501 is a tyrosine residue; a fourth SARS-CoV-2 variant in which the amino acid residue at position 417 is a threonine residue, the amino acid residue at position 484 is a lysine residue, and the amino acid residue at position 501 is a tyrosine residue; a fifth SARS-CoV-2 variant in which the amino acid residue at position 452 is an arginine residue and the amino acid residue at position 478 is a lysine residue; a sixth SARS-CoV-2 variant, in which the amino acid residue at position 452 is an arginine residue, and the amino acid residue at position 484 is a glutamine residue; a seventh SARS-CoV-2 variant, in which the amino acid residue at position 339 is an aspartic acid residue, the amino acid residue at position 371 is a leucine residue, the amino acid residue at position 373 is a proline residue, the amino acid residue at position 375 is a phenylalanine residue, the amino acid residue at position 417 is an asparagine residue, the amino acid residue at position 440 is a lysine residue, the amino acid residue at position 446 is a serine residue, the amino acid residue at position 477 is an asparagine residue, the amino acid residue at position 478 is a lysine residue, the amino acid residue at position 484 is an alanine residue, the amino acid residue at position 493 is an arginine residue, the amino acid residue at position 496 is a serine residue, the amino acid residue at position 498 is an arginine residue, the amino acid residue at position 501 is a tyrosine residue, and the amino acid residue at position 505 is a histidine residue; and an eighth SARS-CoV-2 variant, in which the amino acid residue at position 339 is an aspartic acid residue, the amino acid residue at position 371 is a phenylalanine residue, the amino acid residue at position 373 is a proline residue, the amino acid residue at position 375 is a phenylalanine residue, the amino acid residue at position 376 is an alanine residue, the amino acid residue at position 405 is an asparagine residue, the amino acid residue at position 408 is a serine residue, the amino acid residue at position 417 is an asparagine residue, the amino acid residue at position 440 is a lysine residue, the amino acid residue at position 477 is an asparagine residue, the amino acid residue at position 478 is a lysine residue, the amino acid residue at position 484 is an alanine residue, the amino acid residue at position 493 is an arginine residue, the amino acid residue at position 498 is an arginine residue, the amino acid residue at position 501 is a tyrosine residue, and the amino acid residue at position 505 is a histidine residue.

31. The pharmaceutical composition for treating COVID-19 according to claim 29, wherein the composition is an aqueous solution having the above peptide dissolved therein.
